(19) Europäisches Patentamt
European Patent Office
Office européen des brevets

(11) **EP 4 393 440 A1**

(12) # EUROPEAN PATENT APPLICATION

(43) Date of publication:
**03.07.2024 Bulletin 2024/27**

(21) Application number: **23218602.3**

(22) Date of filing: **20.12.2023**

(51) International Patent Classification (IPC):
***A61B 34/00*** *(2016.01)* ***A61B 34/37*** *(2016.01)*

(52) Cooperative Patent Classification (CPC):
**A61B 34/37; A61B 34/25;** A61B 90/90; A61B 2034/2059; A61B 2034/256; A61B 2034/258; A61B 2090/0803; A61B 2090/0804; A61B 2090/0807; A61B 2090/0814

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 JP 2022209879**

(71) Applicant: **SYSMEX CORPORATION**
**Kobe-shi**
**Hyogo 651-0073 (JP)**

(72) Inventors:
- **OHASHI, Masanao**
  **Kobe-shi, Hyogo, 651-0073 (JP)**
- **KITAYAMA, Yutaka**
  **Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

(54) **METHOD OF SURGICAL INSTRUMENT MANAGEMENT, INFORMATION PROCESSOR, MANAGEMENT SYSTEM, SURGICAL INSTRUMENT AND SURGICAL ROBOT**

(57) To provide a method of managing a surgical instrument, an information processor, a management system, a surgical instrument, and a surgical robot that can enhance surgical safety. A method of managing a surgical instrument 120 that is removably attached to a surgical robot and is reusable up to a usage limit, obtains information on a usage status of the surgical instrument 120 used in a surgery by the surgical robot, obtains a judgment result indicating that the surgical instrument 120 whose usage status is within the usage limit is unavailable for use, and associates the information on the surgical instrument 120 with the judgment result.

FIG.9

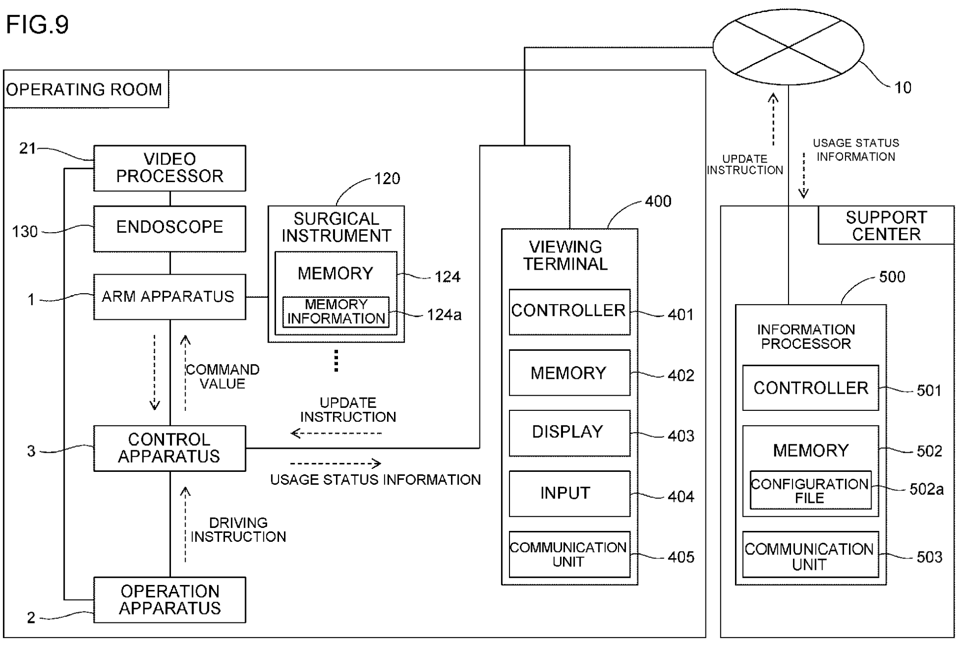


EP 4 393 440 A1

## Description

TECHNICAL FIELD

The invention relates to a method of surgical instrument management, an information processor, a management system, a surgical instrument, and a surgical robot.

In a related art, a surgical operation has been performed using a surgical robot. The surgical robot is provided with a console and a surgical robot arm. During surgery, surgical instruments such as forceps are attached to the robotic arm, and the robotic arm and surgical instruments are operated by an operator operating the console.

In order to prevent a malfunction of a surgical instrument attached to a surgical robot and a surgical robotic arm during a surgical operation and to complete a surgical operation safely, it is important to track and properly manage the usage status of a surgical instrument used with a surgical robot, such as the number of uses and duration of use. For example, some surgical instruments have predefined limits on the number of times to be used and the period of time to be used as usage limits. The specification of US Patent No. 8285517 (Patent Document 1) describes a medical robot system that tracks the number of uses or period of use of a surgical instrument used in a surgical robot and notifies an operator that preventive maintenance is required when the number of uses or period of use exceeds a threshold value.

However, the system described in the above Patent Document 1 only determines whether or not a surgical instrument needs to be replaced based on the number of times the surgical instrument has been used and the period of use, which is uniform. Even if the number of use or period of use has not reached the threshold values, for example, even if a surgical instrument meets the performance evaluation criteria set by the manufacturer, the performance of the surgical instrument may differ slightly from expectations, and for some operators, the performance of the surgical instruments the operators start using may differ from their expectations, and it may be better to refrain from using the surgical instrument. Also, in case that a surgical instrument is damaged during surgery or during cleaning and sterilization after surgery, it may be better to refrain from using the surgical instrument. In such cases, the system described in Patent Document 1 above does not sufficiently ensure the safety of surgery. It is desirable to be able to enhance the safety of surgery by considering various situations as well as the number of times and duration of use of a surgical instrument.

In view of such problems, the purpose of the present invention is to provide a method of managing a surgical instrument, an information processor, a management system, a surgical instrument, and a surgical robot that can enhance surgical safety.

SUMMARY

The present invention is a method of managing a surgical instrument (120) that is removably attached to a surgical robot (4) and reusable up to a usage limit, wherein information concerning the usage status of the surgical instrument (120) used for a surgery by the surgical robot (4) is obtained (Step S301 in FIGS. 14, 23, and 27), a judgment result indicating that the surgical instrument (120) whose usage status is within the usage limit cannot be used is obtained (Step S3064 in FIG. 15: YES, Step S3126 in FIG. 24: YES), and information about the surgical instrument (120) is associated with the judgment result (Step S3065 in FIG. 15, Step S3127 in FIG. 24).

According to the method of managing a surgical instrument, even if a surgical instrument is within the usage limit, a judgment result indicating that the surgical instrument cannot be used can be managed in association with the information about the surgical instrument. Therefore, a uniform judgment based on a single condition, such as whether an instrument is within the usage limit, can be suppressed, thereby enhancing the safety of surgery.

The present invention is an information processor (500) that manages a surgical instrument (120), which is removably attached to a surgical robot (4) and is reusable up to a usage limit, and includes a communication unit (503) that communicates with the surgical robot (4) and a controller (501) that is connected to the communication unit (503). The controller (501) obtains information on a usage status of the surgical instrument (120) used for surgery by the surgical robot (4), obtains a judgment result indicating that the surgical instrument (120) whose usage status is within the usage limit cannot be used, and associates the information on the surgical instrument (120) with the judgment result.

According to the information processor of the present invention, even if a surgical instrument is within the usage limit, the judgment result indicating that the surgical instrument is not usable can be managed in association with the information about the surgical instrument. Therefore, the uniform judgment based on a single condition of whether the surgical instrument is within the usage limit can be suppressed, thereby enhancing the safety of surgery.

The present invention is a management system (5) that manages the surgical instrument (120), which is removably attached to the surgical robot (4) and reusable up to the usage limit, and includes the information processor (500) and viewing terminals (400, 410) that are communicably connected to the information processor (500). The information processor (500) has the communication unit (503) that communicates with the surgical robot (4) and the controller (501) that is connected to the communication unit (503). The controller (501) obtains information on the usage status of the surgical instrument (120) used for surgery by the surgical robot (4), obtains a judgment result indicating that the surgical instrument (120) whose usage status is within the usage limit cannot be used, and provides information based on the judgment result to the viewing terminals (400, 410).

According to the management system of the present invention, even if a surgical instrument is within the usage limit, the judgment result indicating that the surgical instrument cannot be used can be managed in association with the information about the surgical instrument. Therefore, the uniform judgment based on a single condition of whether the surgical instrument is within the usage limit can be suppressed, thereby enhancing the safety of surgery. In addition, the judgment result of whether the surgical instrument is usable can easily be confirmed via the viewing terminal.

The present invention is a surgical instrument (120) that is removably attached to the surgical robot (4) and is reusable up to the usage limit, and has a memory (124) that stores information concerning the surgical instrument (120). The memory (124) includes a memory area (124b) that stores information on the surgical instrument (120) used for a surgery by the surgical robot (4) and a judgment result indicating unavailable determined for the surgical instrument (120) whose usage status is within the usage limit in association with the information on the surgical instrument (120).

According to the surgical instrument of the present invention, even if the surgical instrument is within the usage limit, the judgment result indicating that the surgical instrument is not usable can be managed in association with the information about the surgical instrument. Therefore, the uniform judgment based on a single condition, such as whether the instrument is within the usage limit, can be suppressed, thereby enhancing the safety of surgery.

The present invention is the surgical robot (4) that performs a surgical operation using the surgical instrument (120) that is reusable up to the usage limit, and includes an arm (110) to which the surgical instrument (120) is removably attached and a controller (141) that controls an operation of the arm (110). The controller (141) obtains a judgment result indicating that the surgical instrument (120) that is within the usage limit attached to the arm (110) cannot be used, and outputs an alert indicating that the judgment result is obtained.

According to the surgical robot of the present invention, a surgical instrument that is preferable for not being used even within the expiration date is prevented from being used in surgery, thereby enhancing the safety of surgery.

EFFECTS OF THE INVENTION

According to the present invention, the safety of surgery can be improved.

BRIEF DESCRIPTION OF DRAWINGS

FIG. 1 is a block diagram illustrating a surgical robot and a surgical instrument according to Embodiment 1;
FIG. 2 is a perspective view illustrating appearances of an arm apparatus and the surgical instrument according to Embodiment 1;
FIG. 3 is a diagram illustrating a configuration of the surgical instrument attached to the arm and an end of the arm according to Embodiment 1;
FIG. 4 is a perspective view illustrating an appearance of an operation apparatus according to Embodiment 1;
FIG. 5 is a block diagram illustrating a configuration of a controller according to Embodiment 1;
FIG. 6 is a block diagram illustrating configurations of the arm apparatus and the surgical instrument according to Embodiment 1;
FIG. 7 is a block diagram illustrating a configuration of the operation apparatus according to Embodiment 1;
FIG. 8 is a block diagram illustrating a configuration of a management system according to Embodiment 1;
FIG. 9 is a diagram illustrating an overview of connections of the surgical robot and an information processor, etc., and information transmitted and received between each apparatus according to Embodiment 1;
FIG. 10 is a diagram schematically illustrating an item included in the memory information stored in a memory of the surgical instrument according to Embodiment 1;
FIG. 11 is a diagram schematically illustrating a configuration of a configuration file stored in the memory of the information processor according to Embodiment 1;
FIG. 12 is a flowchart illustrating a process by a controller of the arm apparatus according to Embodiment 1;
FIG. 13 is a flowchart illustrating a process by a controller of the control apparatus according to Embodiment 1;
FIG. 14 is a flowchart illustrating a process by a controller of the information processor according to Embodiment 1;
FIG. 15 is a flowchart illustrating the details of a judgment screen display process according to Embodiment 1;
FIG. 16 is a diagram schematically illustrating a configuration of the judgment screen displayed on a display of the viewing terminal according to Embodiment 1;
FIG. 17 is a diagram schematically illustrating a configuration of the judgment screen displayed on the display of the viewing terminal according to Embodiment 1;
FIG. 18 is a diagram schematically illustrating a configuration of a configuration file for a case in which the cumulative time attached to the arm (usage time) is stored according to a variation of Embodiment 1;

FIG. 19 is a diagram schematically illustrating the judgment screen for the case in which the cumulative time attached to the arm (usage time) is stored according to the variation of Embodiment 1;

FIG. 20 is a block diagram illustrating configurations of an arm apparatus and a surgical instrument according to Embodiment 2;

FIG. 21 is a flowchart illustrating a process by a controller of the arm apparatus according to Embodiment 2;

FIG. 22 is a flowchart illustrating a process by a controller of a control apparatus according to Embodiment 2;

FIG. 23 is a flowchart illustrating a process by a controller of an information processor according to Embodiment 2;

FIG. 24 is a flowchart illustrating the details of an automatic detection process according to Embodiment 2;

FIG. 25 is a diagram schematically illustrating the details of a calculation process in the automatic detection process according to Embodiment 2;

FIG. 26 is a diagram schematically illustrating a configuration of a judgment screen displayed on a display of a viewing terminal according to Embodiment 2;

FIG. 27 is a flowchart illustrating a process by a controller of an information processor according to Embodiment 3;

FIG. 28 is a diagram schematically illustrating a configuration of a judgment screen displayed on a display of a viewing terminal according to Embodiment 3;

FIG. 29 is a diagram schematically illustrating a configuration of a configuration file according to Embodiment 4;

FIG. 30 is a diagram schematically illustrating a configuration of a configuration file according to a variation according to Embodiment 4;

FIG. 31 is a flowchart illustrating a process related to a display of an extraction screen by a controller of an information processor according to Embodiment 5;

FIG. 32 is a diagram schematically illustrating a configuration of a configuration file according to Embodiment 5;

FIG. 33 is a diagram schematically illustrating a configuration of an extraction screen displayed on a display of a viewing terminal according to Embodiment 5;

FIG. 34 is a diagram schematically illustrating a configuration of a configuration file according to a variation of Embodiment 5;

FIG. 35 is a diagram schematically illustrating a configuration of an extraction screen displayed on a display of a viewing terminal according to a variation of Embodiment 5;

FIG. 36 is a flowchart illustrating a process by a controller of an operation apparatus according to Embodiment 6;

FIG. 37 is a diagram illustrating an overview of a connection of a surgical robot and an information processor, etc. and the information transmitted and received between each apparatus according to Embodiment 7;

FIG. 38 is a diagram schematically illustrating a configuration of a configuration file stored in a memory of the information processor according to Embodiment 7;

FIG. 39 is a diagram schematically illustrating a configuration of an inventory DB according to Embodiment 7;

FIG. 40 is a flowchart illustrating an updating process of the inventory DB by a controller of the information processor according to Embodiment 7;

FIG. 41 is a flowchart illustrating a display process of an inventory management screen by the controller of the information processor according to Embodiment 7;

FIG. 42 is a diagram schematically illustrating a configuration of a list screen displayed on a display of a viewing terminal according to Embodiment 7;

FIG. 43 is a flowchart illustrating the details of a status screen display process according to Embodiment 7;

FIG. 44 is a diagram schematically illustrating a configuration of a status screen displayed on the display of the viewing terminal according to Embodiment 7;

FIG. 45 is a diagram schematically illustrating a configuration of the status screen displayed on the display of the viewing terminal according to Embodiment 7;

FIG. 46 is a diagram schematically illustrating a configuration of a status screen displayed on a display of a viewing terminal according to Embodiment 7;

FIG. 47 is a diagram schematically illustrating a configuration of a list screen displayed on the display of the viewing terminal according to a variation of Embodiment 7;

FIG. 48 is a diagram schematically illustrating a configuration of an inventory DB according to a variation of Embodiment 7;

FIG. 49 is a diagram schematically illustrating a configuration of an inventory quantity list screen displayed on the display of the viewing terminal according to a variation of Embodiment 7;

FIG. 50 is a diagram illustrating an overview of a connection of each apparatus according to Embodiment 8;

FIG. 51 is a flowchart illustrating a display process of a compensation screen by a controller of an information processor according to Embodiment 8;

FIG. 52 is a diagram schematically illustrating a configuration of the compensation screen displayed on a display of a viewing terminal according to Embodiment 8;

FIG. 53 is a diagram schematically illustrating a configuration of a configuration file stored in a memory of an information processor according to a variation 1 of Embodiment 8;
FIG. 54 is a diagram schematically illustrating a configuration of the compensation screen displayed on the display of the viewing terminal according to a variation 1 of Embodiment 8;
FIG. 55 is a diagram schematically illustrating a configuration of the compensation screen displayed on the display of the viewing terminal according to a variation 1 of Embodiment 8;
FIG. 56 is a flowchart illustrating a determination process of compensation details by the controller of the information processor according to a variation 2 of Embodiment 8;
FIG. 57 is a diagram schematically illustrating a configuration of a compensation details screen displayed on the display of the viewing terminal according to a variation 2 of Embodiment 8;
FIG. 58 is a flowchart illustrating a process of determining the compensation details by the controller of the information processor according to a variation 2 of Embodiment 8;
FIG. 59 is a diagram schematically illustrating a configuration of user information according to Embodiment 9;
FIG. 60 is a flowchart illustrating a login process by a controller of an information processor according to Embodiment 9;
FIG. 61 is a diagram schematically illustrating a configuration of a status screen displayed on a display of an operator terminal according to Embodiment 9;
FIG. 62 is a diagram schematically illustrating an arrangement of an apparatus in an operating room of a medical facility according to Embodiment 10; and
FIG. 63 is a diagram schematically illustrating a configuration and a connection of a cradle according to Embodiment 11.

DETAILED DESCRIPTION

<Embodiment 1>

FIG. 1 is a block diagram illustrating configurations of a surgical robot 4 and a surgical instrument 120.

The surgical robot 4 is an apparatus used in endoscopic surgery. The surgical robot 4 is provided with an arm apparatus 1, an operation apparatus 2, and a control apparatus 3 that controls the arm apparatus 1 and the operation apparatus 2. The control apparatus 3 is built into the arm apparatus 1. The arm apparatus 1 with the built-in control apparatus 3 and the operation apparatus 2 may be installed in the same room in a medical facility, in different rooms in a medical facility, or in different facilities via a network. Three surgical instruments 120 are attached to the arm apparatus 1. The three surgical instruments 120 are operated to perform surgery on a patient. The number of the surgical instrument 120 attached to the arm apparatus 1 is not limited to three, but may be one, two, or four or more.

FIG. 2 is a perspective view illustrating an appearance of the arm apparatus 1 and the surgical instrument 120.

The arm apparatus 1 is a patient-side apparatus with four arms 110 that can be equipped with three surgical instruments 120 and one endoscope 130. The arm apparatus 1 operates according to a command value transmitted from the control apparatus 3 when the operation apparatus 2 is operated by an operator, a physician.

The arm apparatus 1 includes a base 101, an operating terminal 102, a base arm 103, a support 104, and four arms 110. Three surgical instruments 120 and one endoscope 130 are removably attached to the four arms 110, respectively.

The operating terminal 102 is located on the base 101 and includes a display and an input device. The arm 110 of the arm apparatus 1 is positioned to an initial position that is suitable for surgery by operating the input device of the operating terminal while viewing the display of the operating terminal 102. The base arm 103 is an arm whose rear end is installed on the base 101 and is provided with multiple joints. The support 104 has its upper surface connected to a tip of the base arm 103 in a rotatable manner, and moves with a movement of the tip of the base arm 103 and rotates around an axis of the tip of the base arm 103.

Four arms 110 have their upper ends installed on an underside of the support 104. One arm 110 is an arm with 12 axes and inter-axial joints, respectively. At a lower end of the arm 110 is a supporting tool configured for attaching and detaching the surgical instrument 120 and the endoscope 130, and the surgical instrument 120 or the endoscope 130 is attached to the supporting tool.

The surgical instrument 120 has an elongated shaft 121, and the endoscope 130 has an elongated shaft 131. The surgical instrument 120 has an end effector 122 (see FIG. 3) at the tip. The endoscope 130 is, for example, a 3D videoscope.

Prior to a start of surgery, a patient lying on an operating table is positioned below the surgical instrument 120 and the endoscope 130. Once the surgery is started, a guide tube (trocar) is inserted into the patient's abdomen, and the shafts 121 and 131 of the surgical instrument 120 and the endoscope 130 are inserted into the patient's body through the guide tube. Thus, the surgery using the surgical instrument 120 proceeds. Usually, the surgical instrument 120 is replaced several times during surgery with a different type of the surgical instrument 120, depending on a procedure.

FIG. 3 is a diagram illustrating configurations of the arm 110 and the surgical instrument 120 attached to an end of the arm 110. The surgical instrument 120 is a various type of an instrument, such as grasping forceps, scissors, a hook, a highfrequency knife, a bipolar, a monopolar, a snare wire, a clamp, and a stapler.

The surgical instrument 120 has the shaft 121, the end effector 122, a housing 123, a memory 124, and an adapter 125. The end effector 122 varies depending on the type of the surgical instrument 120. FIG. 3 illustrates the end effector 122 in case that the surgical instrument 120 is grasping forceps. A drape 111 is put on the arm 110.

The shaft 121 has an elongated shape. The end effector 122 is provided at the end of the shaft 121. The housing 123 has an abbreviated cylindrical shape and supports the end of the shaft 121 opposite the end effector 122.

The memory 124 is located in the housing 123 and is configured with, for example, a flash memory. The memory 124 stores a memory information 124a that includes information about the surgical instrument 120. The memory information 124a is described later with reference to FIG. 10. The adapter 125 is located on the underside of the housing 123. The drape 111 is a cover to prevent infection to the patient caused by the arm 110.

In case that the surgical instrument 120 is connected to the arm 110, the adapter 125 of the surgical instrument 120 is attached to the supporting tool on the side of the arm 110. Therefore, each part of the surgical instrument 120 and the arm 110 are connected mechanically and electrically. The arm apparatus 1 can detect the attachment of the surgical instrument 120 by detecting the energization with the surgical instrument 120. Once each part of the surgical instrument 120 and the arm 110 are electrically connected, the arm apparatus 1 can read information from and write information to the memory 124 of the surgical instrument 120. In case that the surgical instrument 120 is removed from the arm 110, the adapter 125 of the surgical instrument 120 is removed from the supporting tool on the side of the arm 110.

FIG. 4 is a perspective view illustrating an appearance of the operation apparatus 2.

The operation apparatus 2 is an operator-side apparatus that drives the arm apparatus 1 by being operated by an operator, a physician, during endoscopic surgery.

The operation apparatus 2 includes a base 201, a support 202, a frame member 203, an operation panel 204, a viewer unit 210, two hand controllers 220, and a foot unit 230.

The support 202 is installed on top of the base 201 so as to be movable upward and downward. The frame member 203 is installed on the support 202. The operation panel 204 is installed on a top surface of a front center of the frame member 203. Armrests 203a are formed on the left and right sides of the operation panel 204 for the operator to rest his/her elbows on when operating the hand controller 220. The height of the armrest 203a can be changed by moving the support 202 up and down with respect to the base 201.

The viewer unit 210 is supported by the end of an arm 213 installed on the top surface of the support 202, and the height of the viewer unit 210 can be changed by rotating the joints of the arm 213. The viewer unit 210 is provided with a viewer 211 and a head sensor 212. The viewer 211 is a display that displays an image taken by the endoscope 130 (endoscopic image) and various screens. The head sensor 212 is provided across an area in front of the viewer 211 and is a transmission-type photoelectric sensor. When an operator looks into the viewer 211, the operator's head approaches the viewer 211, an area between the head sensor 212 and the operator's head are shaded, and a condition that the operator is looking at the viewer 211 is detected.

The two of the hand controllers 220, each with seven axes and inter-axial joints, are located on the support 202. The left and right hand controllers 220 are input devices for the operator to operate the four of the arms 110 of the arm apparatus 1 using the left and right hands, respectively.

When the operator inputs an operation of movement to the hand controller 220, each joint of the hand controller 220 rotates on its axis. As a result, the target arm 110 is driven and the surgical instrument 120 or endoscope 130 attached to the arm 110 moves. In case that the surgical instrument 120 with forceps as the end effector 122 is attached to the target arm 110, when the operator inputs a grasping operation to the hand controller 220, the tip of the forceps is driven to open and close.

The foot unit 230 is located at a lower front side of the base 201. The foot unit 230 is an input apparatus for the operator to operate the arm apparatus 1 using the left and right feet.

By performing an operation on the foot unit 230, the operator can switch the surgical instrument 120 and the endoscope 130 to be operated by the left and right hand controllers 220. The operator can also cause the end effector 122 on the tip of the surgical instrument 120 to perform a predetermined operation (e.g., incision, coagulation, etc.) by performing an operation on the foot unit 230.

Before the start of the surgery, the operator sits in a chair, places both arms on the armrests 203a and both feet inside or in front of the foot unit 230. The operator places his/her head inside the viewer unit 210 and looks into the viewer 211. When the head sensor 212 detects that the operator's head is positioned inside the viewer unit 210, the arm apparatus 1 can be operated by the operation apparatus 2.

FIG. 5 is a block diagram illustrating a configuration of the control apparatus 3.

The control apparatus 3 has a controller 301, a memory 302, and a communication unit 303. The controller 301 is configured with, for example, a CPU. The controller 301 controls each part of a hardware of the control apparatus 3 and executes various processes by executing a computer program stored in the memory 302. The memory 302 is configured with, for example, an SSD or HDD. The communication unit 303 is provided with a communication interface capable of communicating with the arm apparatus 1, the operation apparatus 2, and an information processor 500 (see FIG. 8) based on a predetermined communication standard.

FIG. 6 is a block diagram of the arm apparatus 1 and the surgical instrument 120.

The arm apparatus 1 has a controller 141, a memory 142, a communication unit 143, a plurality of actuators 150, and four sensors 161.

The controller 141 is configured with, for example, an FPGA or a CPU. The memory 142 is configured with, for example, a ROM and RAM. The communication unit 143 is provided with a communication interface capable of communicating with the control apparatus 3 based on a predetermined communication standard.

A plurality of actuators 150 are provided corresponding to the base arm 103 and each of the four arms 110 of the arm apparatus 1. The plurality of the actuators 150 corresponding to one arm 110 drive the plurality of joints of the arm 110 and the plurality of mechanisms for operating the instrument 120 or the endoscope 130 connected to the arm 110. Each of the actuators 150 is provided with a motor 151. The motor 151 is a stepping motor. The controller 141 drives the motor 151 of the corresponding actuator 150 based on a command value (see FIG. 9) received from the control apparatus 3 via the communication unit 143.

Four of the sensors 161 are installed on each of the four arms 110 (see FIG. 2) to detect the attachment and detachment of the surgical instrument 120 and the endoscope 130 to and from the arm 110. The sensor 161 may be, for example, a sensor that detects the electrical connection of the surgical instrument 120 and the endoscope 130 to the arm 110, or a photoelectric sensor that detects the physical attachment and detachment of the surgical instrument 120 and the endoscope 130.

The memory 124 of the surgical instrument 120 stores a surgical instrument ID, type, and other information of the surgical instrument 120. When the surgical instrument 120 is attached to the arm 110, the controller 141 reads the surgical instrument ID, type, etc. attached to the surgical instrument 120 from the memory 124 of the surgical instrument 120.

FIG. 7 is a block diagram illustrating a configuration of the operation apparatus 2.

The operation apparatus 2 has a controller 241, a memory 242, a communication unit 243, a viewer 211, a head sensor 212, a plurality of encoders 251, a plurality of limit sensors 252, and a plurality of foot sensors 253.

The controller 241 is configured with, for example, an FPGA or CPU. The memory 142 is configured with, for example, an SSD or HDD. The communication unit 243 is provided with a communication interface capable of communicating with the control apparatus 3 and a video processor 21 (see FIG. 9) based on a predetermined communication standard.

The plurality of encoders 251 are provided on the left and right hand controllers 220 and output the amount of movement and rotation corresponding to an operation input to the left and right hand controllers 220. The plurality of limit sensors 252 detect whether a foot pedal in the foot unit 230 is stepped on. The plurality of foot sensors 253 are reflective photoelectric sensors that detect a position of the operator's foot in the foot unit 230.

The controller 241 transmits a drive instruction based on an output value of the plurality of encoders 251, a detection signal of the plurality of limit sensors 252, and a detection signal of the plurality of foot sensors 253 to the control apparatus 3 via the communication unit 243.

FIG. 8 is a block diagram illustrating a configuration of a management system 5.

The management system 5 is provided with a viewing terminal 400 and an information processor 500. A network 10 is, for example, the Internet.

The viewing terminal 400 includes a controller 401, a memory 402, a display 403, an input 404, and a communication unit 405.

The controller 401 is configured with, for example, a CPU. The controller 401 controls each part of the hardware of the viewing terminal 400 and executes various processes by executing a computer program stored in the memory 402. The memory 402 is configured with, for example, an SSD or HDD. The display 403 is configured with, for example, a liquid crystal display. The input 404 is configured with, for example, a keyboard and a mouse. The display 403 and the input 404 may be configured with a touch panel display. The communication unit 405 is provided with a communication interface capable of communicating with the information processor 500 based on a predetermined communication standard, such as Ethernet or Wi-Fi, for example.

The controller 401 receives screen information from the information processor 500 by executing a web browser or a predetermined application. Then, the controller 401 displays various screens on the display 403 based on received screen information. An operator of the viewing terminal 400 operates an operator included in the screen displayed on the display 403 via the input 404. Therefore, an instruction is sent from the viewing terminal 400 to the information processor 500.

The information processor 500 includes a controller 501, a memory 502, and a communication unit 503.

The controller 501 is configured with, for example, a CPU. The controller 501 controls each part of the hardware of the information processor 500 and executes various processes by executing a computer program stored in the memory 502. The memory 502 is configured with, for example, an SSD or HDD. The memory 502 stores a configuration file 502a that includes information about the surgical instrument 120. The configuration file 502a is described later with reference to FIG. 11. The communication unit 503 includes a communication interface that is capable of communicating with the viewing terminal 400 and the control apparatus 3 of the surgical robot 4 based on a predetermined communication standard, such as Ethernet or Wi-Fi, for example.

FIG. 9 is a diagram illustrating an overview of connections between the surgical robot 4, the information processor 500, etc. and information transmitted and received between each apparatus.

In an operating room of a medical facility, the arm apparatus 1, the operation apparatus 2, the control apparatus 3, the video processor 21, and the viewing terminal 400 are arranged. Three surgical instruments 120 and one endoscope 130 are attached to the arm apparatus 1, as described above. The video processor 21 processes a video obtained by the endoscope 130 and transmits the processed video to the operation apparatus 2. The operation apparatus 2 displays an endoscope image received from the video processor 21 on the viewer 211 (see FIG. 4). The information processor 500 is installed in an environment outside the operating room. The information processor 500 is installed, for example, in a support center of the surgical robot 4. The information processor 500 is communicatively connected to the control apparatus 3 and the viewing terminal 400.

The operation apparatus 2 and the viewing terminal 400 do not necessarily have to be installed in the operating room and may be installed in other rooms in the medical facility. The viewing terminal 400 may be installed in a support center. The information processor 500 is not limited to being installed in a support center, but may also be installed in a data center or other location. Also, the information processor 500 may be a computer that provides cloud computing, or may be a computer that provides the on-premise.

As described above, an operator, a physician, operates the operation apparatus 2 to drive the arm apparatus 1 to perform surgery. At this time, the operation apparatus 2 transmits a driving instruction in response to the operator's operation to the control apparatus 3 in real time. The control apparatus 3 converts the drive instruction received from the operation apparatus 2 into a command value for the arm apparatus 1, and transmits the generated command value to the arm apparatus 1 in real time, i.e., each time the control apparatus 3 receives the drive instruction from the operation apparatus 2. The arm apparatus 1 is driven based on the received command value.

The arm apparatus 1 reads the memory information 124a from the memory 124 of each surgical instrument 120 that is attached. The memory information 124a includes identification information for individually identifying the surgical instrument 120 and information regarding the usage status of the surgical instrument 120. The arm apparatus 1 transmits the identification information for individually identifying the surgical instrument 120 and the information on the usage status of the surgical instrument 120 to the control apparatus 3. The control apparatus 3 transmits the identification information for individually identifying the surgical instrument 120 and the information on the usage status of the surgical instrument 120 received from the arm apparatus 1 to the information processor 500.

The information processor 500 updates the configuration file 502a stored in the memory 502 based on the identification information for individually identifying the surgical instrument 120 and the information on the usage status of the surgical instrument 120 received from the control apparatus 3. Also, when a judgment flag in the configuration file 502a, which is described below, is updated, the information processor 500 transmits an update instruction including a value of the judgment flag to the arm apparatus 1 via the control apparatus 3. The arm apparatus 1 updates the judgment flag in the memory information 124a based on the update instruction received from the control apparatus 3. Furthermore, the information processor 500 transmits information on various screens to the viewing terminal 400 in response to a request from the viewing terminal 400.

FIG. 10 is a diagram schematically illustrating an item included in the memory information 124a stored in the memory 124 of the surgical instrument 120.

The memory 124 of the surgical instrument 120 includes a memory area 124b for storing, in association with the information about the surgical instrument 120, information about the usage status of the surgical instrument 120 used in the surgery by the surgical robot 4 and a judgment result indicating non-usability determined for the surgical instrument 120 whose usage status is within the usage limit. The memory area 124b may be a fixed memory area or a variable memory area. Also, the memory area 124b may be a contiguous memory area or a distributed memory area.

The memory information 124a includes items of a surgical instrument ID, type, maximum number of uses, current number of uses, and judgment flag for the surgical instrument 120 for which the memory information 124a is stored. The surgical instrument ID, type, maximum number of uses, and current number of uses are information about the surgical instrument 120 and are associated with the judgment flag. The current number of uses is information about the usage status of the surgical instrument 120.

Information about the surgical instrument 120 does not need to be all of the following: surgical instrument ID, type, maximum number of uses, and current number of uses, but may include information that can identify an individual or a specific group of the surgical instrument 120. For example, the information about the surgical instrument 120 may be the surgical instrument ID, and the information about the surgical instrument 120 may include information other than the surgical instrument ID, type, maximum number of uses, and current number of uses. Information that can identify a particular group of the surgical instrument 120 may be, for example, a lot number to identify a particular manufacturing lot, a company identification number to identify a particular manufacturer, etc.

The surgical instrument ID is an ID that can individually identify the surgical instrument 120. The surgical instrument ID is also referred to as a serial number. In the memory information 124a, the type, the maximum number of uses, the current number of uses, and the judgment flag are associated based on the surgical instrument ID. The memory information 124a may include a lot number to identify the manufacturing lot.

The type is the type of the surgical instrument 120, and indicates a function that the surgical instrument 120 has and the type of the end effector 122. The type is, for example, forceps A, forceps B, forceps C, high frequency knife A, high frequency knife B, high frequency knife C, etc. The maximum number of uses is the number of times the surgical instrument 120 can be used from new, e.g., 10 times. The current number of uses is the cumulative number of times the surgical instrument 120 has been used up to the present time. The current number of uses is the number of surgeries in which the surgical instrument 120 is used, and is counted as one when used in one surgery. Therefore, the current number of uses is counted as one even if the instrument is attached and removed and used multiple times in a single surgery.

The judgment flag is information indicating whether the surgical instrument 120 can be used continuously in the future. In case that the judgment flag is 0, the judgment flag indicates that the surgical instrument 120 is ready for use, and in case that the judgment flag is 1, the judgment flag indicates that the surgical instrument 120 is unavailable for use. The initial value of the judgment flag is 0.

FIG. 11 is a diagram schematically illustrating a configuration of the configuration file 502a stored in the memory 502 of the information processor 500.

The configuration file 502a stores information about a plurality of the surgical instruments 120. The configuration file 502a includes items of the surgical instrument ID, type, maximum number of uses, current number of uses, and judgment flag. For each surgical instrument ID, the type, maximum number of uses, current number of uses, and judgment flag are associated. As with the judgment flag in the memory information 124a, the judgment flag of 0 indicates that the surgical instrument 120 is ready for use, while the judgment flag of 1 indicates that the surgical instrument 120 is unavailable for use. The initial value of the judgment flag is 0.

For the surgical instrument 120 with a surgical instrument ID "SI001" in FIG. 11, the judgment flag value is 0 because the current number of uses has not reached the maximum number of uses, and the disablement of the surgical instrument 120 is not set by an operator, as described below. For the surgical instrument 120 with a surgical instrument ID "SI002" in the second line of FIG. 11, the current number of uses has not reached the maximum number of uses, but the value of the judgment flag is 1 because the operator sets that the surgical instrument 120 is not available for use. For the surgical instrument 120 with a surgical instrument ID "SI003" in the third row of FIG. 11, the current number of uses has reached the maximum number of uses, so the value of the judgment flag is 1.

In case that the information processor 500 is connected to multiple medical facilities via the network 10, the configuration file 502a for each medical facility may be stored in the memory 502 of the information processor 500. Alternatively, information for identifying each medical facility may be stored in the common configuration file 502a in association with a surgical instrument ID. Therefore, a process of searching for the surgical instrument 120 with a specific surgical instrument ID in the memory 502 can be speeded up.

FIG. 12 is a flowchart illustrating a process by the controller 141 of the arm apparatus 1.

A flow illustrated in FIG. 12 begins when the controller 141 detects that the surgical instrument 120 is attached to the arm 110. After a process illustrated in FIG. 12 is started, when other surgical instrument 120 is attached, steps S101 to S106 are performed for the other surgical instrument 120. Also, when a plurality of the surgical instruments 120 are attached at the start of the process illustrated in FIG. 12, steps S101 to S106 are performed for each of the surgical instruments 120 in turn.

When the surgical instrument 120 is attached, in step S101, the controller 141 determines whether the attached surgical instrument 120 is the first one attached within the same surgery.

When the surgical instrument 120 is attached for the first time, in step S102, the controller 141 queries the information processor 500 via the control apparatus 3 for the configuration file 502a. Specifically, the controller 141 reads the memory information 124a from the memory 124 of the surgical instrument 120 that is installed, and obtains the surgical instrument ID contained in the read memory information 124a. Then, the controller 141 transmits the query information of the configuration file 502a including the obtained surgical instrument ID to the information processor 500, and obtains the contents of the configuration file 502a corresponding to the surgical instrument ID from the information processor 500.

In step S103, the controller 141 updates the memory information 124a stored in the memory 124 of the surgical instrument 120 based on the contents of the configuration file 502a of the surgical instrument 120 obtained by the inquiry of step S102. Specifically, the controller 141 updates a value of the judgment flag in the memory information 124a to match a value of the judgment flag in the configuration file 502a.

In step S104, the controller 141 determines whether the value of the judgment flag updated by the inquiry of step S102 is 0, that is, whether the surgical instrument 120 can be used continuously in the future. The surgical instrument 120, for which the value of the judgment flag is determined to be 1 in step S104, is judged to be applied for the first time in step S101, even if the surgical instrument 120 is applied again in the same surgery. Therefore, the surgical instrument 120 that is disabled from being used by mistake is prevented since the process proceeds to steps S104 and S105.

In case that the value of the judgment flag obtained by the inquiry of step S102 is 1, the controller 141 outputs an alert in step S105. The output of an alert includes, for example, displaying an alert screen on the display of the operating terminal 102 (see FIG. 2) of the arm apparatus 1, outputting an alarm sound from a speaker installed in the arm apparatus 1, turning on a lamp installed in the arm apparatus 1, sending alert information to other apparatus, etc. After outputting the alert, the process is returned to step S101 by inputting a cancellation of the alert or by the elapse of a predetermined time.

In the embodiment, the processes of steps S102 to S105 are performed when the judgment result of step S101 is positive, but the invention is not limited to this and may be performed periodically, for example, every predetermined period (e.g., every second). In this way, even if an unavailable button 614 on a judgment screen 610 (described below) is operated at any timing during the surgery, an alert is output from the arm apparatus 1, so that the operator, a physician, can easily recognize that the surgical instrument 120 that is the subject of the unavailable designation is unavailable for use. On the other hand, when the operator does not like the output of the alert due to the surgical instrument 120 that is subject to the designation of unavailable, the process of step S105 may be omitted.

On the other hand, in case that the value of the judgment flag updated by the inquiry of step S102 is 0, in step S106, the controller 141 increases the current number of uses in the memory information 124a by 1.

In case that the surgical instrument attached is determined not to be the first one attached within the same surgery, the process proceeds to step S107.

The controller 141, as illustrated in a connector A1, receives the command value transmitted from the control apparatus 3 in step S201 of FIG. 13. Then, in step S107, the controller 141 operates the arm 110 and the surgical instrument 120 based on the command value from the control apparatus 3. Subsequently, as illustrated in a connector A2, in case that of a notification the end of the surgery is sent from the control apparatus 3 in step S203 of FIG. 13, the controller 141 receives the notification. Then, in step S108, the controller 141 determines whether the notification of the end of surgery is received from the control apparatus 3.

In case that the end-of-surgery notification is not received, the process is returned to step S101, and steps S101 to S107 are repeated. In case that the end-of-surgery notification is received, in step S109, the controller 141 obtains information about the surgical instrument 120 in the memory information 124a and transmits information about the usage status of the surgical instrument 120 to the control apparatus 3, as illustrated in a connector B1, by associating the information with the surgical instrument ID. The information on the usage status of the surgical instrument 120 according to Embodiment 1 is the current number of uses. In case that a plurality of the surgical instruments 120 are attached to the arm apparatus 1, the process of step S109 is performed for each surgical instrument 120 in turn.

Then, in step S110, the controller 141, as illustrated in a connector A3, determines whether the controller 141 receives an update instruction sent from the control apparatus 3 in step S207 of FIG. 13. The update instruction is an instruction to update the value of the judgment flag in the memory information 124a to 1, and includes the surgical instrument ID. Upon receipt of the update instruction, in step S111, the controller 141 updates the memory information 124a of the corresponding surgical instrument 120 so that the value of the judgment flag becomes 1.

In case that the target surgical instrument 120 is already removed from the arm apparatus 1 at the time when step S111 is performed, the memory information 124a of the surgical instrument 120 is not updated in step S111. In this case, when the surgical instrument 120 is attached to the arm apparatus 1 in the next surgery, the memory information 124a is updated to the latest judgment flag value in step S103.

FIG. 13 is a flowchart illustrating a process by a controller 301 of the control apparatus 3.

When the surgery is started, in step S201, the controller 301 transmits the command value to the arm apparatus 1 as illustrated in the connector A1 based on a drive instruction from the operation apparatus 2. In step S202, the controller 301 determines whether the operator inputs the end of surgery via the operation apparatus 2. In case that the end of surgery is not input, the process is returned to step S201, and step S201 is repeated. As a result, the arm apparatus 1 is continuously driven based on the drive instruction from the operation apparatus 2. In case that the end of surgery is input, in step S203, the controller 301 transmits a notice of the end of surgery to the arm apparatus 1, as illustrated in the connector A2.

In step S204, the controller 301 waits for the process until the controller 301 receives the information on the usage status of the surgical instrument 120 transmitted from the arm apparatus 1 in step S109 of FIG. 12, as illustrated in the connector B1. Upon receiving the information on the usage status of the surgical instrument 120, in step S205, the controller 301 transmits the received information on the usage status to the information processor 500 by associating the information with the surgical instrument ID, as illustrated in a connector C1.

In step S206, the controller 301 waits for the process until the controller 301 receives the update instruction transmitted from the information processor 500 in step S308 of FIG. 14, as illustrated in a connector B2. Upon receiving the update instruction, in step S207, the controller 301 transmits the received update instruction to the arm apparatus 1, as illustrated in the connector A3.

FIG. 14 is a flowchart illustrating a process by a controller 501 of the information processor 500. The following steps S301 to S308 are performed for one target surgical instrument 120.

In step S301, the controller 501 determines whether the information on the usage status of the surgical instrument 120 transmitted from the control apparatus 3 in step S205 of FIG. 13 is received, as illustrated in the connector C1. The information on the usage status of the surgical instrument 120 is the current number of times the surgical instrument 120 is used, as described above. The received information on the usage status is assigned a surgical instrument ID. When the controller 501 receives the information on the usage status, the controller 501 performs steps S302 to S304, and when the information on the usage status is not received, the controller 501 proceeds to step S305.

In step S302, the controller 501 updates the configuration file 502a based on the received information on the usage status of the surgical instrument 120. Specifically, in the configuration file 502a, the controller 501 updates the current usage count corresponding to the surgical instrument ID with the received current usage count.

In step S303, the controller 501 determines whether the usage status of the surgical instrument 120 reaches the usage limit. Specifically, the controller 501 determines whether the current number of uses in the configuration file 502a is greater than or equal to the maximum number of uses. In case that the current number of uses is greater than or equal to the maximum number of uses, the controller 501 executes the process in step S304, and in case that the current number of uses is less than the maximum number of uses, the process proceeds to step S305. In step S304, the controller 501 sets the value of the judgment flag corresponding to the surgical instrument ID to 1 in the configuration file 502a. As a result, the surgical instrument ID is associated with the judgment flag value in the configuration file 502a.

In step S305, the controller 501 determines whether a display instruction of a judgment screen 610 (see FIGS. 16 and 17) of the surgical instrument 120 is received from the viewing terminal 400. The display instruction of the judgment screen 610 is given by specifying the surgical instrument ID. In case that the controller 501 accepts the display instruction of the judgment screen 610, the controller 501 executes the process of step S306, and in case that the controller 501 does not accept the display instruction of the judgment screen 610, the controller 501 proceeds to step S307. In step S306, the controller 501 executes a judgment screen display process. The judgment screen display process is described later with reference to FIG. 15. Since the controller 501 continuously executes the process of steps S301 to S308, the operator can input the display instruction of the judgment screen at any timing.

In step S307, the controller 501 determines whether the value of the judgment flag in the configuration file 502a is 1. In case that the value of the judgment flag is 1, the controller 501 executes the process of step S308, and in case that the value of the judgment flag is 0, the controller returns the process to step S301. In step S308, the controller 501 transmits an update instruction to the control apparatus 3 as illustrated in the connector B2. The update instruction transmitted in step S308 is an instruction to update the value of the judgment flag in the memory information 124a to 1, and includes the surgical instrument ID of the surgical instrument 120 whose value of the judgment flag is set to 1.

With the transmission of the update instruction in step S308, in step S111 of FIG. 12, the controller 141 of the arm apparatus 1 updates the memory information 124a of the target surgical instrument 120 so that the value of the judgment flag becomes 1. As a result, the surgical instrument ID is associated with the judgment flag value in the memory information 124a.

FIG. 15 is a flowchart illustrating the details of a judgment screen display process in step S306 of FIG. 14.

In step S3061, the controller 501 of the information processor 500 determines whether the usage status of the target surgical instrument 120 is within the usage limit based on the surgical instrument ID specified in the display instruction received in step S305 of FIG. 14. Specifically, the controller 501 determines whether the current number of uses in the configuration file 502a is less than the maximum number of uses.

When the usage status is within the usage limit, in step S3062, the controller 501 determines whether the value of the judgment flag of the target surgical instrument 120 in the configuration file 502a is 0. In case that the value of the judgment flag is 0, in step S3063, the controller 501 transmits information on the judgment screen 610 indicating that the surgical instrument 120 is ready for use to the viewing terminal 400. When the controller 401 of the viewing terminal 400 receives the information of the judgment screen 610, the controller 401 displays the judgment screen 610 illustrated in an upper part of FIG. 16 on the display 403. When the unavailable button 614 in the judgment screen 610 illustrated in the upper part of FIG. 16 is operated, the controller 401 of the viewing terminal 400 transmits information indicating that the unavailable button 614 is operated to the information processor 500.

In step S3064, the controller 501 of the information processor 500 determines whether information indicating that the unavailable button 614 is operated on the judgment screen 610 illustrated in the upper part of FIG. 16 is received. In case that the unavailable button 614 is operated, in step S3065, the controller 501 updates the value of the judgment flag of the target surgical instrument 120 in the configuration file 502a to 1, thereby associating the information about the surgical instrument 120 with the judgment result indicating unavailable.

On the other hand, in case that it is determined in step S3061 that the usage status reaches the usage limit and in case that it is determined in step S3062 that the value of the judgment flag is 1, the process proceeds to step S3066. In step S3066, the controller 501 transmits information on the judgment screen 610 indicating that the surgical instrument 120 is not available to the viewing terminal 400. When the controller 401 of the viewing terminal 400 receives the information of the judgment screen 610, the controller 401 causes the display 403 to display the judgment screen 610 illustrated in a lower part of FIG. 16 or FIG. 17.

FIGS. 16 and 17 are diagrams schematically illustrating configurations of the judgment screen 610 displayed on the display 403 of the viewing terminal 400.

The judgment screen 610 illustrated in FIGS. 16 and 17 includes a surgical instrument information display area 611, a usage status display area 612, and a usability display area 613. The judgment screen 610 illustrated in the upper part of FIG. 16 is further provided with the unavailable button 614. The surgical instrument information display area 611 displays the type of surgical instrument 120 and the surgical instrument ID are displayed. The usage status display area 612 displays the current number of uses and maximum number of uses of the surgical instrument 120 in a form of a numerator and denominator of a fraction, respectively.

As illustrated in the upper part of FIG. 16, the judgment screen 610 indicating that the surgical instrument 120 is ready for use is the screen displayed when the usage status is determined to be within the usage limit in step S3061 in FIG. 15 and the judgment flag value is determined to be 0 in step S3062. In this case, "Available" is displayed in the usability display area 613. When the operator of the viewing terminal 400 operates the unavailable button 614, step S3065 in FIG. 15 is executed on the information processor 500, and the value of the judgment flag of the target surgical instrument 120 in the configuration file 502a is set to 1.

The operator, a physician operating the operation apparatus 2, operates the unavailable button 614 on the judgment screen 610 illustrated in the upper part of FIG. 16 when the operator feels that the surgical instrument 120 becomes less sharp, when the operator feels discomfort in operating the surgical instrument 120, when the operator feels that the surgical instrument 120 may be faulty, etc. Alternatively, the operator of the operation apparatus 2 instructs the operator of the viewing terminal 400 to operate the unavailable button 614. As a result, the surgical instrument 120 whose number of uses has not reached the maximum number of uses is set to be unavailable.

As illustrated in the lower part of FIG. 16, the judgment screen 610 indicating that unavailable is set for use due to the number of uses is the screen that is displayed when the usage status is determined to have reached the usage limit in step S3061 in FIG. 15. In this case, the usability display area 613 indicates that the current number of uses of the surgical instrument 120 has reached the maximum number of uses. In the usability display area 613, "Not available (maximum number of uses)" is displayed.

As illustrated in FIG. 17, the judgment screen 610 indicating that unavailability is set for use by the operator is the screen displayed when the usage status is determined to be within the usage limit in step S3061 in FIG. 15 and the judgment flag value is 1 in step S3062. In this case, "Not available (operator's judgment)" is displayed in the usability display area 613. The judgment screen 610 illustrated in FIG. 17 may also display an available button for setting the surgical instrument 120 that is once set to be unavailable to be available. In that case, when the available button is operated by the operator, the value of the judgment flag is changed from 1 to 0.

<Effects of a method of managing a surgical instrument, an information processor, a management system, a surgical instrument, and a surgical robot according to Embodiment 1>

In a method of managing the surgical instrument 120 that is reusable up to the usage limit (e.g., the maximum number of uses), the controller 501 of the information processor 500 obtains information on the usage status (e.g., the current number of uses) of the surgical instrument 120 used in a surgery by the surgical robot 4 (step S301 in FIG. 14), obtains a judgment result indicating that the surgical instrument 120 is not available (step S3064 in FIG. 15: YES) regarding the surgical instrument 120 whose usage status is within the usage limit (step S3061 in FIG. 15: YES), and associates information about the surgical instrument 120 (e.g., surgical instrument ID) with the judgment result (step S3065 in FIG. 15).

According to the process, even if the surgical instrument 120 is within the usage limit, the judgment result indicating that the surgical instrument 120 is not available can be managed in association with the information about the surgical instrument 120. Therefore, a uniform judgment is prevented based on a single condition, such as whether a surgical instrument is within the usage limit, thereby enhancing the safety of surgery.

In case that the surgical instrument 120 is used up to the usage limit (e.g., the maximum number of uses) (step S303 in FIG. 14: YES), the surgical instrument 120 is determined to be unavailable (step S304 in FIG. 14).

According to the process, even for the surgical instrument 120 that is used up to the usage limit, the use of the surgical instrument 120 can be properly managed.

The controller 141 of the arm apparatus 1 outputs an alert (step S105 in FIG. 12) when the surgical instrument 120 that is determined to be unavailable (the value of the judgment flag is 1) is attached to the surgical robot 4.

According to the process, the surgical instrument 120 that is determined to be unavailable (e.g., the surgical instrument 120 that should not be used even if the surgical instrument 120 is within the expiration date) can be prevented from being used in surgery, thereby enhancing the safety of surgery.

In case that the surgical instrument 120 determined to be unavailable is attached to the surgical robot 4, step S104 of FIG. 12 determines to be NO, and the process does not proceed to step S106 or later. As a result, the operation of the surgical robot 4 is prohibited until the surgical instrument 120 determined to be unavailable is replaced with the available surgical instrument 120.

According to the process, the surgical instrument 120 that is determined to be unavailable is prevented from being mistakenly used in surgery, thereby enhancing the safety of surgery.

When the controller 501 of the information processor 500 receives an instruction from the operator to disable the surgical instrument 120 via the unavailable button 614 in FIG. 16, the controller 501 obtains the judgment result indicating that the instrument is not available (step S3064: YES).

According to the process, the surgical instrument 120 can be flexibly set to be unavailable for use according to the instruction from the operator. Therefore, in case that the usage of the surgical instrument 120 does not exceed the usage limit, the operator of the surgical robot 4 can disable the surgical instrument 120 at his/her discretion, for example, when the operator feels that the sharpness of the surgical instrument 120 is deteriorated, when the operator feels uncomfortable operating the surgical instrument 120, or when the operator feels that the surgical instrument 120 may be faulty.

On a display screen displaying information about the usage status (e.g., the judgment screen 610 illustrated in the upper part of FIG. 16), the instruction to disable the use of the surgical instrument 120 is accepted.

According to the process, the instruction to disable the use of the surgical instrument 120 can be given with reference to its usage status.

The controller 501 of the information processor 500 transmits the information of the judgment screen 610 in the lower part of FIG. 16 and of FIG. 17 indicating that the surgical instrument 120 is not available to the viewing terminal 400 (step S3066 in FIG. 15).

According to the process, at any given time, such as before using the surgical instrument 120, the operator can know that the surgical instrument 120 should be refrained from using. Thus, the safety of surgery can be enhanced.

The information indicating that the surgical instrument 120 is unavailable displayed on the judgment screen 610 may be a message indicating that the surgical instrument 120 is unavailable as illustrated in the lower part of FIG. 16 and FIG. 17, as well as information such as warnings or information indicating that the surgical instrument 120 needs to be replaced or maintained, etc.

The information processor 500 is installed in a second facility (e.g., support center in FIG. 9) different from the first facility (e.g., medical facility with an operating room in FIG. 9) where the surgical robot 4 is installed, and the communication unit 503 of the information processor 500 communicates with the surgical robot 4 installed in the first facility.

According to the configuration, the information processor 500 installed in the second facility can be used to manage the surgical instrument 120 in the surgical robot 4 installed in the first facility.

<Variation of Embodiment 1>

The configuration file 502a of Embodiment 1, as illustrated in FIG. 11, includes the current number of times the surgical instrument 120 has been used as information on the usage status of the surgical instrument 120, but may include other information indicating a degree to which the surgical instrument 120 has been used to date, instead of or in addition to the current number of uses.

For example, the configuration file 502a, as information of the usage status of the surgical instrument 120, may include the cumulative time attached to the arm 110 (hours of use), the cumulative number of days attached to the arm 110 (days of use), the cumulative duration of power supply to the surgical instrument 120, the cumulative number of times the power supply to the surgical instrument 120 has been turned on and off, the time since the surgical instrument 120 is last used, the number of surgeries in which the surgical instrument 120 is used, the number of operations of the surgical instrument 120 (e.g., the number of cutting operations in the case of scissors-type forceps), and the number of times the surgical instrument 120 is attached to and detached from the arm 110.

When other information is stored in the configuration file 502a, other information may be also stored in the memory information 124a of the surgical instrument 120 illustrated in FIG. 10. The controller 141 of the arm apparatus 1 updates the other information in the memory information 124a at step S106 of FIG. 12 or at the timing when the surgical instrument 120 is removed. Then, in step S109, the controller 141 transmits the other information to the information processor 500 via the control apparatus 3 as information on the usage status of the surgical instrument 120.

When the controller 501 of the information processor 500 receives other information as information on the usage status of the surgical instrument 120 in step S301 of FIG. 14, the controller 501 updates the configuration file 502a based on the received other information in step S302. Then, in step S303, the controller 501 determines whether the usage status based on the other information is within the usage limit.

FIG. 18 is a diagram schematically illustrating a configuration of the configuration file 502a when the cumulative time attached to the arm 110 (usage time) is stored. In this case, the configuration file 502a stores the usage time of the surgical instrument 120 for each surgical instrument ID. The controller 501 of the information processor 500 determines in step S303 of FIG. 14 and step S3061 of FIG. 15 whether the current number of uses is less than the maximum number of uses and whether the usage time is less than the maximum usage time. The maximum usage time may be included in the configuration file 502a in advance or stored in the memory 502 of the information processor 500.

FIG. 19 is a diagram schematically illustrating the judgment screen 610 when the cumulative time attached to the arm 110 (usage time) is stored. An upper part of FIG. 19, like the upper part of FIG. 16, is a diagram illustrating a judgment screen 610 indicating availability for use, and a lower part of FIG. 19, like the lower part of FIG. 16, is a diagram illustrating the judgment screen 610 indicating unavailability for use due to time of use.

In the upper part and the lower part of the judgment screen 610 in FIG. 19, the usage status display area 612 indicates the time of use. In the upper part of FIG. 19, "Available" is displayed in the usability display area 613 because both the number of uses and the time of use are less than the limit. In the lower part of FIG. 19, "Not available (usage time limit)" is displayed in the usability display area 613 because the number of uses is less than the maximum number of uses, but the usage time is greater than the maximum use time (e.g., 10 hours) or equal.

In the following embodiments and variations, the usage status information may be other information indicating the degree of use of the surgical instrument 120.

<Embodiment 2>

In Embodiment 1, when the operator operates the unavailable button 614, the surgical instrument 120 is set to be unavailable even if the usage status is within the usage limit. In contrast, in Embodiment 2, when the possibility of a failure of the surgical instrument 120 is detected based on information on an operation status of the surgical instrument 120, the surgical instrument 120 is disabled in the same way. In other words, while in Embodiment 1, the disablement is set by the operator, in Embodiment 2, the disablement is further set automatically by the information processor 500.

FIG. 20 is a block diagram illustrating the arm apparatus 1 and the surgical instrument 120 according to Embodiment 2.

The arm 110 of the arm apparatus 1 of Embodiment 2 includes a plurality of actuators 150, a terminal 171, and a plurality of rotary drives 172. Although the arm apparatus 1 includes four arms 110 as described above, one arm 110 is illustrated in FIG. 20 for convenience. Also, in FIG. 20, the memory 142, the communication unit 143, and plurality of sensors 161 of the arm apparatus 1 are omitted for convenience.

One arm 110 includes one terminal 171, four rotary drives 172, and a plurality of actuators 150. One actuator 150 includes a motor 151 and an encoder 152 that outputs a drive amount of the motor 151. The motor 151 of the actuator 150 corresponding to the rotary drive 172 drives the rotary drive 172. The terminal 171 is connected to the controller 141.

In FIG. 20, the surgical instrument 120, grasping forceps, is illustrated for convenience. The surgical instrument 120 illustrated in FIG. 20 includes a terminal 126, four drive transmission members 127, and four shafts 128. The terminal 126 is connected to the memory 124. The four shafts 128 are each connected to the drive transmission member 127.

When the surgical instrument 120 is attached to the arm 110, the terminal 171 of the arm 110 and the terminal 126 of the surgical instrument 120 are electrically connected to each other. As a result, the controller 141 of the arm apparatus 1 can read the memory information 124a from the memory 124 and update the memory information 124a via the terminals 171 and 126.

Also, when the surgical instrument 120 is attached to the arm 110, the four rotary drives 172 of the arm 110 and the four drive transmission members 127 of the surgical instrument 120 are mechanically connected. In this state, when the motor 151 corresponding to the rotary drive 172 is driven, a driving force of the motor 151 is transmitted to the shaft 128 via the rotary drive 172 and the drive transmission member 127, and the shaft 128 is driven. At this time, the drive amount output by the four encoders 152 corresponding to the four shafts 128 of the surgical instrument 120 reflects a movement of each shaft 128 of the surgical instrument 120, i.e., the actual movement of the surgical instrument 120.

As described in Embodiment 1, the control apparatus 3 converts a drive instruction received from the operation apparatus 2 for the arm apparatus 1, generates a command value, and transmits the generated command value to the arm apparatus 1. In Embodiment 2, the controller 141 of the arm apparatus 1 drives each motor 151 based on the command value received from the control apparatus 3. At this time, the controller 141 transmits the drive amount output from the encoder 152 of the actuator 150 connected to the rotary drive 172 to the control apparatus 3 as a current value. The controller 301 of the control apparatus 3 transmits the command value and the current value corresponding to the command value to the information processor 500. The controller 501 of the information processor 500 determines the possibility of a failure of the surgical instrument 120 based on the received command value and the current value.

FIG. 21 is a flowchart illustrating a process by the controller 141 of the arm apparatus 1 according to Embodiment 2.

Compared to the process of Embodiment 1 illustrated in FIG. 12, a process in FIG. 21 has an additional step S121 at the end of step S107. The following is an explanation of the process that differs from that of Embodiment 1.

In step S107, the controller 141 drives the motor 151 based on the command value received from the control apparatus 3 to operate the arm 110 and the surgical instrument 120. In step S121, the controller 141 transmits the drive amount output from the encoder 152 of the actuator 150 connected to the rotary drive 172 to the control apparatus 3 as the current value, as illustrated in a connector B3. The controller 141 repeats the process of steps S101 to S121 until an end-of-operation notification is received. Thereby, the transmission of the current value in step S121 is repeated while the surgery is being performed. The transmission of step S121 is performed at intervals of 1 second, for example.

FIG. 22 is a flowchart illustrating a process by the controller 301 of the control apparatus 3 according to Embodiment 2.

Compared to the process of Embodiment 1 illustrated in FIG. 13, a process in FIG. 22 has steps S211 and S212 added after step S201. The following is an explanation of the process that differs from that of Embodiment 1.

In step S211, the controller 301 receives the current value transmitted in step S121 of FIG. 21, as illustrated in the connector B3. Subsequently, in step S212, the controller 301 associates the current value received in step S211 with the command value corresponding to the current value, as illustrated in a connector C2, and transmits the current value to the information processor 500. The controller 301 repeats the process of steps S201 to S212 until the operator inputs the end of the surgery. Thereby, the transmission of the current value and the command value in step S212 is repeated while the surgery is being performed. The transmission of step S212 is performed at intervals of 1 second, for example.

FIG. 23 is a flowchart illustrating a process by the controller 501 of the information processor 500 according to Embodiment 2.

Compared to the process of Embodiment 1 illustrated in FIG. 14, a process in FIG. 23 has steps S311 and S312 added after step S304. The following is an explanation of the process that differs from that of Embodiment 1.

The controller 501 receives the current value and the command value transmitted in step S212 of FIG. 22 and stores the current value and the command value in the memory 502 of the information processor 500 as needed, as illustrated in the connector C2. The current value and the command value transmitted in step S212 are information about an operation status of the surgical instrument 120. Also, as illustrated in a connector C3, when a notice of the end of surgery is transmitted from the control apparatus 3 in step S203 of FIG. 22, the controller 501 receives the notice of the end of surgery. Then, in step S311, the controller 141 determines whether the notice of the end of surgery is received from the control apparatus 3. In case that the end-of-surgery notification is received, in step S312, the controller 501 executes an automatic detection process illustrated in FIG. 24.

FIG. 24 is a flowchart illustrating the details of an automatic detection process in step S312 of FIG. 23. The process in FIG. 24 is performed for each surgical instrument 120 based on the current value and the command value stored in the memory 502 during one surgery.

In step S3121, the controller 501 of the information processor 500 determines whether the usage status of the target surgical instrument 120 is within the usage limit. In case that the usage status is not within the usage limit, steps S3122 to S3127 are skipped.

In case that the usage status is within the usage limit, in step S3122, the controller 501 determines whether the value of the judgment flag of the target surgical instrument 120 in the configuration file 502a is 0. In case that the value of the judgment flag is 0, the process proceeds to step S3123, and in case that the value of the judgment flag is 1, steps S3123 to S3127 are skipped.

In step S3123, the controller 501 calculates the difference between a pair of the current value and the command value. When the surgical instrument 120 operates normally according to the command value, the difference between the current value and the command value is usually zero. In step S3124, the controller 501 calculates an average value of the plurality of differences calculated in step S3123 for each shaft 128 (see FIG. 20). In step S3125, the controller 501 stores the average value of the differences calculated in step S3124 for each shaft 128 in the memory 502 by associating it with the current number of uses of the surgical instrument 120.

In step S3126, the controller 501 determines whether there is the shaft 128 for which the difference between the average value of the previous difference and the average value of the current difference is greater than a threshold value. In case that the above difference is greater than the threshold value in one or more shafts 128 out of four, in step S3127, by updating the configuration file 502a of the target surgical instrument 120 so that the value of the judgment flag becomes 1, the controller 501 associates the information about the surgical instrument 120 with the judgment result indicating unavailability for use. On the other hand, in case that the above difference is less than the threshold value in all shafts 128, the process of step S3127 is skipped.

FIG. 25 is a diagram schematically illustrating the details of a calculation process in the automatic detection process.

As illustrated in an upper part of FIG. 25, the memory 502 stores the received current value and command value along the time series for each of the shafts 128. In step S3123 of FIG. 24, the difference between the current value and the command value, which are pairs received simultaneously, is calculated for each pair. In this way, a plurality of differences along the time series are obtained for each of the shafts 128.

As illustrated in the middle of FIG. 25, in step S3124 of FIG. 24, for each shaft 128, a plurality of differences along the time series are averaged and an average value of the differences is calculated. In step S3125 of FIG. 24, the average value of the differences for each shaft 128 is stored in association with the current number of times the surgical instrument 120 is used. Thereby, as illustrated in a lower table in FIG. 25, the average value of the differences is stored for each shaft 128 in correspondence with the number of uses.

In step S3126 of FIG. 24, the difference between the average value of the current difference and the average value of the previous difference is obtained in each shaft 128, and whether the difference is greater than a threshold value is determined. In an example illustrated in a lower part of FIG. 25, the difference 0.004 between the average value 0.005 of the current difference (number of use = 5) and the average value 0.001 of the previous difference (number of use = 4) is obtained, and whether the difference 0.004 is greater than the threshold value stored in the memory 502 in advance is determined. The threshold value used in step S3126 is set in advance for each type of the surgical instrument 120 and the shaft 128 and stored in the memory 502. Thus, when the difference between the average value of the current difference and the average value of the previous difference is used for the determination, the possibility of a failure of the surgical instrument can be detected.

In the embodiment, the average of the differences is calculated for each surgery, but for example, the average of the differences may be calculated for each predetermined period within a single surgery. Also, in the embodiment, the automatic detection process is performed after the surgery is completed, but the automatic detection process may be performed during the surgery. Furthermore, the automatic detection process may be performed multiple times during the surgery, e.g., every predetermined period of time (e.g., every second).

Next, the judgment screen 610 displayed in Embodiment 2 is described.

A judgment screen display process in step S306 of Embodiment 2 illustrated in FIG. 23 is the same as the judgment screen display process of Embodiment 1 illustrated in FIG. 15. In Embodiment 2, in step S3063 of FIG. 15, the judgment screen 610 illustrated in the upper part of FIG. 16 is displayed as in Embodiment 1. Also, in step S3066 of FIG. 15, the judgment screen 610 illustrated in FIG. 26 is displayed as the judgment screen 610 when the usage status is determined to be within the usage limit and the value of the judgment flag is 1.

In the judgment screen 610 of FIG. 26, "Not available" is displayed in the usability display area 613, and unlike the judgment screen 610 of FIG. 17, a reason for the unavailability is not displayed in the usability display area 613. The reason for the above is that the value of the judgment flag is set to 1 in the case where unavailability is set by the operator and in the case where unavailability is set by the automatic detection process, and the distinction between the two is not stored. In contrast, an example in which a flag that can distinguish between the two is stored in the configuration file 502a is explained later with reference to Embodiment 4.

<Effects of a method of managing a surgical instrument, an information processor, a management system, a surgical instrument, and a surgical robot according to Embodiment 2>

In Embodiment 2, the following effect is achieved in addition to that of Embodiment 1 above.

The controller 501 of the information processor 500 obtains information on the usage status of the surgical instrument 120 (e.g., the current number of uses) (step S301 in FIG. 23), obtains a judgment result indicating unavailable for use for the surgical instrument 120 whose usage status is within the usage limit (step S3121 in FIG. 24: YES) (step S3126 in FIG. 24: YES), and associate the information about the surgical instrument 120 (e.g., surgical instrument ID) with the judgment result (step S3127 in FIG. 24).

In this process, even if the surgical instrument 120 is within the usage limit, the judgment result indicating that the surgical instrument 120 is unavailable for use can be managed in association with the information about the surgical instrument 120. Therefore, a uniform judgment based on a single condition, such as whether the surgical instrument is within the usage limit is prevented, thereby enhancing the safety of surgery.

Obtaining the judgment result indicating unavailability can be determined based on at least one of a plurality of conditions. Specifically, the judgment result indicating unavailability can be obtained based on the receipt of an instruction from the operator via the unavailable button 614 to disable the surgical instrument 120 and the automatic detection of the unavailability by the information processor 500.

According to the configuration, the safety of surgery can be further enhanced.

The judgment result indicating unavailability can be obtained based on information about the operation status of the surgical instrument 120 (e.g., the current value and the command value).

According to the configuration, the surgical instrument 120 can be determined to be unusable flexibly according to the prediction of a failure of the surgical instrument 120. Thus, for example, in case that a possible failure, which is not visually noticed by the operator from the external appearance, is detected based on information about the operation status, the surgical instrument 120 can be made unavailable for use.

<Embodiment 3>

In Embodiment 3, compared to Embodiment 2, the process in which unavailability is set by the operator is omitted.

FIG. 27 is a flowchart illustrating a process by the controller 501 of the information processor 500 according to Embodiment 3.

A process in FIG. 27 omits steps S305 and S306 compared to the process in Embodiment 2 illustrated in FIG. 23. Therefore, in Embodiment 3, the unavailability of the surgical instrument 120 is not entered by the operator via the judgment screen 610.

In Embodiment 3, the process of FIG. 23 may be executed as in Embodiment 2, and information on the judgment screen 610 with the unavailable button 614 omitted may be transmitted to the viewing terminal 400 in S3063 of the judgment screen display process illustrated in FIG. 15. In this case, steps S3064 and S3065 are omitted in FIG. 15. Also, in case that the usage status is within the usage limit and the value of the judgment flag is determined to be 1, the case is limited to a case where the automatic detection process is set the instrument to be disabled for use. Therefore, the judgment screen 610 of FIG. 28 is displayed based on step S3066 of FIG. 15.

The judgment screen 610 in FIG. 28 displays "Not available (automatic detection)" in the usability display area 613.

In Embodiment 3, as in Embodiments 1 and 2, even if the surgical instrument 120 is within the usage limit, the judgment result indicating that the surgical instrument 120 are unavailable for use can be managed in association with the information about the surgical instrument 120.

<Embodiment 4>

In Embodiment 2, when unusable is set by the operator or the automatic detection process, the reason for the unusable is not displayed on the judgment screen 610, as illustrated in FIG. 26. In contrast, in Embodiment 4, the reason for the unusable is displayed on the judgment screen 610 even in the above case. In Embodiment 4, for example, the configuration file 502a is configured as illustrated in FIG. 29 to distinguish the reason for unavailability.

FIG. 29 is a diagram schematically illustrating the configuration file 502a according to Embodiment 4.

Compared to the configuration file 502a in FIG. 11, the configuration file 502a in FIG. 29 has an additional item for an unavailable factor. The item for the unavailable factor stores one of the following values: "001" indicating that the number of uses has reached the maximum number of uses, "010" indicating that the unavailability is set by the operator, and "011" indicating that the unavailability is set by the automatic detection process. In an initial state, the value of the unavailable factor item is "000".

In this case, the controller 501 of the information processor 500 sets 1 to the value of the judgment flag of the configuration file 502a and sets "001" to a value of the unavailable factor of the configuration file 502a in step S304 of FIG. 23. In step S3127 of the automatic detection process illustrated in FIG. 24, the controller 501 sets 1 to the value of the judgment flag of the configuration file 502a and also sets "011" to the value of the unavailable factor of the configuration file 502a. Also, in step S3065 of the judgment screen display process illustrated in FIG. 15, the controller 501 sets 1 to the value of the judgment flag of the configuration file 502a and sets "010" to the value of the unavailable factor of the configuration file 502a. When "001", "010", and "011" are set to the value of the unavailable factor in the judgment screen display process illustrated in FIG. 15, the judgment screen 610 illustrated in the lower part of FIG. 16, FIG. 17, and FIG. 28, respectively, is displayed on the display 403 of the viewing terminal 400.

The configuration file 502a may store other items so that the configuration file 502a can distinguish whether the unavailable factor of the surgical instrument 120 is a setting of unavailability by the operator or by the automatic detection process.

For example, in the configuration file 502a in FIG. 30, compared to the configuration file 502a in FIG. 29, a flag A and a flag B items are added in place of the unavailable factor. The flag A item stores either a value of "1" indicating that the unavailability is set by the operator or a value of "0" indicating that the unavailability is not set by the operator. The flag B item stores either a value of "1" indicating that the unavailability is set by the automatic detection process or a value of "0" indicating that the unavailability is not set by the automatic detection process. In this case, the judgment screen 610 can also be switched according to the reason for the unavailability.

<Effects of the method of managing a surgical instrument, an information processor, a management system, a surgical instrument, and a surgical robot according to Embodiment 4>

In addition to the effects of Embodiment 1 to 3 above, the following effect is achieved in Embodiment 4.

In updating the configuration file 502a illustrated in FIGS. 29 and 30, information about the surgical instrument 120 and the judgment result are associated so that whether the surgical instrument 120 is determined to be unavailable because the surgical instrument 120 is used up to the usage limit and whether a judgment result indicating unavailability is obtained for the surgical instrument 120 whose usage status is within the usage limit can be determined.

According to the configuration, a factor by which the surgical instrument 120 is determined to be unavailable can be distinguished. For example, whether the surgical instrument 120 is determined to be unavailable because the number of uses or period of use of the surgical instrument 120 has reached the maximum limit and whether the surgical instrument 120 is determined to be unavailable because a possible failure is detected before the number of uses or period of use of the surgical instrument 120 reaches the maximum limit can be distinguished.

In updating the configuration file 502a illustrated in FGIS. 29 and 30, information about the surgical instrument 120 is associated with the judgment result so as to be able to be determined whether the judgment result indicating that the instrument 120 is unavailable for use is obtained based on an instruction from the operator that the instrument 120 is unavailable for use or based on information about the operation status of the surgical instrument 120.

According to the configuration, the operator can distinguish which of the operator's instruction and operation status determined that the surgical instrument 120 is unavailable for use.

<Embodiment 5>

In Embodiment 5, a screen that extracts the surgical instrument 120 according to the condition and displays a list of the extracted surgical instrument 120 is displayed on the display 403 of the viewing terminal 400. In addition, in Embodiment 5, compared to Embodiments 1 to 4, other items are further stored in the configuration file 502a.

FIG. 31 is a flowchart illustrating a process related to a display of an extraction screen 620 by the controller 501 of the information processor 500 according to Embodiment 5.

In step S401, the controller 501 determines whether a display instruction of the extraction screen 620 of the surgical instrument 120 is accepted from the viewing terminal 400. When the display instruction of the extraction screen 620 is accepted, in step S402, the controller 501 transmits the information of the extraction screen 620 to the viewing terminal 400. As a result, the extraction screen 620 is displayed on the display 403 of the viewing terminal 400. The extraction screen 620 is described later with reference to FIG. 33.

In step S403, the controller 501 determines whether an unavailable button 626 is accepted to be operated on the extraction screen 620. When the unavailable button 626 is operated, in step S404, the controller 501 sets the value of the judgment flag in the configuration file 502a to 1 for all of the surgical instruments 120 displayed on the extraction screen 620. Then, in step S405, the controller 501 transmits the updated information to the arm apparatus 1 via the control apparatus 3. The updated information transmitted in step S405 is an instruction to update the value of the judgment flag in the memory information 124a to 1 and includes the surgical instrument ID. When the arm apparatus 1 receives the updated information, the arm apparatus 1 sets the value of the judgment flag in the memory information 124a to 1 for the surgical instrument 120 corresponding to the surgical instrument ID included in the updated information.

FIG. 32 is a diagram schematically illustrating a configuration of the configuration file 502a according to Embodiment 5.

The configuration file 502a in FIG. 32 has an additional manufacturer's item compared to the configuration file 502a in FIG. 11. In the manufacturer's item, a string that can identify the manufacturer of the surgical instrument 120 (e.g., manufacturer name or manufacturer ID) is stored.

FIG. 33 is a diagram schematically illustrating a configuration of the extraction screen 620 displayed on the display 403 of the viewing terminal 400.

The extraction screen 620 has pull-down menus 621 to 623, an extract button 624, an extraction result display area 625, and an unavailable button 626.

The operator of the viewing terminal 400 selects a manufacturer of the surgical instrument 120 to be extracted from the configuration file 502a, a type of the surgical instrument 120, and a range of the surgical instrument ID by operating the pull-down menus 621 to 623, respectively. When the extract button 624 is operated, the conditions selected in the pull-down menus 621 to 623 are transmitted to the information processor 500, and the contents of the configuration file 502a are extracted in the information processor 500 with the judgment flag value of 0 and the received conditions. The extraction result obtained by the information processor 500 is then transmitted to the viewing terminal 400 and displayed in the extraction result display area 625 of the extraction screen 620.

When the unavailable button 626 is operated, the value of the judgment flag in the configuration file 502a and the memory information 124a is set to 1 for all of the surgical instrument 120 displayed in the extraction result display area 625.

The configuration file 502a illustrated in FIG. 32 may be configured as illustrated in FIG. 34.

The configuration file 502a in FIG. 34 has an additional lot number item compared to the configuration file 502a in FIG. 32. In the lot number item, a string indicating the lot number of the surgical instrument 120 is stored.

In case that the configuration file 502a is configured as illustrated in FIG. 34, the extraction screen 620 illustrated in FIG. 33 is configured as illustrated in FIG. 35, for example.

Compared to the extraction screen 620 in FIG. 33, the extraction screen 620 in FIG. 35 has an additional pull-down menu 627 in place of the pull-down menu 623. The operator of the viewing terminal 400 selects a lot number of the surgical instrument 120 to be extracted from the configuration file 502a by operating the pull-down menu 627. Again, when the extract button 624 is operated, the contents of the configuration file 502a are extracted with the judgment flag value of 0 and the conditions selected in the pull-down menus 621, 622, and 627, and the extraction result is displayed in the extraction result display area 625. Also, when the unavailable button 626 is operated, all of the surgical instrument 120 displayed in the extraction result display area 625 are set to be unavailable for use.

<Effects of a method of managing a surgical instrument, an information processor, a management system, a surgical instrument, and a surgical robot according to Embodiment 5>

In addition to the effects of Embodiment 1 to 4 above, the following effects is achieved in Embodiment 5.

As illustrated in FIG. 31, the controller 501 accepts the display instruction of the extraction screen 620 regardless of the timing of the surgery. Therefore, the operator of the viewing terminal 400 can use the extraction screen 620 at any timing. In addition, by operating the unavailable button 626, one or more of the surgical instruments 120 displayed in the extraction result display area 625 are set to be unavailable for use at once. Therefore, the operator of the viewing terminal 400 can smoothly set the applicable surgical instrument 120 to be unavailable by the manufacturer, type, surgical instrument ID range, lot number, and other units.

<Embodiment 6>

In Embodiments 1 and 2, the operator sets the surgical instrument 120 to be unavailable via the judgment screen 610 displayed on the viewing terminal 400. In contrast, in Embodiment 6, the operator of the operation apparatus 2 sets the surgical instrument 120 to be unavailable via the judgment screen 610 displayed on the operation apparatus 2.

FIG. 36 is a flowchart illustrating a process by the controller 241 of the operation apparatus 2 according to Embodiment 6.

In case that the controller 141 of the arm apparatus 1 detects that the surgical instrument 120 is attached to the arm 110, the controller 141 sends a notification to the operation apparatus 2 indicating that the surgical instrument 120 is detected. The notification includes the surgical instrument ID of the detected surgical instrument 120.

When the controller 241 of the operation apparatus 2 receives a notification of detection of the surgical instrument 120 from the arm apparatus 1 in step S501, the controller 241 queries the information processor 500 based on the surgical instrument ID included in the notification in step S502 to obtain the value of the judgment flag corresponding to the surgical instrument ID.

In step S503, the controller 241 determines whether the value of the judgment flag obtained by the inquiry of step S502 is 0, that is, whether the surgical instrument 120 in question is continuously available for use in the future.

When the value of the judgment flag is 1, the controller 241 outputs an alert in step S504. Outputting an alert is, for example, displaying an alert screen on the viewer 211 (see FIG. 4) of the operation apparatus 2, outputting an alarm sound from a speaker installed in the operation apparatus 2, or turning on a lamp installed in the operation apparatus 2, etc. After outputting the alert, the process is returned to step S501 by inputting a cancellation of the alert or by an elapse of a predetermined time.

In case that the notification of the detection of the surgical instrument 120 is not received from the arm apparatus 1 in step S501, and in case that the value of the judgment flag is 0 in step S503, the process proceeds to step S505.

In step S505, the controller 241 determines whether a display instruction of the judgment screen is input via the operation panel 204 (see FIG. 4) by the operator of the operation apparatus 2. When the display instruction of the judgment screen is received, in step S506, the controller 241 displays the judgment screen 610 illustrated in the upper part of FIG. 16 on the viewer 211 in case that the value of the judgment flag is 0, and displays the judgment screen 610 illustrated in FIG. 26 on the viewer 211 in case that the value of the judgment flag is 1.

In case that the judgment screen 610 illustrated in the upper part of FIG. 16 is displayed, the operator of the operation apparatus 2 can operate the unavailable button 614 (see the upper part of FIG. 16) by operating the operation panel 204. In step S507, the controller 241 determines whether the unavailable button 614 is operated. In case that the unavailable button 614 is operated, in step S508, the controller 241 transmits an instruction including the surgical instrument ID of the target surgical instrument 120 to the information processor 500 to change the value of the judgment flag of the surgical instrument 120 to 1. When the controller 501 of the information processor 500 receives the instruction, the controller 501 changes the value of the judgment flag corresponding to the surgical instrument ID included in the instruction to 1 in the configuration file 502a.

Although the controller 241 performs the process of step S508 in case that the unavailable button 614 is operated in step S507, it is not limited to this, for example, in case that a predetermined operation is performed on the hand controller 220 or the foot unit 230, as the judgment result of step S507 is positive, the process of step S508 may be performed. Also, the unavailable button 614 may be displayed on the operating unit 102 of the arm apparatus 1. In this case, the controller 141 of the arm apparatus 1 may perform the above steps S505 to S508 and accept the operation of the unavailable button 614 via the operating terminal 102.

<Effects of a method of managing a surgical instrument, an information processor, a management system, a surgical instrument, and a surgical robot according to Embodiment 6>

In addition to the effects of the above embodiments, the following effect is achieved in Embodiment 6.

The physician, who is the operator of the operation apparatus 2, can display the judgment screen 610 illustrated in the upper part of FIG. 16 and FIG. 26 on the viewer 211 by operating the operation panel 204 during the execution of the surgery. Then, the operator, the physician, can set the surgical instrument 120 that the operator feels a possibility of malfunctioning as unavailable for use by operating the unavailable button 614 on the judgment screen 610. Therefore, the surgical instrument 120 that has the possibility of malfunctioning during the execution of a surgery to be quickly set to be unavailable for use.

<Embodiment 7>

In Embodiment 7, the available surgical instrument 120 is managed by an inventory DB of the information processor 500, and an inventory amount of the available surgical instrument 120 based on the inventory DB is displayed on the display 403 of the viewing terminal 400.

FIG. 37 is a diagram illustrating an overview of a connection of the surgical robot 4 and the information processor 500, etc., and information transmitted and received between each apparatus according to Embodiment 7. Compared to the configuration of Embodiment 1 illustrated in FIG. 9, a configuration of FIG. 37 has an inventory DB 502b stored in the memory 502 of the information processor 500.

FIG. 38 is a diagram schematically illustrating a configuration of the configuration file 502a stored in the memory 502 of the information processor 500 according to Embodiment 7. Compared to the configuration file 502a of Embodiment 1 illustrated in FIG. 11, the configuration file 502a of FIG. 38 includes items of a start date and time of use and comments. The display of comment items is described later with reference to FIGS. 44 and 45.

FIG. 39 is a diagram schematically illustrating a configuration of the inventory DB 502b. The inventory DB 502b includes items for the type of the surgical instrument 120, the total number of the surgical instrument 120 corresponding to the type, and the number of available surgical instrument 120 corresponding to the type.

FIG. 40 is a flowchart illustrating an updating process of the inventory DB 502b by the controller 501 of the information processor 500.

In step S601, the controller 501 determines whether there is a change in the configuration file 502a. In case that there is a change in the configuration file 502a, in step S602, the controller 501 counts the number of the surgical instrument 120 of each type for which the value of the judgment flag is 1 in the configuration file 502a. In step S603, the controller 501 updates the inventory DB 502b based on the count obtained in step S602. Specifically, the controller 501 subtracts the count number for each type obtained in step S603 from the total number of surgical instruments 120 for each type to obtain the number of available surgical instruments 120 for each type. The controller 501 then updates the inventory DB 502b based on the number of available surgical instruments 120 per type.

FIG. 41 is a flowchart illustrating a display process of an inventory management screen by the controller 501 of the information processor 500.

In step S611, the controller 501 determines whether a display instruction of the inventory management screen is accepted from the viewing terminal 400. Upon receiving the display instruction of the inventory management screen, the controller 501 reads information from the inventory DB 502b in step S612, and in step S613, based on the information read from the inventory DB 502b, transmits information on a list screen 630 of an inventory quantity of the surgical instrument 120 to the viewing terminal 400. The controller 401 of the viewing terminal 400 then displays the list screen 630 illustrated in the upper part of FIG. 42 on the display 403.

As illustrated in the upper part of FIG. 42, the list screen 630 of the inventory quantity displays a button 631 for each type of the surgical instrument 120. Above button 631, a name of the type of the surgical instrument 120 is displayed, and inside button 631, the number of the available surgical instrument 120 is displayed. When the button 631 is operated by the operator on the list screen 630, the controller 401 of the viewing terminal 400 transmits information indicating the type of the surgical instrument 120 to the information processor 500.

Returning to FIG. 41, in step S614, the controller 501 of the information processor 500 determines whether a specification of the type of the surgical instrument 120 is accepted from the viewing terminal 400. When the specification of the type of the surgical instrument 120 is accepted, the controller 501 reads information from the configuration file 502a based on the specified type in step S615, and transmits information on the list screen 640 of the surgical instrument 120 of the specified type to the viewing terminal 400 in step S616. Then, the controller 401 of the viewing terminal 400 displays the list screen 640 illustrated in the lower part of FIG. 42 on the display 403.

As illustrated in the lower part of FIG. 42, the list screen 640 has a list display area 641 that displays a list of the surgical instrument 120 of the target type. The list display area 641 includes an item indicating the usage status, an item for the surgical instrument ID, and an item for the number of times used. In the item indicating the usage status, an icon 641a indicating that the surgical instrument 120 is ready for use, an icon 641b indicating that unavailability is set by the operator or automatic detection process, and an icon 641c indicating that the maximum number of uses is reached are displayed. The list screen 640 illustrated in the lower part of FIG. 42 is an example of a screen that is displayed when the button 631 corresponding to forceps A is operated in the upper part of FIG. 42. When a line corresponding to the surgical instrument 120 is operated by the operator in the list screen 640, the controller 401 of the viewing terminal 400 transmits the information including the surgical instrument ID to the information processor 500.

Returning to FIG. 41, in step S617, the controller 501 of the information processor 500 determines whether a specification of the surgical instrument 120 is accepted from the viewing terminal 400. When the specification of the surgical instrument 120 is accepted, the controller 501 reads information from the configuration file 502a based on the specified surgical instrument 120 in step S618, and executes a status screen display process for the specified surgical instrument 120 in step S619.

FIG. 43 is a flowchart illustrating the details of a status screen display process in step S619 of FIG. 41.

In step S6191, the controller 501 transmits information of a status screen to the viewing terminal 400 based on the information read in step S618 of FIG. 41. As a result, a status screen 650 illustrated in FIGS. 44 and 45 is displayed on the display 403 of the viewing terminal 400.

As illustrated in FIGS. 44 and 45, the status screen 650 includes a surgical instrument information display area 651, a usage status display area 652, an elapsed time display area 653, and an OK button 654. The surgical instrument information display area 651 displays the type of the surgical instrument 120 and the surgical instrument ID. The usage status display area 652 displays the current number of times the surgical instrument 120 has been used, the maximum number of uses, and the date when the surgical instrument 120 is first used. Also, the usage status display area 652 has an unavailable button 652a, an in-use button 652b, and a comment area 652c. The operator of the viewing terminal 400 operates the input 404 (see FIG. 9) to enter comments in the comment area 652c.

The elapsed time display area 653 displays the wearing time and energization time of the surgical instrument 120. In this case, the information on the usage status transmitted from the arm apparatus 1 includes the wearing time and energization time. The controller 501 of the information processor 500 generates display contents of the elapsed time display area 653 based on the information.

The status screen 650 of the upper part in FIG. 44 is displayed in case that the surgical instrument 120 is available for use. In this case, the unavailable button 652a is operable, but the in-use button 652b is inoperable. The status screen 650 of the lower part in FIG. 44 is displayed in case that the surgical instrument 120 is set to be unavailable for use by the operator or by the automatic detection process. In this case, the in-use button 652b is operable, but the unavailable button 652a is inoperable. The status screen 650 illustrated in FIG. 45 is displayed in case that the surgical instrument 120 reaches the usage limit. In this case, the unavailable button 652a and the in-use button 652b are omitted.

Returning to FIG. 43, in step S6192, the controller 501 determines whether the unavailable button 652a is operated in the status screen 650 of the upper part in FIG. 44 or the in-use button 652b is operated in the status screen 650 of the lower part in FIG. 44.

In case that the unavailable button 652a is operated, the controller 501 updates the configuration file 502a so that the value of the judgment flag for the target surgical instrument 120 becomes 1 in step S6193, and updates the inventory DB 502b so that the number of availability for the type of the surgical instrument 120 is reduced by 1 in step S6194. On the other hand, in case that the in-use button 652b is operated, the controller 501 updates the configuration file 502a so that the value of the judgment flag of the target surgical instrument 120 becomes 0 in step S6193, and updates the inventory DB 502b so that the number of availability for the type of the surgical instrument 120 is increased by 1 in step S6194.

In step S6195, the controller 501 determines whether the OK button 654 is operated while a comment is entered in the comment area 652c on the status screen 650 of FIGS. 44 and 45. In case that the OK button 654 is operated while a comment is entered, the controller 501 stores the entered comment in the comment item of the target surgical instrument 120 in the configuration file 502a at step S6196. After the OK button 654 is operated, the status screen 650 may be erased or left displayed to accept further comment input.

<Effects of a method of managing a surgical instrument, an information processor, a management system, a surgical instrument, and a surgical robot according to Embodiment 7>

In addition to the effects of the above embodiments, the following effect is achieved in Embodiment 7.

The controller 501 of the information processor 500 accepts a comment from the operator regarding the unavailability of the surgical instrument 120 via the comment area 652c and stores the accepted comment in the configuration file 502a by associating the comment with the surgical instrument 120.

According to the process, a note can be left as to why the surgical instrument 120 is deemed unavailable by the operator, for example, "poor sharpness" as illustrated in the lower part of FIG. 44. Therefore, a comment can be shared among a plurality of the operators when there are a plurality of operators.

The list screens 630 and 640 illustrated in FIG. 42 display information about the inventory of the surgical instrument 120 (e.g., number of availability and total number of instruments) based on the judgment flag.

According to the process, information about the inventory of the surgical instrument 120 can be provided, reflecting the judgment result of usability. Therefore, accurate inventory information can be provided, such as the amount of the surgical instrument 120 that is actually available for use.

The information about inventory displayed on the list screens 630, 640, etc. includes information about the number of surgical instruments 120 that are available for use.

According to the configuration, the number of surgical instruments 120 that are actually available can be ascertained, making it easy to check whether there is a shortage of the surgical instrument 120.

<Variation of Embodiment 7>

The status screen 650 illustrated in FIGS. 44 and 45 may be configured as illustrated in FIG. 46.

The status screen 650 illustrated in FIG. 46 has two comment areas 652d in place of the comment area 652c, compared to FIGS. 44 and 45. The comment area 652d consists of a pull-down menu from which a plurality of comments can be selected.

The operator of the viewing terminal 400 can easily enter a comment by operating the comment area 652d.

The list screen 630 of the inventory quantity illustrated in the upper part of FIG. 42 may be configured as illustrated in FIG. 47.

The list screen 630 of the inventory quantity illustrated in FIG. 47 includes a switch 632 compared to FIG. 42. The switch 632 is a switch that allows a display state of the available surgical instrument 120 in the list screen 630 to be set to either on or off. When the switch 632 is set to ON, the number of available surgical instruments 120 of the target type is displayed in the button 631, as illustrated in the upper part of FIG. 47. On the other hand, when the switch 632 is set to OFF, the total number of surgical instruments 120 of the target type is displayed in the button 631, as illustrated in the lower part of FIG. 47.

The operator of the viewing terminal 400 can smoothly ascertain both the number of available surgical instruments and the total number of surgical instruments by operating the switch 632.

The inventory DB 502b illustrated in FIG. 39 may be configured as illustrated in FIG. 48.

Compared to FIG. 39, the inventory DB 502b illustrated in FIG. 48 includes items for the number of remaining counts of 1, the number of remaining counts of 2, the number of remaining counts of 3 or more, the number of counts that have reached the maximum number of uses, and the number of counts for which the unavailable button is operated. The number of remaining counts is the number of counts obtained by subtracting the current number of uses from the maximum number of uses. The number of counts for which the unavailable button is pressed is the number of surgical instruments 120 for which the value of the judgment flag is set to 1 due to the operation of the unavailable button 614 on the judgment screen 610 (see the upper parts of FIGS. 16 and 19), the unavailable button 626 on the extraction screen 620 (see FIGS. 33 and 35), or the unavailable button 652a on the status screen 650 (see FIGS. 44 and 46).

By configuring the inventory DB 502b as illustrated in FIG. 48, the list screen 660 of the inventory quantity illustrated in FIG. 49 can be smoothly displayed on the display 403 of the viewing terminal 400 based on the inventory DB 502b.

The list screen 660 of the inventory quantity in FIG. 49 includes a plurality of pie charts 661. One pie chart 661 is displayed for one type of the surgical instrument 120. In a peripheral area of the pie chart 661, an area 661 a is displayed corresponding to the number of surgical instruments 120 for which the unavailable button is operated, the number of surgical instruments 120 that reach the maximum number of uses, the number of surgical instruments 120 with remaining counts of 1, the number of surgical instruments 120 with remaining counts of 2, and the number of surgical instruments 120 with remaining counts of 3 or more, respectively. An area of each area 661a corresponds to the ratio of each number, and each area 661a is displayed in a different color or pattern. The center area of the pie chart 661 displays the total number of surgical instruments 120 of the type.

By referring to the list screen 660, the operator of the viewing terminal 400 can visually grasp the usage status of the surgical instrument 120. Also, the information about the inventory displayed on the list screen 660 includes information about the number or percentage of surgical instruments 120 that is determined to be unavailable for use. According to the configuration, how many of the surgical instruments 120 in the inventory are unavailable for use can be grasped.

The list screen 660 illustrated in FIG. 49 may be generated by extracting the contents of the configuration file 502a instead of being generated based on the inventory DB 502b in FIG. 48.

<Embodiment 8>

The viewing terminal 400 of Embodiments 1 to 7 is installed in a medical facility, but a viewing terminal of Embodiment 6 is installed not only in the medical facility but also in a support center of a company that manufactures or sells the surgical robot 4 and the surgical instrument 120.
FIG. 50 is a diagram illustrating an overview of a connection of each apparatus according to Embodiment 8.
A support center manages the surgical robot 4 installed in a plurality of medical facilities and the surgical instrument 120 used in a plurality of medical facilities. A viewing terminal 410 in the support center has the same configuration as the viewing terminal 400 in the medical facility and can perform the same processing as the viewing terminal 400. Therefore, the operator of the support center can display a screen based on the configuration file 502a and the inventory DB 502b on the display 403 (see FIG. 8) of the viewing terminal 410.
Except for the configuration and process related to the viewing terminal 410, the configuration and process of Embodiment 8 are the same as in the above Embodiments. Thus, the operator of the support center can, for example, set the surgical instrument 120 to be unavailable for use by operating the unavailable button on the judgment screen 610, the extraction screen 620, and the status screen 650 displayed on the viewing terminal 410.
Here, a medical facility may pay a company for the surgical instrument 120 under the assumption that the surgical instrument 120 can be used up to the usage limit. In this case, when the surgical instrument 120 is set to unavailable by the operator in the operating room or by the automatic detection process, the medical facility has consequently paid for the unused period beyond the period of actual use. For this reason, a company may compensate the medical facility, etc. for the purchase cost of the surgical instrument 120 that is set to be unavailable before the usage limit is reached. In this case, the operator of the support center operates the viewing terminal 410 in the support center to display a compensation screen 670 (described below) on the display 403 to confirm the surgical instrument 120 for which compensation is required.
FIG. 51 is a flowchart illustrating a display process of a compensation screen 670 by the controller 501 of the information processor 500.
In step S701, the controller 501 determines whether a display instruction of the compensation screen 670 is accepted from the viewing terminal 410. Upon receiving the display instruction of the compensation screen 670, in step S702, the controller 501 reads the configuration file 502a and extracts the surgical instrument 120 that is set to be unavailable for use, for example, the one whose usage status is within the usage limit and whose judgment flag value is 1. Then, in step S703, the controller 501 transmits information on the compensation screen 670 to the viewing terminal 410 based on the information on the extracted surgical instrument 120. As a result, the compensation screen 670 illustrated in FIG. 52 is displayed on the display 403 of the viewing terminal 410.
FIG. 52 is a diagram schematically illustrating a configuration of the compensation screen 670 displayed on the display 403 of the viewing terminal 410.
The compensation screen 670 includes pull-down menus 671 and 672, an extract button 673, and a result display area 674. In the initial state of the compensation screen 670, by the process of step S702 in FIG. 51, an item for displaying the surgical instrument 120 whose usage status is within the usage limit is selected in the pull-down menu 621, and an item for displaying the surgical instrument 120 whose judgment flag value is 1 is selected in the pull-down menu 672. As a result, information on the surgical instrument 120 whose usage status is within the usage limit and the value of the judgment flag is 1 is displayed in the result display area 674.
The pull-down menu 671 has an item for selecting the usage status, and the pull-down menu 672 has an item for setting the usability. The usability corresponds to the value of the judgment flag. When the operator of the support center selects items in the pull-down menus 671 and 672 and operates the extract button 673, information on the surgical instrument 120 corresponding to the selected items in the pull-down menus 671 and 672 is displayed in the result display area 674.

<Effects of a method of managing a surgical instrument, an information processor, a management system, a surgical instrument, and a surgical robot according to Embodiment 8>

In addition to the effects of the above embodiments, the following effect is achieved in Embodiment 8.
As illustrated in FIG. 52, the display 403 of the viewing terminal 410 displays information on the usage status of the surgical instrument 120 (e.g., the current number of uses) at the time when the usage status is determined to be unavailable for the surgical instrument 120 that is within the usage limit.
As mentioned above, when the value of the judgment flag of the surgical instrument 120 is set to 1, the surgical instrument 120 becomes unavailable for surgery. Therefore, the information on the usage status of the surgical instrument 120 that is determined to be unavailable is the information on the usage status at the time when the surgical instrument is determined to be unavailable. According to the above process, the operator of a manufacturer's support center can refer to the display 403 of the viewing terminal 410 to determine the extent to which the surgical instrument 120 within the usage limit has been used up to the point in time when the surgical instrument 120 is determined to be unavailable. Thus, for example, in case that a medical facility pays a manufacturer or distributor for the surgical instrument 120 on the assumption that the surgical instrument 120 can be used up to the usage limit, the company can process refunds, etc., considering the unused portion.

<Variation 1 of Embodiment 8>

In Embodiment 8, the configuration file 502a stored in the memory 502 may include an item indicating whether compensation is performed for the surgical instrument 120.

The configuration file 502a illustrated in FIG. 53 includes items for a customer ID and a compensated flag compared to FIG. 32. The customer ID is an ID that can identify the medical facility or other entity that purchases the surgical instrument 120. A value of the compensated flag is set to 0 when no compensation is made for the surgical instrument 120, and is set to 1 when compensation is made for the surgical instrument 120. In this case, the process in FIG. 51 displays the compensation screen 670 illustrated in FIG. 54 on the display 403 of the viewing terminal 410.

Compared to FIG. 52, the compensation screen 670 illustrated in FIG. 54 includes pull-down menus 675 and 676 and an add button 677. The pull-down menu 675 is set up for selecting a customer ID, and the pull-down menu 676 is set up for selecting a compensated flag. When the operator of the support center selects items in the pull-down menus 671, 672, 675, and 676 and operates the extract button 673, information on the surgical instrument 120 corresponding to the selected items in the pull-down menus 671, 672, 675, and 676 is displayed in the result display area 674. The operator can further add a pull-down menu for extracting the surgical instrument 120 to the compensation screen 670 by operating the add button 677.

When the operator of the support center operates a line corresponding to one surgical instrument 120 in the result display area 674, the controller 501 of the information processor 500 reads the information on the operated surgical instrument 120 from the configuration file 502a and transmits the information to the viewing terminal 410. As a result, a compensation screen 680 illustrated in FIG. 55 is displayed on the display 403 of the viewing terminal 410.

The compensation screen 680 illustrated in FIG. 55, same as the judgment screen 610 above, includes a surgical instrument information display area 681, a usage status display area 682, and a usability display area 683. In addition, the compensation screen 680 has a compensated button 684 that sets the surgical instrument 120 as compensated. The operator operates the compensated button 684 when compensation is performed for the target surgical instrument 120. As a result, the controller 501 of the information processor 500 sets a value of a compensated flag in the configuration file 502a to 1 for the surgical instrument 120 for which the compensated button 684 is operated.

<Variation 2 of Embodiment 8>

In a variation 1 of Embodiment 8, furthermore, the compensation may be calculated automatically.

FIG. 56 is a flowchart illustrating a determination process of compensation details by the controller 501 of the information processor 500.

In step S711, the controller 501 waits for a process until the timing for compensation calculation arrives. The timing for compensation calculation is, for example, the beginning of the month, the end of the month, the beginning of the week, or the weekend, etc. When the timing for compensation calculation arrives, in step S712, the controller 501 extracts, for each customer ID, the surgical instrument 120 whose usage status is within the usage limit during the period from the timing of the previous compensation calculation to the timing of the current compensation calculation and which is set to be unavailable for use. In step S713, the controller 501 determines compensation details based on the following compensation calculation rule, for example.

In step S713, the controller 501 calculates the basic amount of compensation for one extracted surgical instrument 120 using the following formula.

$$\text{Basic amount of compensation} = \text{basic price of the surgical instrument 120} \times (1 - \text{current usage} / \text{maximum usage})$$

The controller 501 calculates the basic amount of compensation based on the above formula for all surgical instruments 120 extracted corresponding to one customer ID, and calculates the amount of compensation for one customer ID by summing the calculated basic amount of compensation. The controller 501 then obtains the amount of compensation corresponding to each extracted customer ID.

In step S714, the controller 501 transmits the information on a compensation details screen 690 that displays the compensation amount corresponding to each customer ID obtained in step S713 to the viewing terminal 410 of the support center. As a result, the compensation details screen 690 illustrated in FIG. 57 is displayed on the display 403 of the viewing terminal 410.

FIG. 57 is a diagram schematically illustrating a configuration of the compensation details screen 690 displayed on the display 403 of the viewing terminal 410. The compensation details screen 690 has a period display area 691 that indicates the period of the compensation details and a compensation amount display area 692 that indicates the compensation amount obtained for each customer ID.

According to the variation, the controller 501 of the information processor 500 extracts the surgical instrument 120 whose usage status is within the usage limit and which is determined to be unavailable for use (step S712 in FIG. 56), and transmits information on the compensation of the surgical instrument 120 (information corresponding to a display content of the compensation details screen 690 in FIG. 57) to the viewing terminal 410 (step S714 in FIG. 56).

According to the process, the surgical instrument 120 that may be eligible for compensation that is determined to be unavailable before the usage limit is reached is easily identified, and information regarding the compensation is provided. Therefore, the information on compensation can be used for a refund process to a medical facility.

As illustrated in FIG. 58, the controller 501 may set the surgical instrument 120 to be compensated as well as determine the compensation details of the surgical instrument 120.

Compared to the process in FIG. 56, the process in FIG. 58 has an additional step S721 between steps S713 and S714. In step S721, the controller 501 sets the value of the compensated flag of the surgical instrument 120 for which compensation is determined to 1. When the surgical instrument 120 is automatically changed to compensated in this way, the operator of the support center no longer needs to individually set the surgical instrument 120 to compensated by operating the compensated button 684 on the compensation screen 680 illustrated in FIG. 55.

In addition, Embodiment 8 and variations 1 and 2 of Embodiment 8 may be modified as follows.

The compensation details for each surgical instrument 120 determined in the processes of FIGS. 56 and 58 may be stored in the configuration file 502a above.

The controller 501 of the information processor 500 may set a billing amount for a period of time and for each customer based on the amount of compensation for each customer calculated as described above. In this case, the controller 501 conducts a process that reduces the compensation amount calculated for the period from the billing amount for the next period. Also, in case that the billing amount for the next period is less than the compensation amount calculated for the period, the controller 501 further reduces the differential amount from the billing amount for the next period.

<Embodiment 9>

In Embodiment 9, an operator logs in to an operator terminal such as the viewing terminal 400, the operation apparatus 2, and the viewing terminal 410 using a user ID assigned to the operator.

FIG. 59 is a diagram schematically illustrating a configuration of user information.

The user information includes a user ID and a customer ID associated with the user ID. The user ID is an ID that is assigned to each operator and can identify each operator individually. The customer ID is an ID that can identify a medical facility, etc. individually and is associated with one or more user IDs. In an example illustrated in FIG. 59, the operators whose user IDs are "1111" and "1112" are operators belonging to the same medical facility. In addition, the operator whose user ID is "admin1" is an operator belonging to a service center. Therefore, the corresponding customer ID is "service 1".

FIG. 60 is a flowchart illustrating a login process by the controller 501 of the information processor 500. An example of how the status screen 650 illustrated in FIG. 44 is displayed at a request of the operator is described as follows.

In step S801, the controller 501 determines whether a login instruction is received from an operator terminal such as the viewing terminal 400, the operation apparatus 2 and the viewing terminal 410. The login instruction includes a user ID. After receiving the login instruction, in step S802, the controller 501 determines whether a display instruction of the status screen 650 is received from the operator terminal.

Upon receiving the display instruction, in step S803, the controller 501 obtains a customer ID from the user information illustrated in FIG. 59 based on the accepted user ID, and extracts all surgical instruments 120 corresponding to the obtained customer ID from the configuration file 502a. For example, in case that the user ID is "1111", the surgical instrument 120 whose customer ID is "C001" is extracted, and in case that the user ID is "admin1", the surgical instruments 120 corresponding to all customer IDs are extracted. In step S804, the controller 501 transmits the information on the status screen 650 based on the surgical instrument 120 extracted in step S803 to the operator terminal.

FIG. 61 is a diagram schematically illustrating a configuration of the status screen 650 displayed on a display of an operator terminal.

The status screen 650 on the upper part of FIG. 61 is the status screen 650 in case that the user ID is "admin1" and the surgical instrument ID is "SI051".

Here, only the operator logged in with the user IDs "1111" and "1112" corresponding to the customer ID "C001" can view the information of the surgical instrument 120 whose surgical instrument ID is "SI051". However, since the user ID in this case is "admin1", the operator logged in with the user ID "admin1" can also view the information of the surgical instrument 120 whose surgical instrument ID is "SI051", as illustrated in the status screen 650 of the upper part in FIG. 61.

When the operator with the user ID "admin1", i.e., the operator of the support center, enters a comment in the comment area 652c and operates the OK button 654, the comment is stored in the configuration file 502a. The comment stored at this time cannot be viewed by an operator with other user ID than "admin1" because the comment is stored in association with the user ID.

The status screen 650 in the lower part of FIG. 61 is the status screen 650 when the user ID is "1111" and the surgical instrument ID is "SI005". In this case, the same surgical instrument 120 is displayed as in the upper part of FIG. 61, but the comment "possible failure based on specifications" entered by the operator of the support center is not displayed, and only the comment "poor sharpness" entered by the operator of the apparatus is displayed.

<Effects of a method of managing a surgical instrument, an information processor, a management system, a surgical instrument, and a surgical robot according to Embodiment 9>

In addition to the effects of the above embodiments, the following effect is achieved in Embodiment 9.

In case that an operator of a medical facility logs into an operator terminal, the information displayed on the operator terminal is only the information of the surgical instrument 120 corresponding to the medical facility to which the operator belongs. Therefore, the information on the surgical instrument 120 used at one medical facility is prevented from being viewed at other medical facilities. Also, in case that an operator belongs to a support center, the information on all surgical instruments 120 is displayed on the operator terminal. Therefore, the operator at the support center can manage the surgical instrument 120 used at all medical facilities.

The contents of comments entered by an operator at a medical facility can only be viewed by an operator at the medical facility to which the operator belongs. Therefore, a comment entered at one medical facility is prevented from being viewed at other medical facilities. Similarly, the contents of comments entered by the operator of a support center can be viewed only by an operator of the support center. Therefore, comments are shared among support center operators, thus enabling information to be accumulated and analyzed within the support center and improving service quality.

<Embodiment 10>

FIG. 62 is a diagram schematically illustrating an arrangement of an apparatus in an operating room of a medical facility according to Embodiment 10.

Compared to the configuration of Embodiment 1 illustrated in FIG. 9, a configuration of each apparatus in Embodiment 10 omits the viewing terminal 400, and the information processor 500 is located in an operating room. The information processor 500 is connected to the control apparatus 3 in the operating room for communication. Also, compared to the configuration of FIG. 9, the information processor 500 is provided with a display 504 and an input 505. The display 504 and the input 505 have similar configurations to those of the display 403 and the input 404 of the viewing terminal 400 of Embodiment 1.

In Embodiment 10, an operator of the information processor 500 operates the input 505 of the information processor 500 to input an instruction to the information processor 500. The controller 501 of the information processor 500 displays a screen on the display 504 in response to the entered instruction. Therefore, in the information processor 500 of Embodiment 10, the screen displayed on the viewing terminal 400 in the above embodiment is displayed in the same way, and the operator of the information processor 500 can view the same information as the operator of the viewing terminal 400 in the above embodiment.

<Embodiment 11>

In the above embodiment, as illustrated in FIGS. 12 and 21, in case that the target surgical instrument 120 is already removed from the arm apparatus 1 at the time step S111 is executed, the memory information 124a of the surgical instrument 120 is not updated in step S111. In this case, when the surgical instrument 120 is attached to the arm apparatus 1 in the next surgery, the surgical instrument 120 is updated to the latest judgment flag value in step S103.

In contrast, in Embodiment 11, by using a cradle 6 illustrated below, the surgical instrument 120 can always be in communication with the information processor 500. Therefore, the judgment flag in the memory information 124a of the surgical instrument 120 can be quickly updated.

FIG. 63 is a diagram schematically illustrating a configuration and a connection of the cradle 6.

The cradle 6 has a controller 61, a communication unit 62, and a plurality of terminals 63. The controller 61 is configured with, for example, a CPU and an FPGA. The communication unit 62 is selectively connected to the plurality of terminals 63 according to an instruction of the controller 61. Also, the communication unit 62 is communicatively connected to the information processor 500 located in the operating room. The surgical instrument 120 includes a terminal 126 and a memory 124, as illustrated in FIG. 20. The surgical instrument 120 is installed with respect to the cradle 6 so that the terminal 63 of the cradle 6 and the terminal 126 of the surgical instrument 120 are electrically connected.

An operator in the operating room places the surgical instrument 120 removed from the arm apparatus 1 on the cradle 6 as illustrated in FIG. 63. Therefore, the surgical instrument 120 is in a state where the surgical instrument 120 is communicatively connected to the information processor 500.

According to Embodiment 11, even when the surgical instrument 120 is removed from the arm 110, the surgical instrument 120 is always communicatively connected to the information processor 500 by being installed in the cradle 6. Therefore, the memory information 124a can be quickly updated in response to an update instruction from the information processor 500 in step S111 of FIGS. 12 and 21.

The cradle 6 is not limited to being connected to the information processor 500, but may also be connected to the arm apparatus 1, the operation apparatus 2, the control apparatus 3 or the viewing terminal 400. In this case, the apparatus to which the cradle 6 is connected updates the memory information 124a based on the update instruction from the information processor 500. Also, the cradle 6 may be connected to the information processor 500 that is installed in a room other than an operating room in a hospital or in a facility outside the hospital, such as a support center or data center, via a LAN, the Internet, etc.

The embodiment of the invention can be modified in various ways as appropriate within the scope of the technical ideas indicated in the claims.

For example, the surgical robot 4 does not need to consist of the arm apparatus 1, the operation apparatus 2, and the control apparatus 3; and for example, the control apparatus 3 may be omitted and each process performed by the control apparatus 3 may be performed by the controller 141 of the arm apparatus 1 or the controller 241 of the operation apparatus 2; and the surgical robot 4 may be configured so that the controller 141 of the arm apparatus 1 and the controller 241 of the operation apparatus 2 share the responsibility for executing each process performed by the control apparatus 3.

As a supplementary note, a method of surgical instrument management, an information processor, a management system, a surgical instrument, and a surgical robot according to one or more embodiments are summarized.

A method of managing a surgical instrument that is removably attached to a surgical robot and reusable up to a usage limit comprising:

obtaining information on a usage status of the surgical instrument used in a surgery by the surgical robot;
obtaining a judgment result indicating that the surgical instrument whose usage status is within the usage limit is unavailable for use; and
associating information on the surgical instrument with the judgment result.

In the method of managing the surgical instrument, the judgment result is a judgment result based on a possibility of failure of the surgical instrument.

In the method of managing the surgical instrument, the method further comprises:
determining that the surgical instrument is unavailable for use in case that the surgical instrument has been used up to the usage limit.

In the method of managing the surgical instrument, the method further comprises:
outputting an alert in case that the surgical instrument that had been determined to be unavailable for use has been attached to the surgical robot.

In the method of managing the surgical instrument, the method further comprises:
prohibiting an operation of the surgical robot until the surgical instrument is replaced with an available surgical instrument in case that the surgical instrument that had been determined to be unavailable for use has been attached to the surgical robot.

In the method of managing the surgical instrument, the associating the information on the surgical instrument with the judgment result comprises associating the information related to the surgical instrument and the judgment result such that whether (a) the surgical instrument has been determined to be unavailable for use because the surgical instrument had been used up to the usage limit or (b) the judgment result indicating that the surgical instrument whose usage status is within the usage limit is unavailable for use has been obtained is distinguishable.

In the method of managing the surgical instrument, obtaining the judgment result comprises obtaining the judgment result based on at least one of multiple types of conditions.

In the method of managing the surgical instrument, information on the usage status comprises information on a number of times the surgical instrument has been used.

In the method of managing the surgical instrument, information on the number of times the surgical instrument has been used comprises information on a number of surgeries in which the surgical instrument has been used.

In the method of managing the surgical instrument, the surgical instrument whose usage status is within the usage limit is a surgical instrument that has not reached a maximum number of uses, a surgical instrument that has not reached a maximum number of times the surgical instrument has been used in surgery, or a surgical instrument whose period of use has not reached a maximum use period.

In the method of managing the surgical instrument, obtaining the judgment result comprises obtaining the judgment result in case that an instruction to disable a use of the surgical instrument has been received.

In the method of managing the surgical instrument, on a display screen displaying the information on the usage status, the instruction to disable the use of the surgical instrument is accepted.

In the method of managing the surgical instrument, the method further comprises:

receiving a comment regarding unavailability for use of the surgical instrument; and
storing the comment in association with the information on the surgical instrument.

In the method of managing the surgical instrument, the method further comprises providing information on the usage status at a time the judgment result is obtained.

In the method of managing the surgical instrument, obtaining the judgment result comprises obtaining the judgment result based on information regarding an operation status of the surgical instrument.

In the method of managing the surgical instrument, the method further comprises:
providing information indicating that the surgical instrument is unavailable for use.

In the method of managing the surgical instrument,
obtaining the judgment result comprises:

obtaining the judgment result in case that an instruction to disable the use of the surgical instrument has been received; and
obtaining the judgment result based on information on an operation status of the surgical instrument, and
associating the information on the surgical instrument and the judgment result comprising associating the information on the surgical instrument and the judgement result such that whether (a) the judgment result has been obtained by receiving an instruction to disable the use of the surgical instrument or (b) the judgment result has been obtained based on information on the operation status of the surgical instrument is distinguishable.

In the method of managing the surgical instrument, the method further comprises:
providing information on an inventory of the surgical instrument, based on the judgment result.

In the method of managing the surgical instrument, the information on the inventory includes information on a number of surgical instruments that are ready for use.

In the method of managing the surgical instrument, the information on the inventory includes information on a number or percentage of surgical instruments that have been determined to be unavailable for use.

In the method of managing the surgical instrument, the method further comprises:

extracting a surgical instrument for which the judgment result has been obtained;
and providing information on compensation of the surgical instrument.

An information processor that manages a surgical instrument which is removably attached to a surgical robot and is reusable up to a usage limit comprising:

a communication unit for communicating with the surgical robot; and
a controller that is connected to the communication unit, wherein

the controller obtains information on a usage status of the surgical instrument used in a surgery by the surgical robot,
the controller obtains a judgment result indicating that the surgical instrument whose usage status is within the usage limit is unavailable for use, and
the controller associates the information on the surgical instrument with the judgment result.

In the information processor, the information processor is installed in a second facility different from a first facility where the surgical robot is installed, and

the communication unit communicates with the surgical robot installed at the first facility.

A management system that manages a surgical instrument that is removably attached to a surgical robot and is reusable up to a usage limit comprising:

an information processor; and
a viewing terminal that is communicably connected to the information processor, wherein
the information processor comprises:

a communication unit for communicating with the surgical robot, and
a controller that is connected to the communication unit, wherein

the controller obtains information on a usage status of the surgical instrument used in a surgery by the surgical robot,
the controller obtains a judgment result indicating that the surgical instrument whose usage status is within the usage limit is unavailable for use, and
the controller provides information based on the judgment result to the viewing terminal.

A surgical instrument that is removably attached to a surgical robot and is reusable up to a usage limit comprising: a memory for storing information on the surgical instrument, wherein the memory comprises a memory area for storing information on a usage status of the surgical instrument used in a surgery by the surgical robot and a judgment result indicating the surgical instrument whose usage status is within the usage limit has been determined to be unavailable for use, in association with the information on the surgical instrument.

A surgical robot that performs a surgical operation with a surgical instrument that is reusable up to a usage limit comprising:

an arm to which the surgical instrument is removable attached; and
a controller for controlling an operation of the arm, wherein
the controller obtains a judgment result indicating the surgical instrument which has been attached to the arm and is within the usage limit is unavailable for use, and
the controller outputs an alert indicating that the judgment result has been obtained.

## Claims

**1.** A method of managing a surgical instrument that is removably attached to a surgical robot and reusable up to a usage limit comprising:

obtaining information on a usage status of the surgical instrument used in a surgery by the surgical robot;
obtaining a judgment result indicating that the surgical instrument whose usage status is within the usage limit is unavailable for use; and
associating information on the surgical instrument with the judgment result.

**2.** The method of managing the surgical instrument according to claim 1, wherein
the judgment result is a judgment result based on a possibility of failure of the surgical instrument.

**3.** The method of managing the surgical instrument according to claim 1, further comprising:
determining that the surgical instrument is unavailable for use in case that the surgical instrument has been used up to the usage limit.

**4.** The method of managing the surgical instrument according to claim 1, further comprising:
outputting an alert in case that the surgical instrument that had been determined to be unavailable for use has been attached to the surgical robot.

**5.** The method of managing the surgical instrument according to claim 1, further comprising:
prohibiting an operation of the surgical robot until the surgical instrument is replaced with an available surgical instrument in case that the surgical instrument that had been determined to be unavailable for use has been attached to the surgical robot.

6. The method of managing the surgical instrument according to claim 3, wherein
the associating the information on the surgical instrument with the judgment result comprises associating the information related to the surgical instrument and the judgment result such that whether (a) the surgical instrument has been determined to be unavailable for use because the surgical instrument had been used up to the usage limit or (b) the judgment result indicating that the surgical instrument whose usage status is within the usage limit is unavailable for use has been obtained is distinguishable.

7. The method of managing the surgical instrument according to claim 1, wherein
the obtaining the judgment result comprises obtaining the judgment result based on at least one of multiple types of conditions.

8. The method of managing the surgical instrument according to claim 1, wherein
the information on the usage status comprises information on a number of times the surgical instrument has been used.

9. The method of managing the surgical instrument according to claim 8, wherein
the information on the number of times the surgical instrument has been used comprises information on a number of surgeries in which the surgical instrument has been used.

10. The method of managing the surgical instrument according to claim 1, wherein
the surgical instrument whose usage status is within the usage limit is a surgical instrument that has not reached a maximum number of uses, a surgical instrument that has not reached a maximum number of times the surgical instrument has been used in surgery, or a surgical instrument whose period of use has not reached a maximum use period.

11. The method of managing the surgical instrument according to claim 1, wherein
the obtaining the judgment result comprises obtaining the judgment result in case that an instruction to disable a use of the surgical instrument has been received.

12. The method of managing the surgical instrument according to claim 11, wherein
on a display screen displaying the information on the usage status, the instruction to disable the use of the surgical instrument is accepted.

13. The method of managing the surgical instrument according to claim 11, further comprising:

receiving a comment regarding unavailability for use of the surgical instrument; and
storing the comment in association with the information on the surgical instrument.

14. The method of managing the surgical instrument according to claim 1, further comprising:
providing information on the usage status at a time the judgment result is obtained.

15. The method of managing the surgical instrument according to claim 1, wherein
the obtaining the judgment result comprises obtaining the judgment result based on information regarding an operation status of the surgical instrument.

16. The method of managing the surgical instrument according to claim 1, further comprising:
providing information indicating that the surgical instrument is unavailable for use.

17. The method of managing the surgical instrument according to claim 1wherein the obtaining the judgment result comprises:

obtaining the judgment result in case that an instruction to disable the use of the surgical instrument has been received; and
obtaining the judgment result based on information on an operation status of the surgical instrument, and

the associating the information on the surgical instrument and the judgment result comprises associating the information on the surgical instrument and the judgement result such that whether (a) the judgment result has been obtained by receiving an instruction to disable the use of the surgical instrument or (b) the judgment result has been obtained based on information on the operation status of the surgical instrument is distinguishable.

**18.** The method of managing the surgical instrument according to claim 1, further comprising:
providing information on an inventory of the surgical instrument, based on the judgment result.

**19.** The method of managing the surgical instrument according to claim 18, wherein
the information on the inventory includes information on a number of surgical instruments that are ready for use.

**20.** The method of managing the surgical instrument according to claim 18, wherein
the information on the inventory includes information on a number or percentage of surgical instruments that have been determined to be unavailable for use.

**21.** The method of managing the surgical instrument according to claim 1, further comprising:

extracting a surgical instrument for which the judgment result has been obtained;
and providing information on compensation of the surgical instrument.

**22.** An information processor that manages a surgical instrument which is removably attached to a surgical robot and is reusable up to a usage limit comprising:

a communication unit for communicating with the surgical robot; and
a controller that is connected to the communication unit, wherein
the controller

obtains information on a usage status of the surgical instrument used in a surgery by the surgical robot,
obtains a judgment result indicating that the surgical instrument whose usage status is within the usage limit is unavailable for use, and
associates information on the surgical instrument with the judgment result.

**23.** The information processor according to claim 22, wherein

the information processor is installed in a second facility different from a first facility where the surgical robot is installed, and
the communication unit communicates with the surgical robot installed at the first facility.

**24.** A management system that manages a surgical instrument that is removably attached to a surgical robot and is reusable up to a usage limit comprising:

an information processor; and
a viewing terminal that is communicably connected to the information processor, wherein
the information processor comprises:

a communication unit for communicating with the surgical robot, and
a controller that is connected to the communication unit, wherein
the controller

obtains information on a usage status of the surgical instrument used in a surgery by the surgical robot,
obtains a judgment result indicating that the surgical instrument whose usage status is within the usage limit is unavailable for use, and
provides information based on the judgment result to the viewing terminal.

**25.** A surgical instrument that is removably attached to a surgical robot and is reusable up to a usage limit comprising:
a memory for storing information on the surgical instrument, wherein
the memory comprises a memory area for storing information on a usage status of the surgical instrument used in a surgery by the surgical robot and a judgment result indicating the surgical instrument whose usage status is within the usage limit has been determined to be unavailable for use, in association with the information on the surgical instrument.

**26.** A surgical robot that performs a surgical operation with a surgical instrument that is reusable up to a usage limit comprising:

an arm to which the surgical instrument is removably attached; and
a controller for controlling an operation of the arm, wherein
the controller

obtains a judgment result indicating the surgical instrument which has been attached to the arm and is within the usage limit is unavailable for use, and
outputs an alert indicating that the judgment result has been obtained.

FIG.1

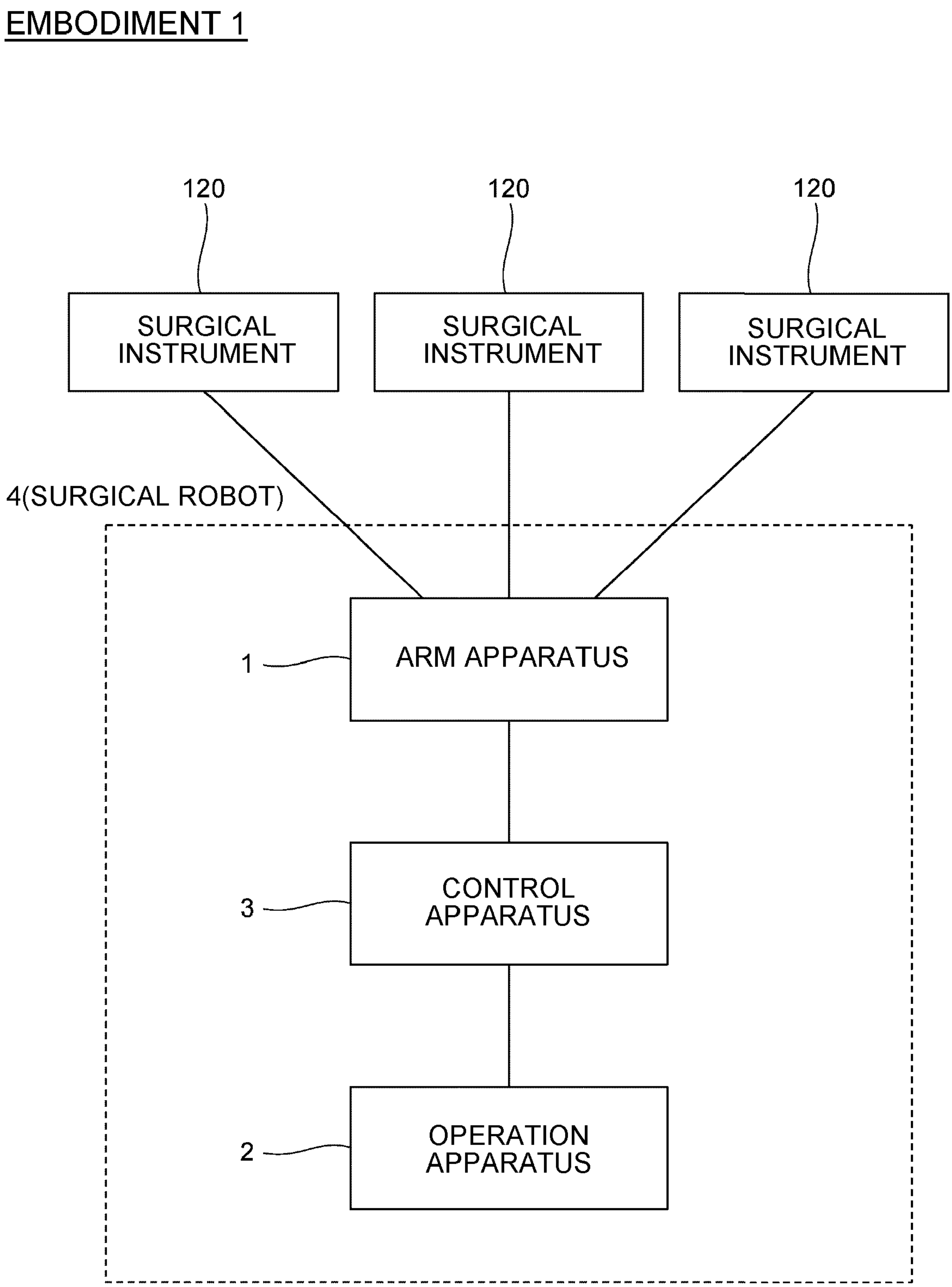
EMBODIMENT 1
120
120
120
SURGICAL INSTRUMENT
SURGICAL INSTRUMENT
SURGICAL INSTRUMENT
4(SURGICAL ROBOT)
1
ARM APPARATUS
3
CONTROL APPARATUS
2
OPERATION APPARATUS

FIG.2

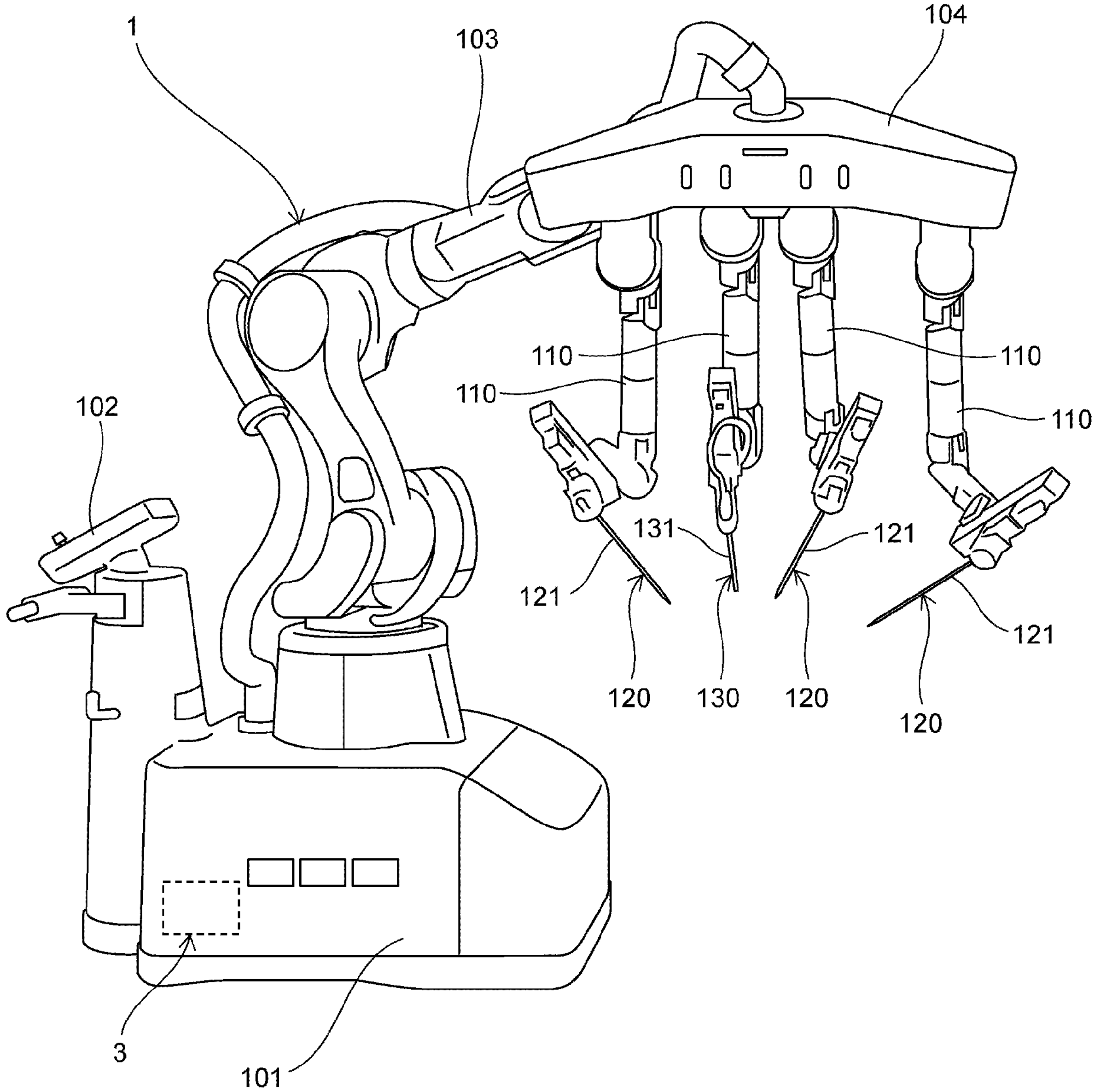
1
103
104
102
110
110
110
110
131
121
121
121
121
120
130
120
120
3
101

FIG.3

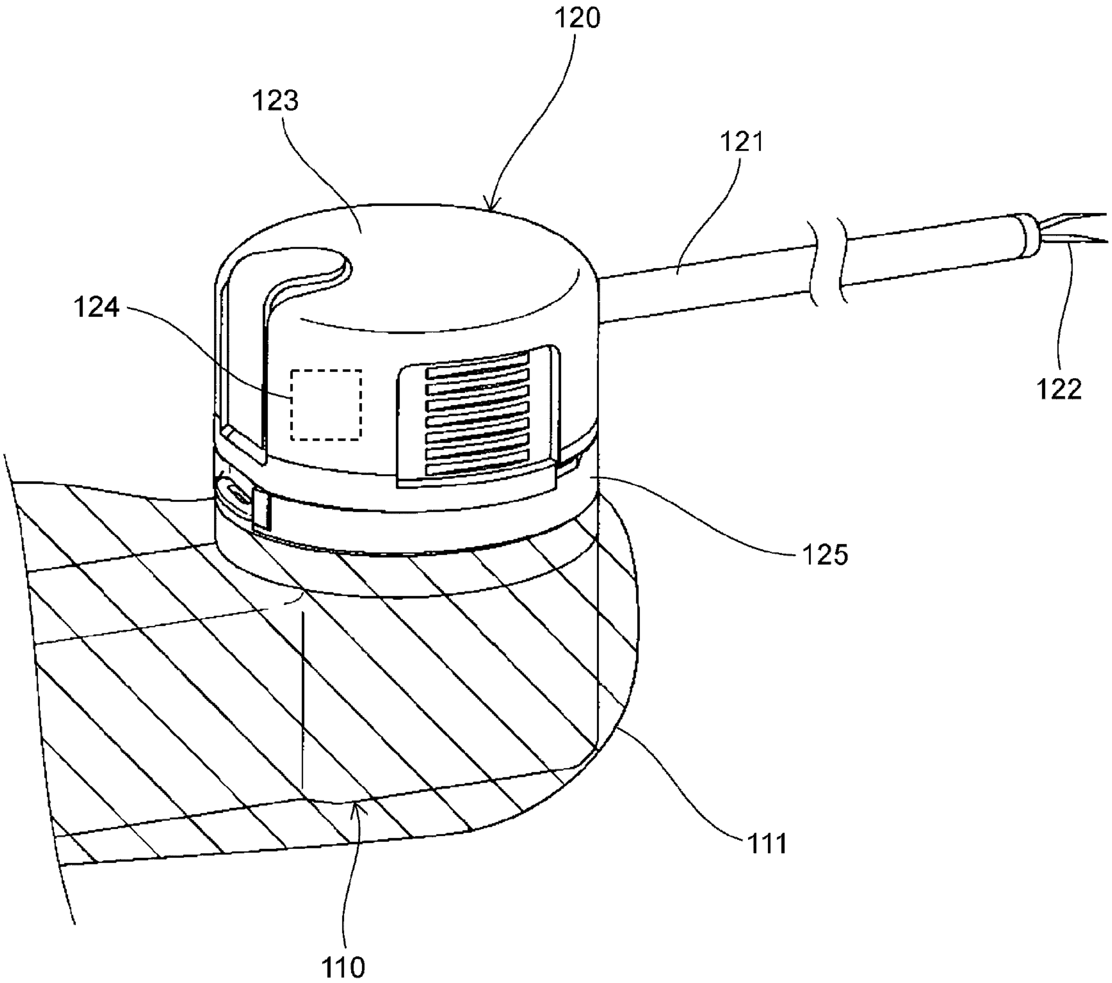

FIG.4

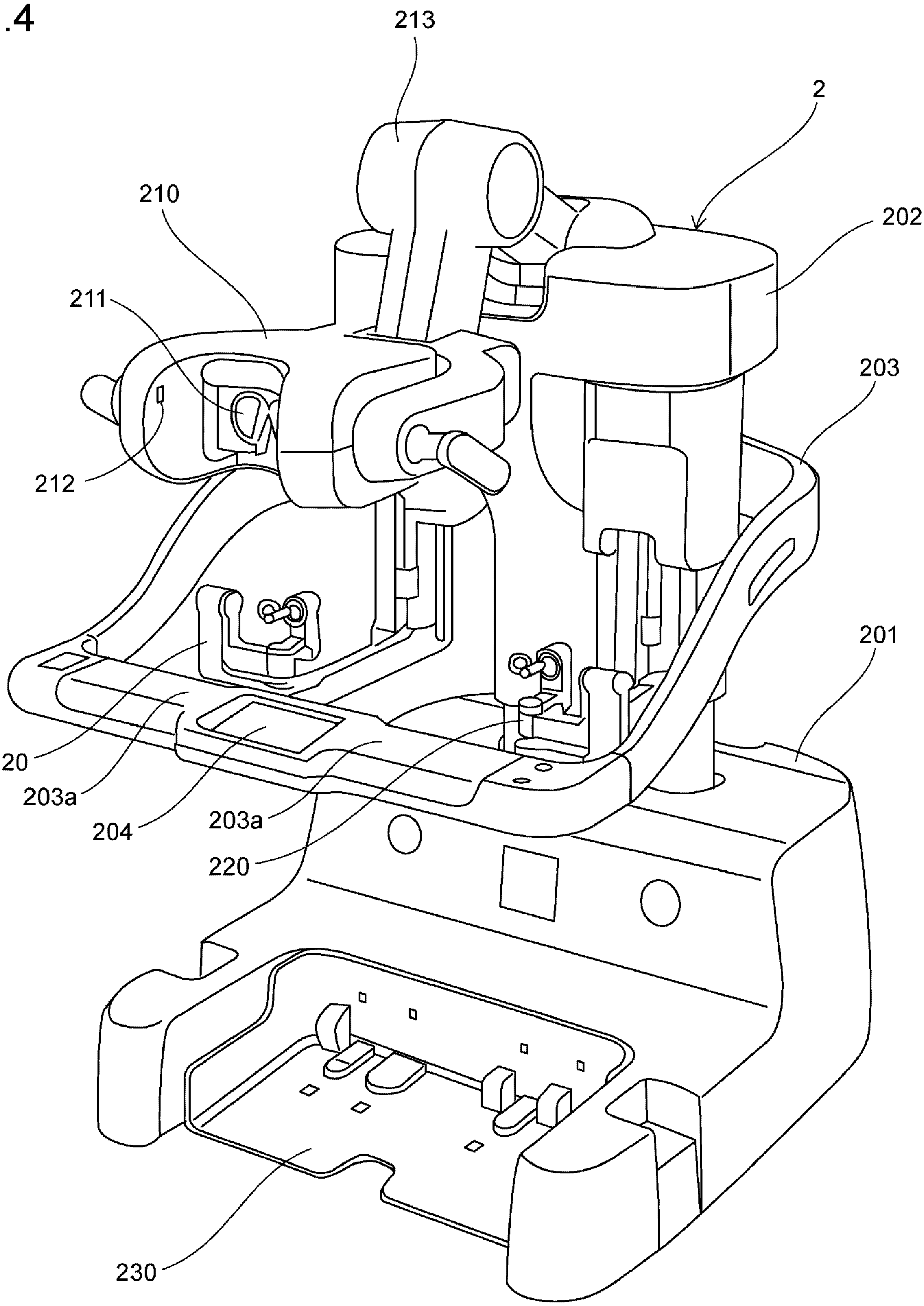

FIG.5

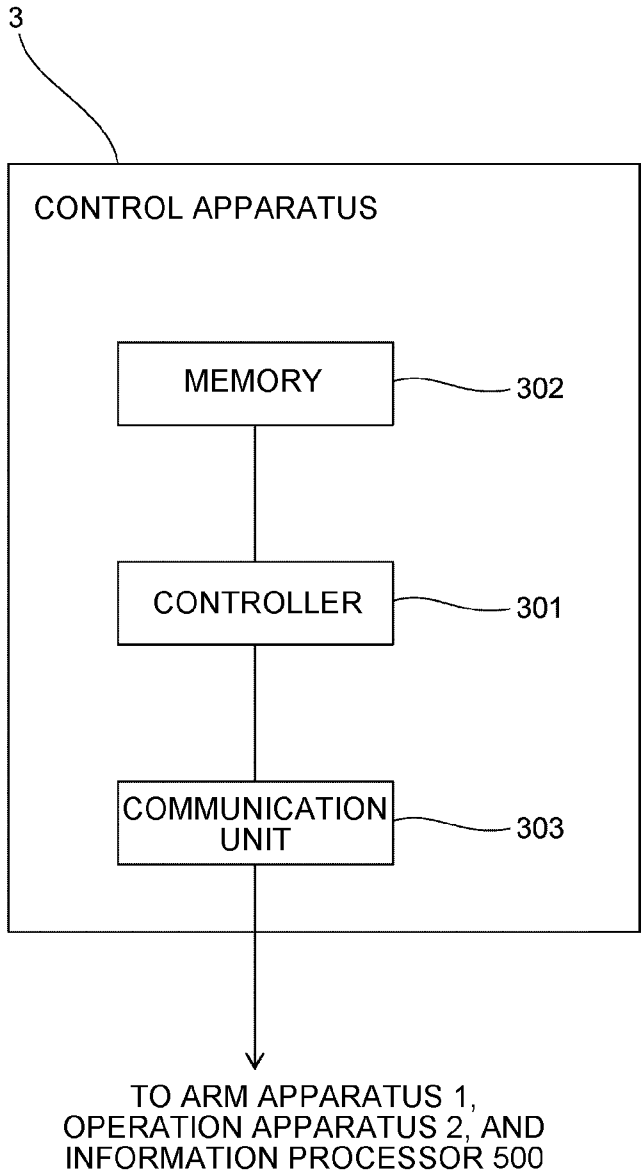
3
CONTROL APPARATUS
MEMORY
302
CONTROLLER
301
COMMUNICATION UNIT
303
TO ARM APPARATUS 1,
OPERATION APPARATUS 2, AND
INFORMATION PROCESSOR 500

FIG.6

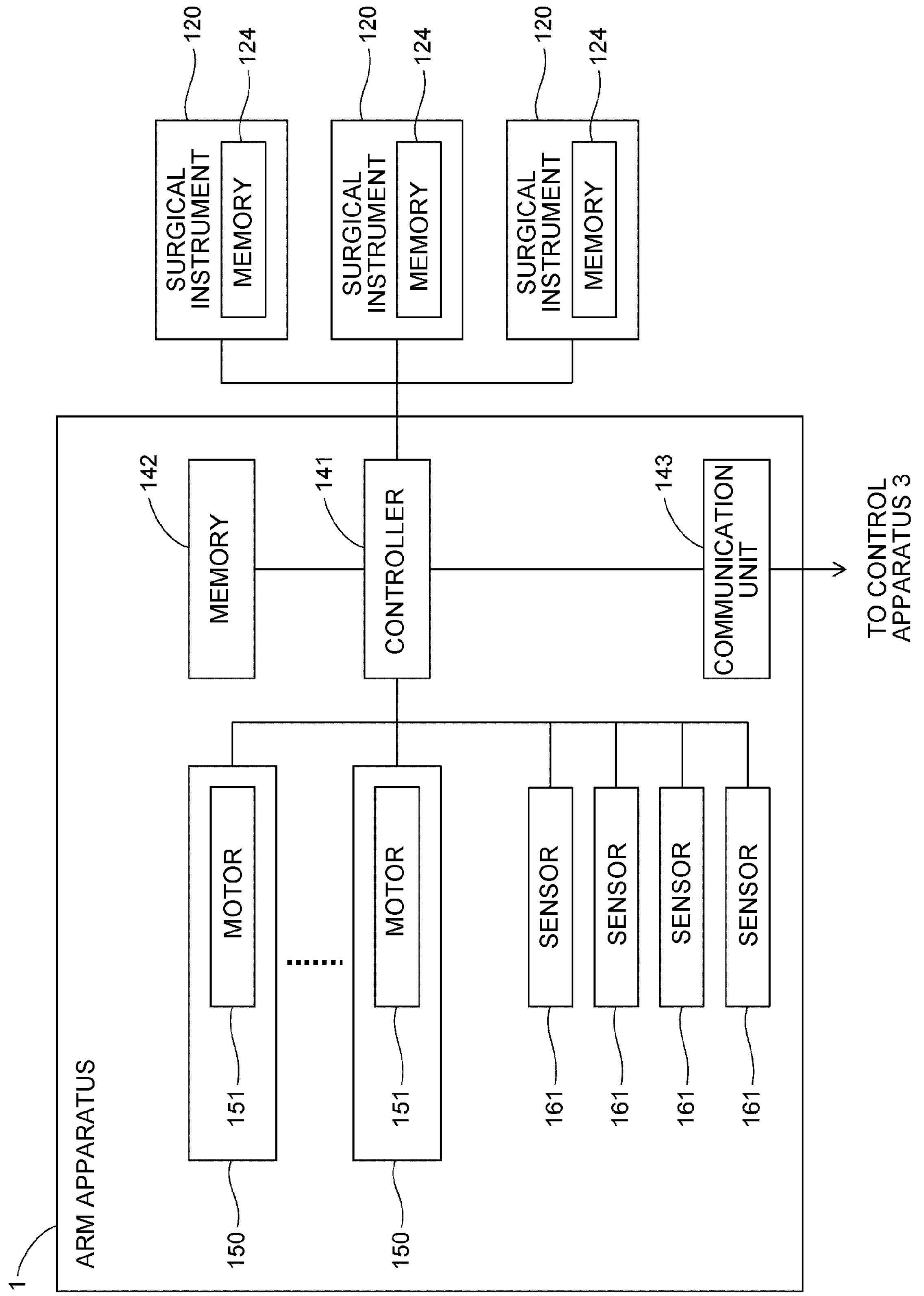
1
ARM APPARATUS
150
151
MOTOR
150
151
MOTOR
161
SENSOR
161
SENSOR
161
SENSOR
161
SENSOR
142
MEMORY
141
CONTROLLER
143
COMMUNICATION UNIT
TO CONTROL APPARATUS 3
120
124
SURGICAL INSTRUMENT
MEMORY
120
124
SURGICAL INSTRUMENT
MEMORY
120
124
SURGICAL INSTRUMENT
MEMORY

FIG.7

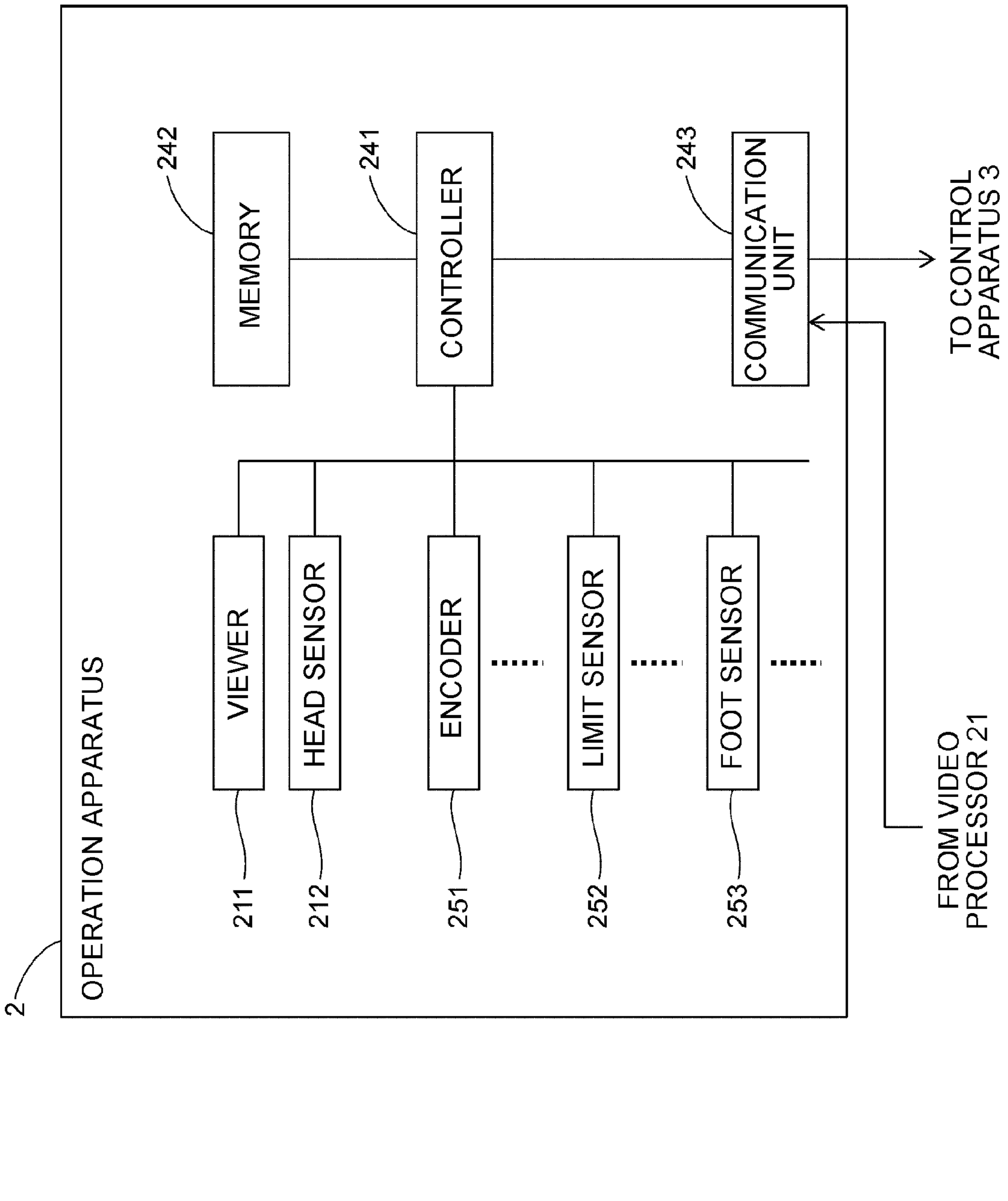

2
OPERATION APPARATUS
MEMORY
242
CONTROLLER
241
COMMUNICATION UNIT
243
TO CONTROL APPARATUS 3
VIEWER
211
HEAD SENSOR
212
ENCODER
251
LIMIT SENSOR
252
FOOT SENSOR
253
FROM VIDEO PROCESSOR 21

FIG.8

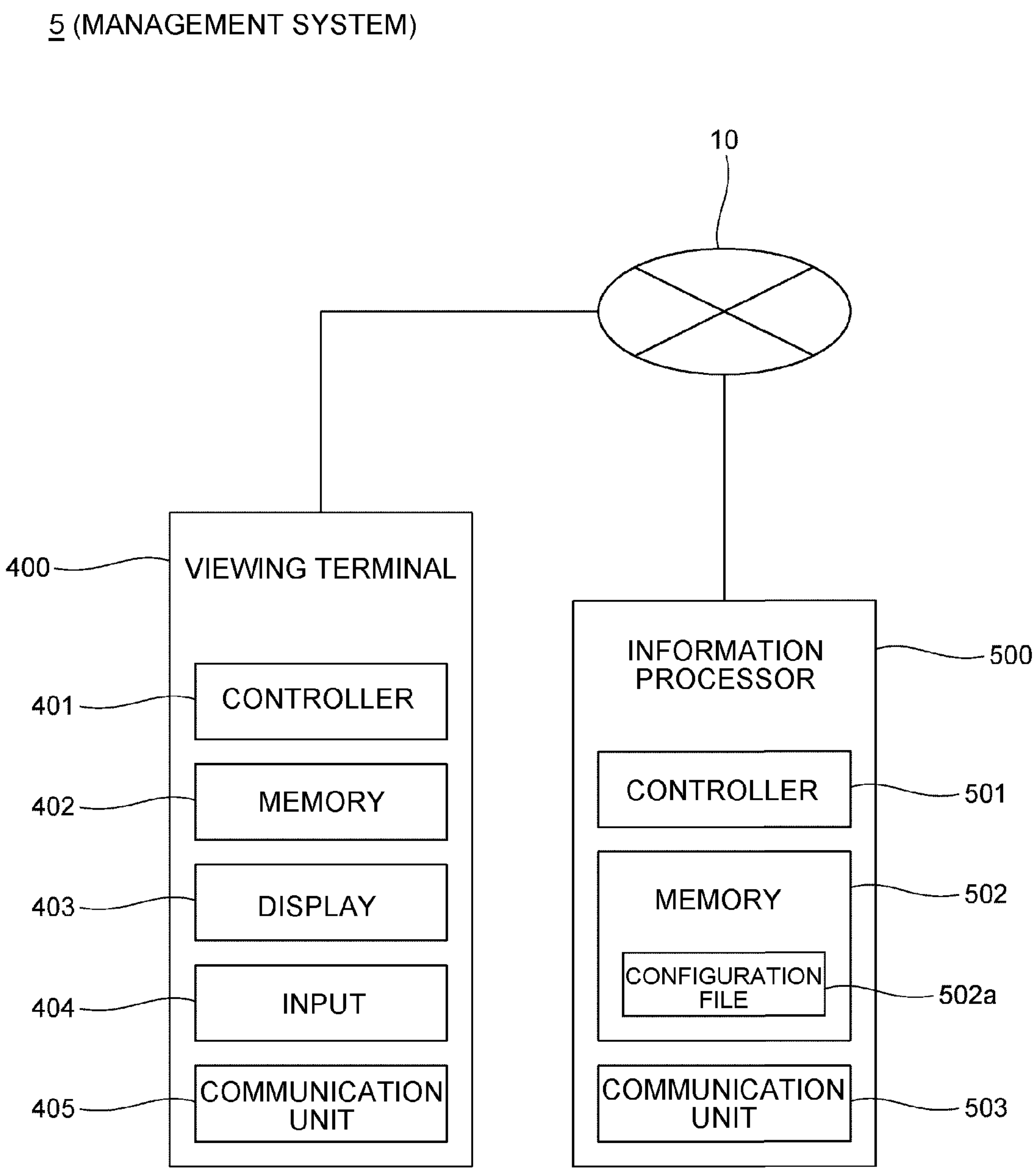
5 (MANAGEMENT SYSTEM)
10
400
VIEWING TERMINAL
401
CONTROLLER
402
MEMORY
403
DISPLAY
404
INPUT
405
COMMUNICATION UNIT
INFORMATION PROCESSOR
500
CONTROLLER
501
MEMORY
502
CONFIGURATION FILE
502a
COMMUNICATION UNIT
503

FIG.9

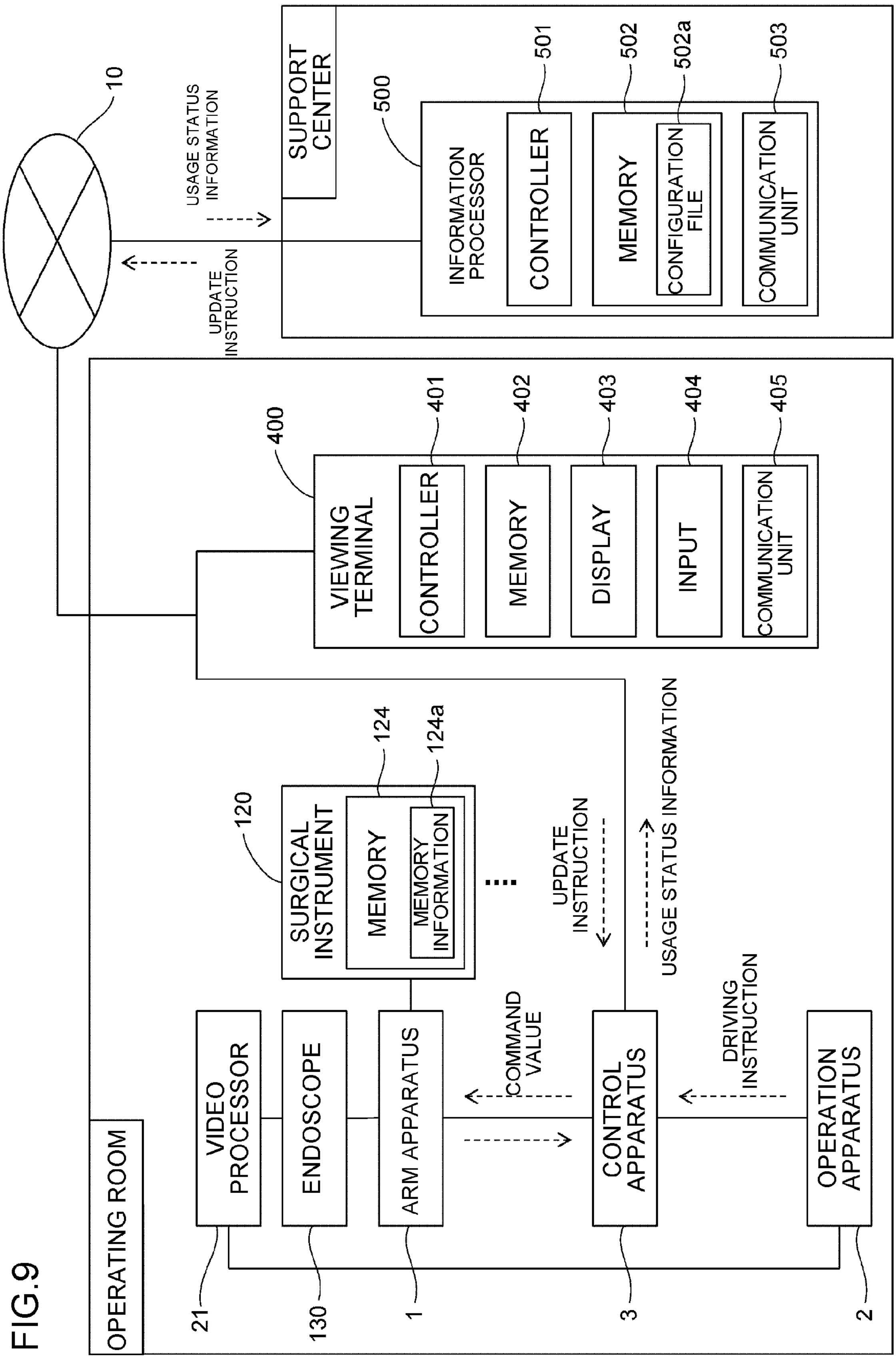
OPERATING ROOM
VIDEO PROCESSOR
ENDOSCOPE
ARM APPARATUS
CONTROL APPARATUS
OPERATION APPARATUS
21
130
1
3
2
COMMAND VALUE
DRIVING INSTRUCTION
SURGICAL INSTRUMENT
MEMORY
MEMORY INFORMATION
120
124
124a
UPDATE INSTRUCTION
USAGE STATUS INFORMATION
VIEWING TERMINAL
CONTROLLER
MEMORY
DISPLAY
INPUT
COMMUNICATION UNIT
400
401
402
403
404
405
10
UPDATE INSTRUCTION
USAGE STATUS INFORMATION
SUPPORT CENTER
INFORMATION PROCESSOR
CONTROLLER
MEMORY
CONFIGURATION FILE
COMMUNICATION UNIT
500
501
502
502a
503

FIG.10

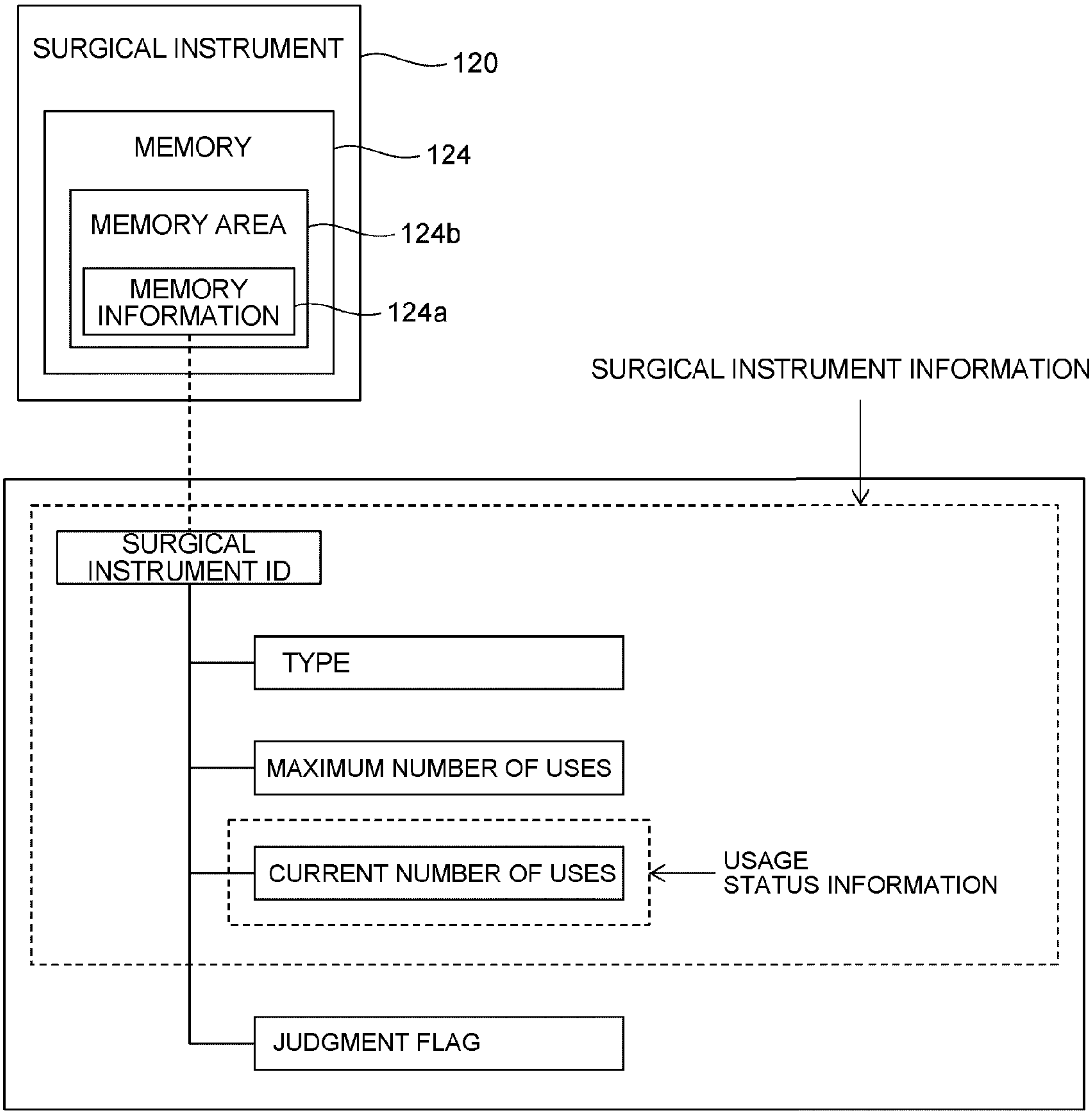
SURGICAL INSTRUMENT
120
MEMORY
124
MEMORY AREA
124b
MEMORY INFORMATION
124a
SURGICAL INSTRUMENT INFORMATION
SURGICAL INSTRUMENT ID
TYPE
MAXIMUM NUMBER OF USES
CURRENT NUMBER OF USES
USAGE STATUS INFORMATION
JUDGMENT FLAG

FIG.11

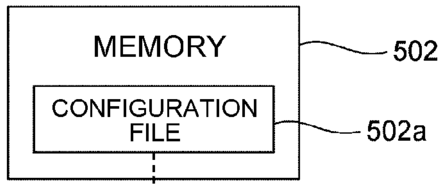


| SURGICAL INSTRUMENT ID | TYPE | MAXIMUM NUMBER OF USES | CURRENT NUMBER OF USES | JUDGMENT FLAG |
|---|---|---|---|---|
| SI001 | FORCEPS A | 10 | 4 | 0 |
| SI002 | FORCEPS A | 10 | 6 | 1 |
| SI003 | FORCEPS A | 10 | 10 | 1 |
| ... | ... | ... | ... | ... |

FIG.12

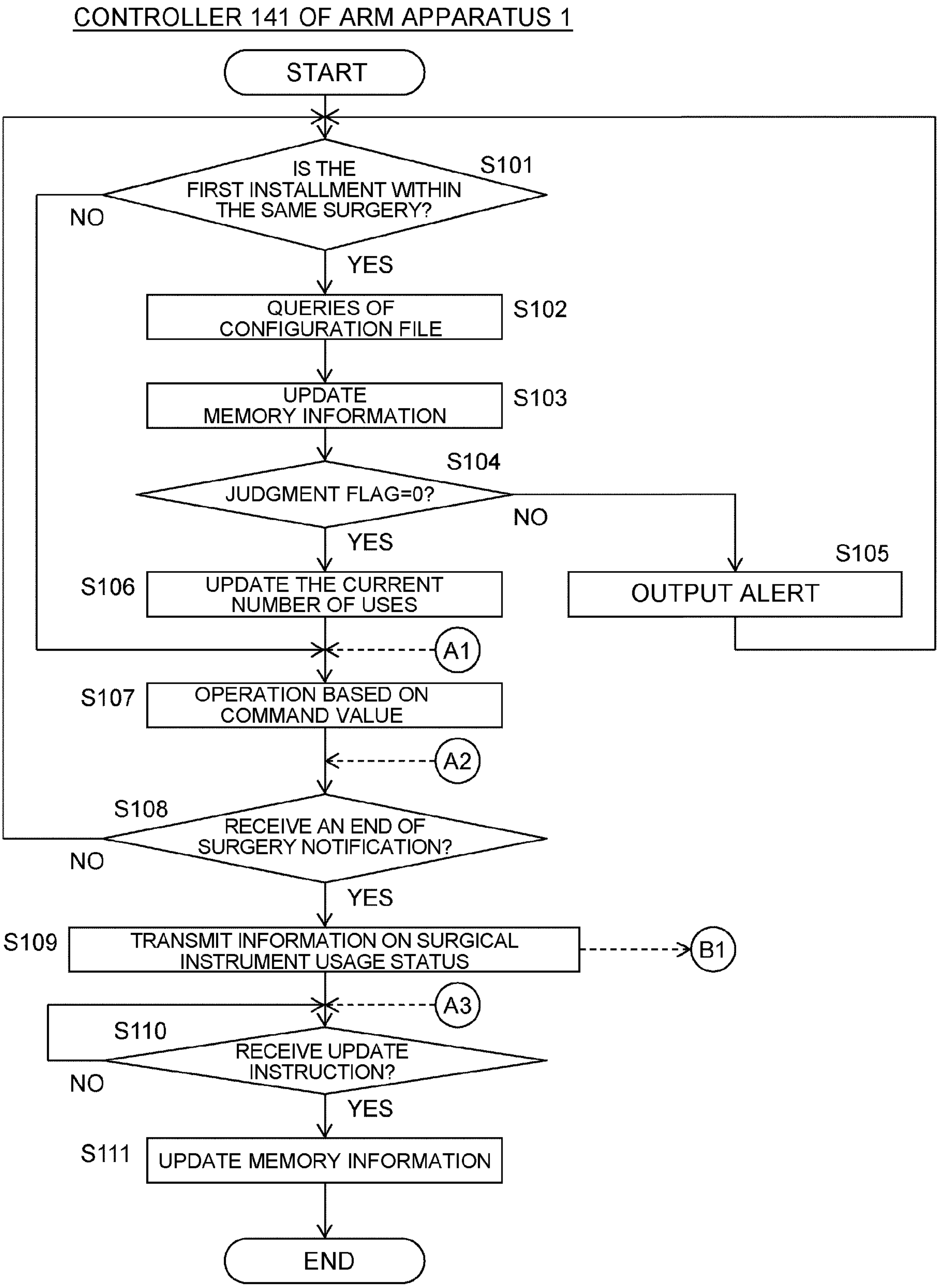
CONTROLLER 141 OF ARM APPARATUS 1
START
IS THE FIRST INSTALLMENT WITHIN THE SAME SURGERY?
S101
NO
YES
QUERIES OF CONFIGURATION FILE
S102
UPDATE MEMORY INFORMATION
S103
S104
JUDGMENT FLAG=0?
NO
YES
S105
S106
UPDATE THE CURRENT NUMBER OF USES
OUTPUT ALERT
A1
S107
OPERATION BASED ON COMMAND VALUE
A2
S108
RECEIVE AN END OF SURGERY NOTIFICATION?
NO
YES
S109
TRANSMIT INFORMATION ON SURGICAL INSTRUMENT USAGE STATUS
B1
A3
S110
RECEIVE UPDATE INSTRUCTION?
NO
YES
S111
UPDATE MEMORY INFORMATION
END

FIG.13

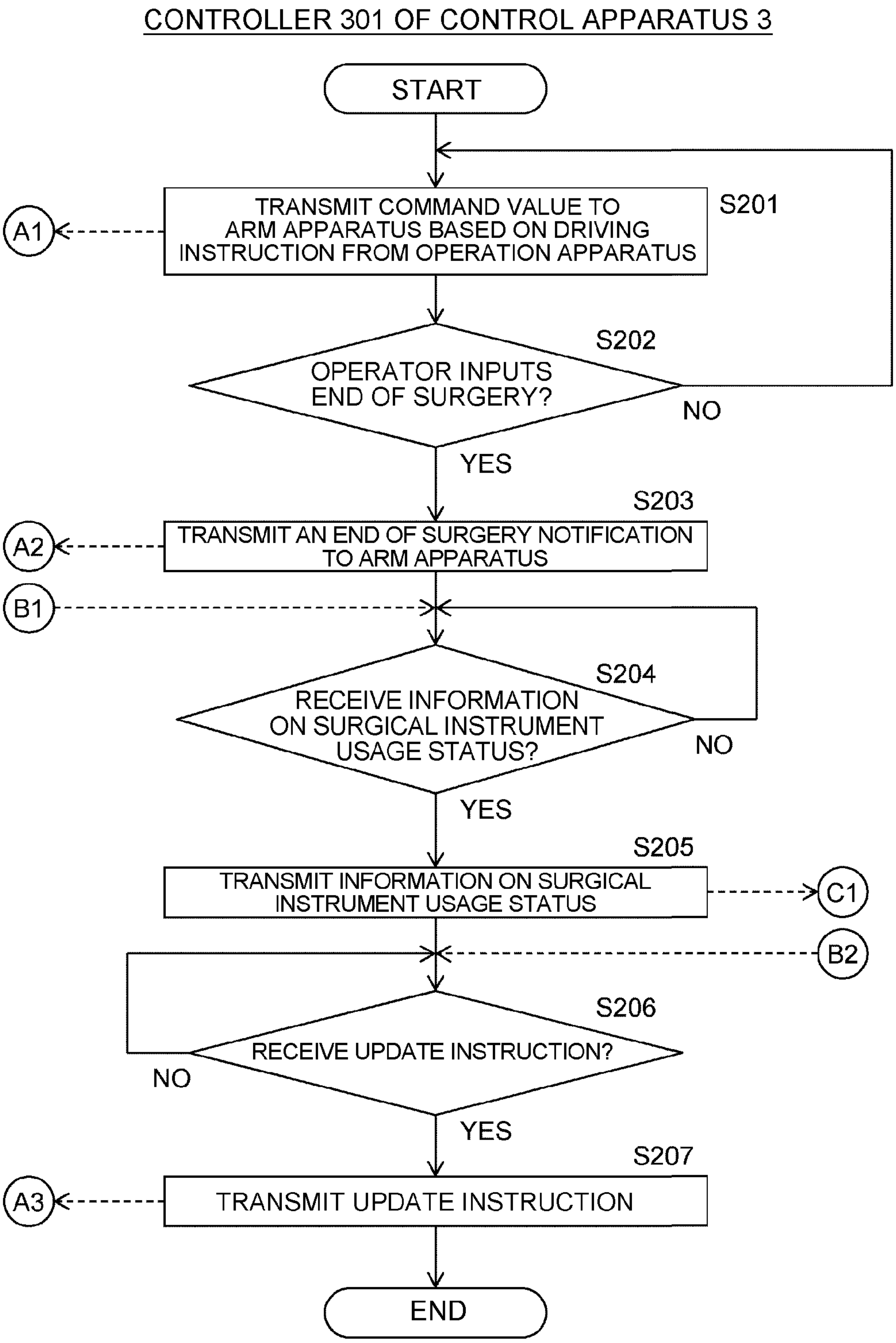
CONTROLLER 301 OF CONTROL APPARATUS 3
START
TRANSMIT COMMAND VALUE TO ARM APPARATUS BASED ON DRIVING INSTRUCTION FROM OPERATION APPARATUS
S201
A1
OPERATOR INPUTS END OF SURGERY?
S202
NO
YES
TRANSMIT AN END OF SURGERY NOTIFICATION TO ARM APPARATUS
S203
A2
B1
RECEIVE INFORMATION ON SURGICAL INSTRUMENT USAGE STATUS?
S204
NO
YES
TRANSMIT INFORMATION ON SURGICAL INSTRUMENT USAGE STATUS
S205
C1
B2
RECEIVE UPDATE INSTRUCTION?
S206
NO
YES
TRANSMIT UPDATE INSTRUCTION
S207
A3
END

FIG.14

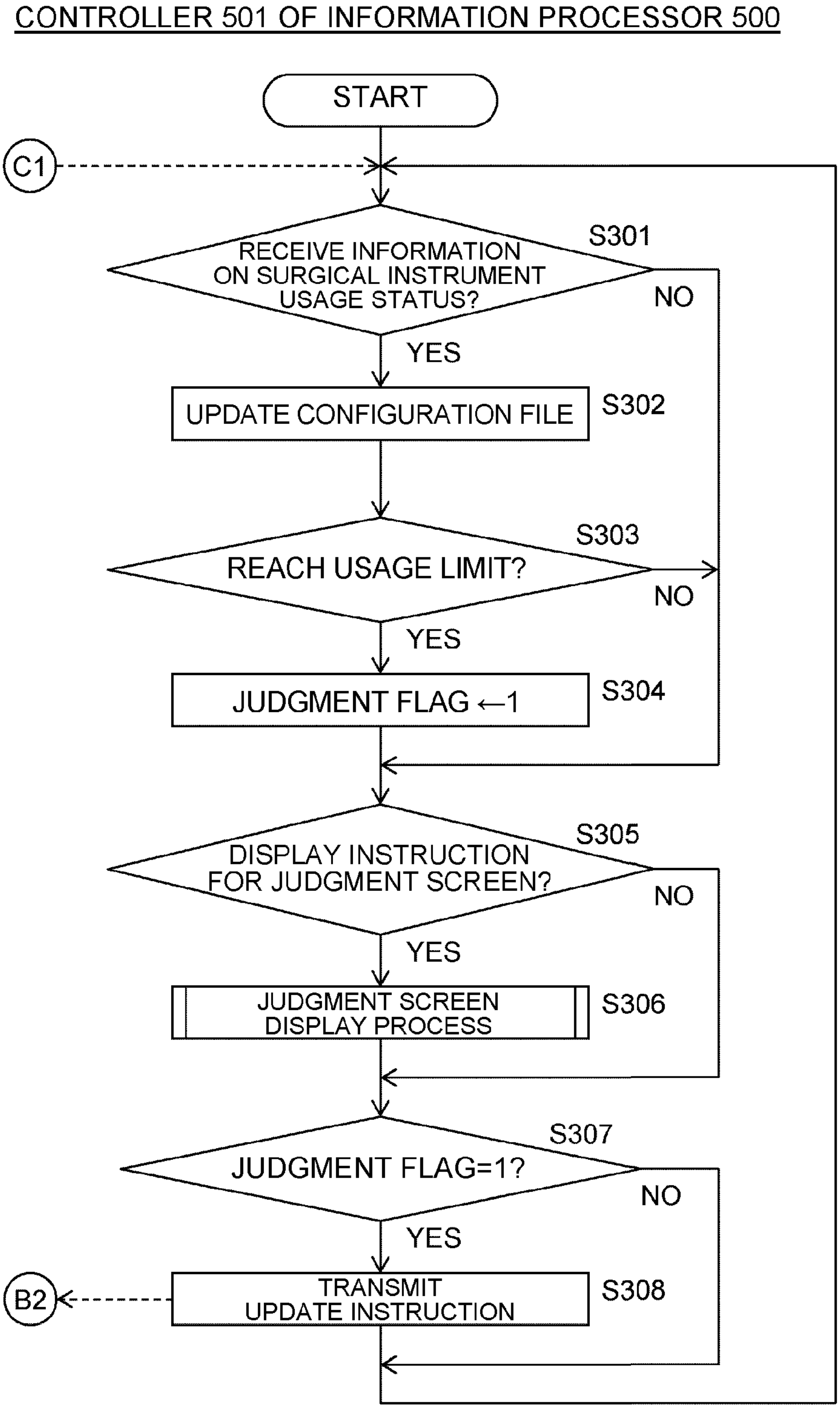
CONTROLLER 501 OF INFORMATION PROCESSOR 500
START
C1
RECEIVE INFORMATION ON SURGICAL INSTRUMENT USAGE STATUS?
S301
NO
YES
UPDATE CONFIGURATION FILE
S302
REACH USAGE LIMIT?
S303
NO
YES
JUDGMENT FLAG ←1
S304
DISPLAY INSTRUCTION FOR JUDGMENT SCREEN?
S305
NO
YES
JUDGMENT SCREEN DISPLAY PROCESS
S306
JUDGMENT FLAG=1?
S307
NO
YES
TRANSMIT UPDATE INSTRUCTION
S308
B2

FIG.15

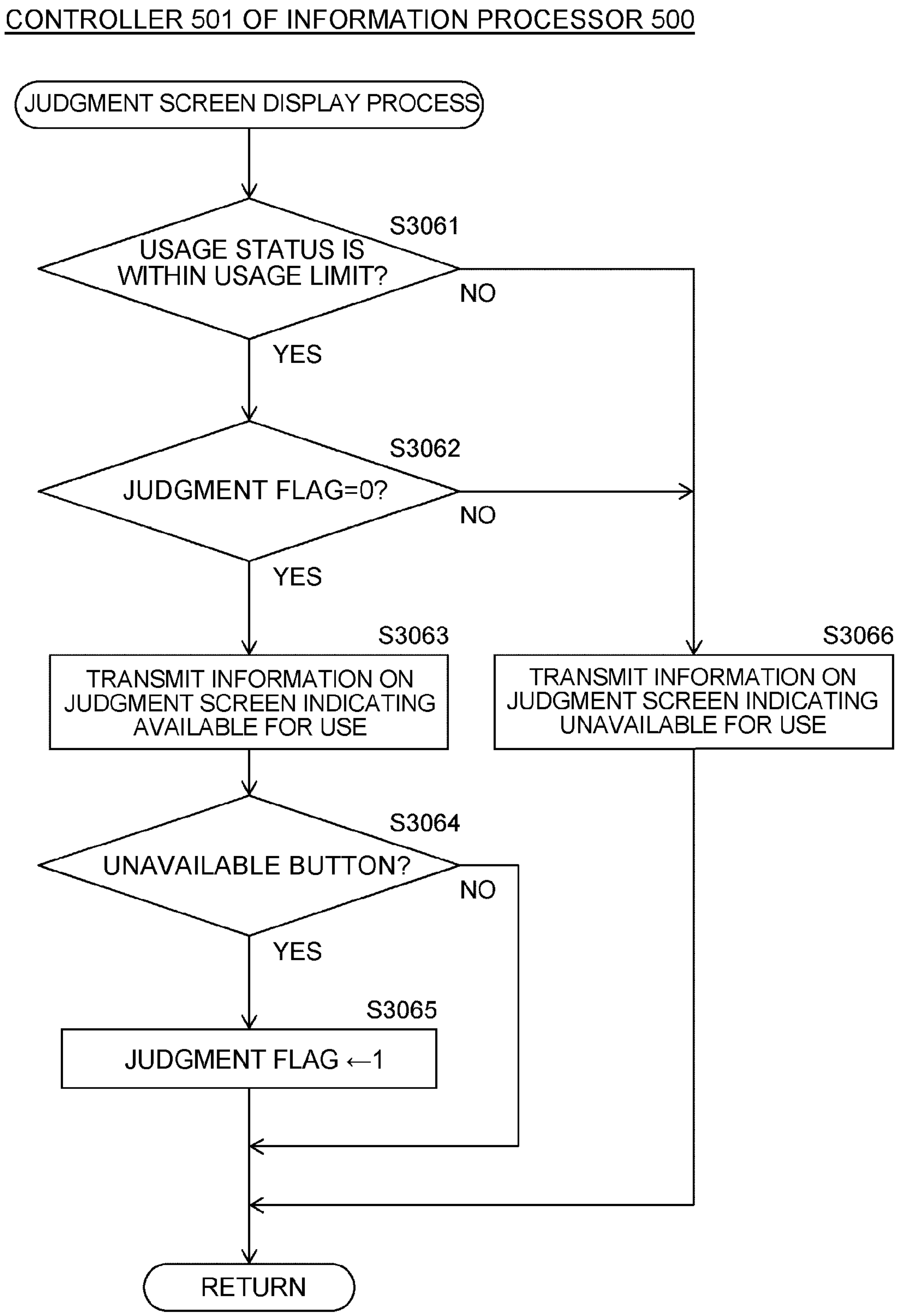
CONTROLLER 501 OF INFORMATION PROCESSOR 500
JUDGMENT SCREEN DISPLAY PROCESS
S3061
USAGE STATUS IS WITHIN USAGE LIMIT?
NO
YES
S3062
JUDGMENT FLAG=0?
NO
YES
S3063
TRANSMIT INFORMATION ON JUDGMENT SCREEN INDICATING AVAILABLE FOR USE
S3066
TRANSMIT INFORMATION ON JUDGMENT SCREEN INDICATING UNAVAILABLE FOR USE
S3064
UNAVAILABLE BUTTON?
NO
YES
S3065
JUDGMENT FLAG ←1
RETURN

FIG.16

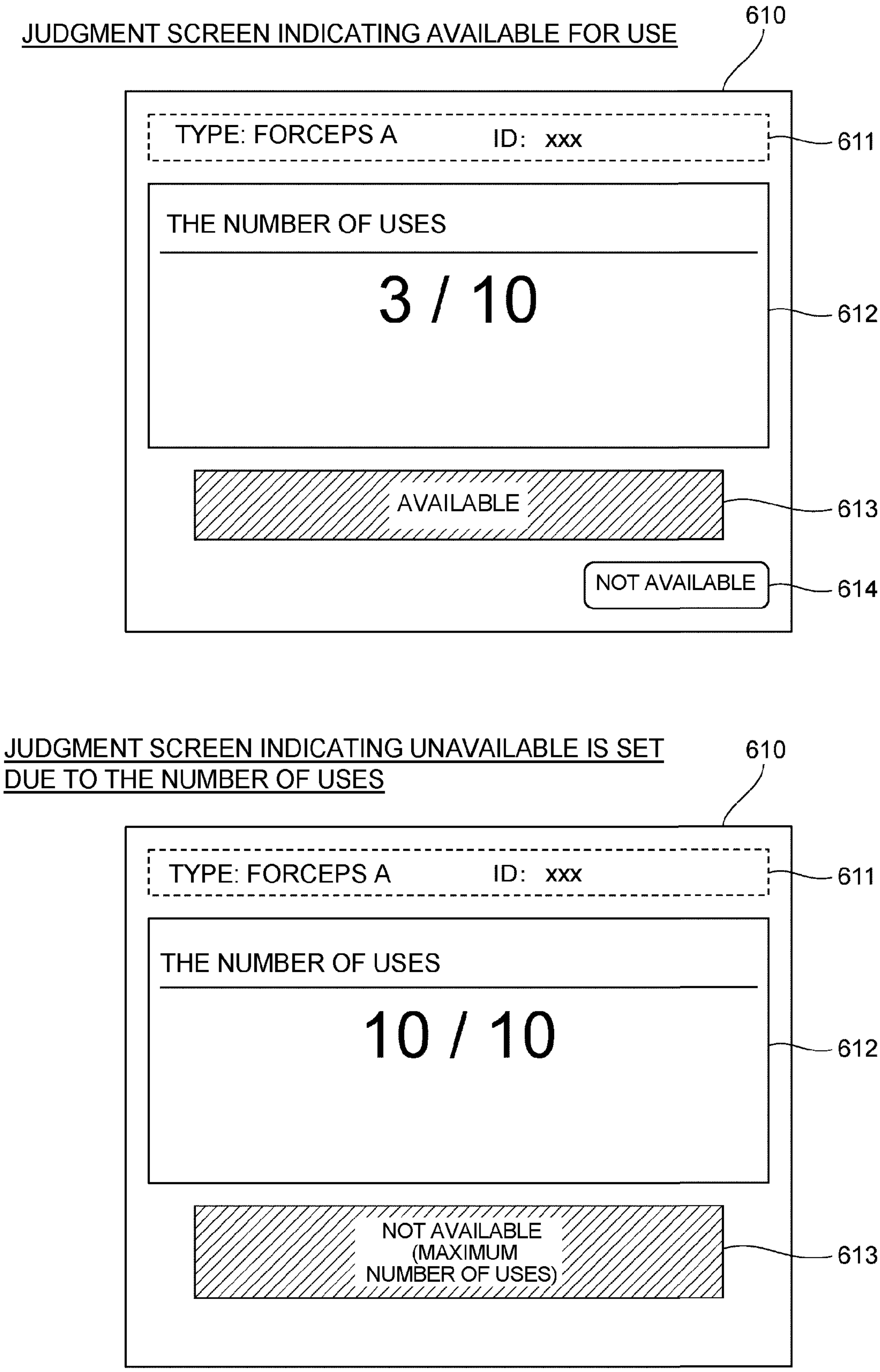
JUDGMENT SCREEN INDICATING AVAILABLE FOR USE
610
TYPE: FORCEPS A
ID: xxx
611
THE NUMBER OF USES
3 / 10
612
AVAILABLE
613
NOT AVAILABLE
614
JUDGMENT SCREEN INDICATING UNAVAILABLE IS SET DUE TO THE NUMBER OF USES
610
TYPE: FORCEPS A
ID: xxx
611
THE NUMBER OF USES
10 / 10
612
NOT AVAILABLE (MAXIMUM NUMBER OF USES)
613

FIG.17

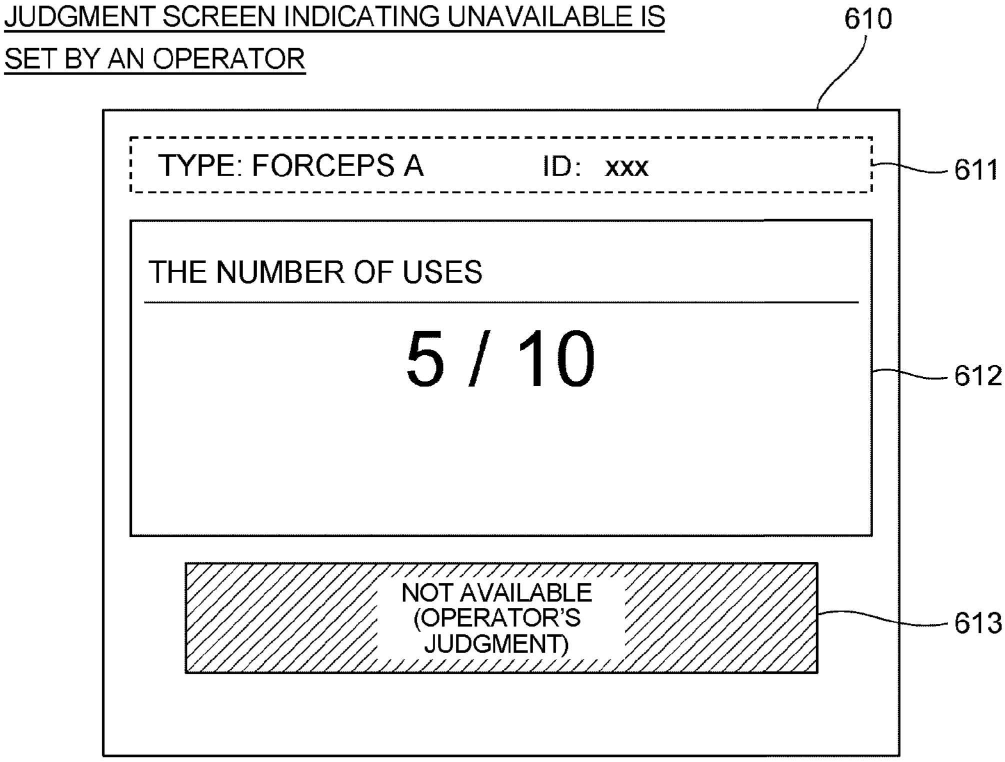
JUDGMENT SCREEN INDICATING UNAVAILABLE IS SET BY AN OPERATOR
610
TYPE: FORCEPS A
ID: xxx
611
THE NUMBER OF USES
5 / 10
612
NOT AVAILABLE (OPERATOR'S JUDGMENT)
613

FIG.18

VARIATION OF EMBODIMENT 1

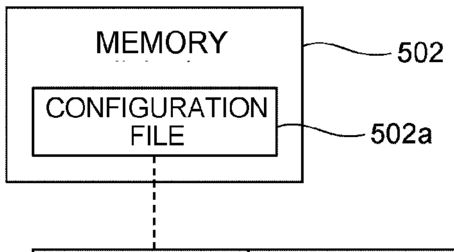


| SURGICAL INSTRUMENT ID | TYPE | MAXIMUM NUMBER OF USES | CURRENT NUMBER OF USES | USAGE TIME | JUDGMENT FLAG |
|---|---|---|---|---|---|
| SI001 | FORCEPS A | 10 | 4 | 2:25:10 | 0 |
| SI002 | FORCEPS A | 10 | 6 | 4:40:15 | 1 |
| SI003 | FORCEPS A | 10 | 10 | 8:00:30 | 1 |
| ... | ... | ... | ... | ... | ... |

FIG.19

JUDGMENT SCREEN INDICATING AVAILABLE FOR USE

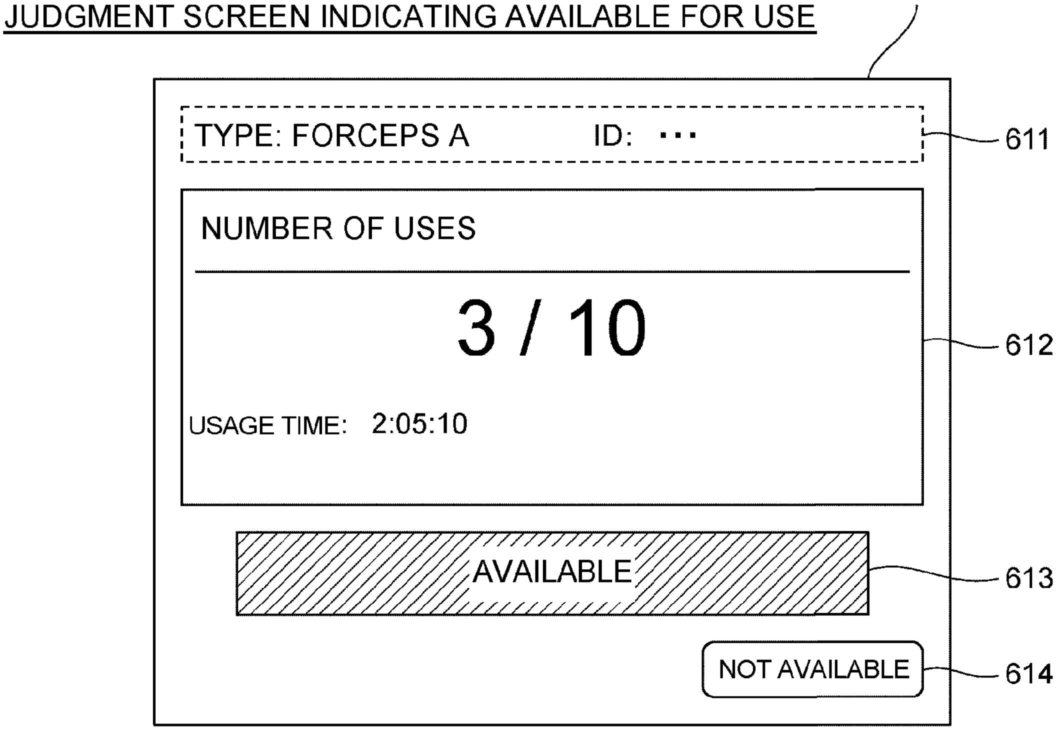


JUDGEMENT SCREEN INDICATING
UNAVAILABLE FOR USE DUE TO USAGE TIME

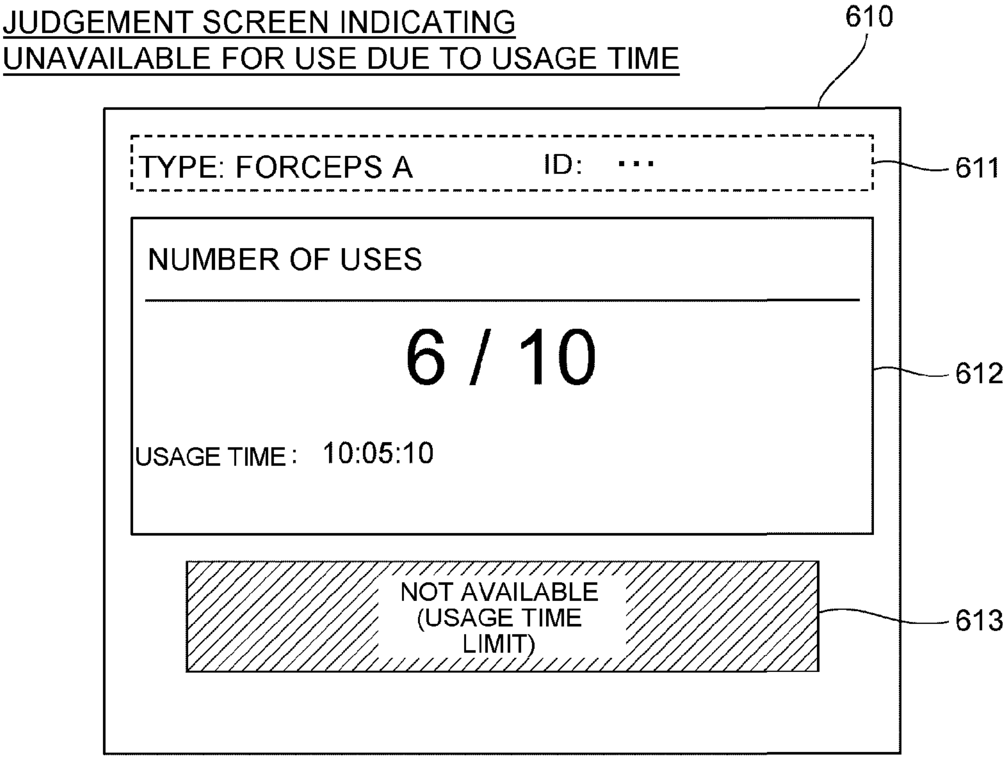

FIG.20

EMBODIMENT 2

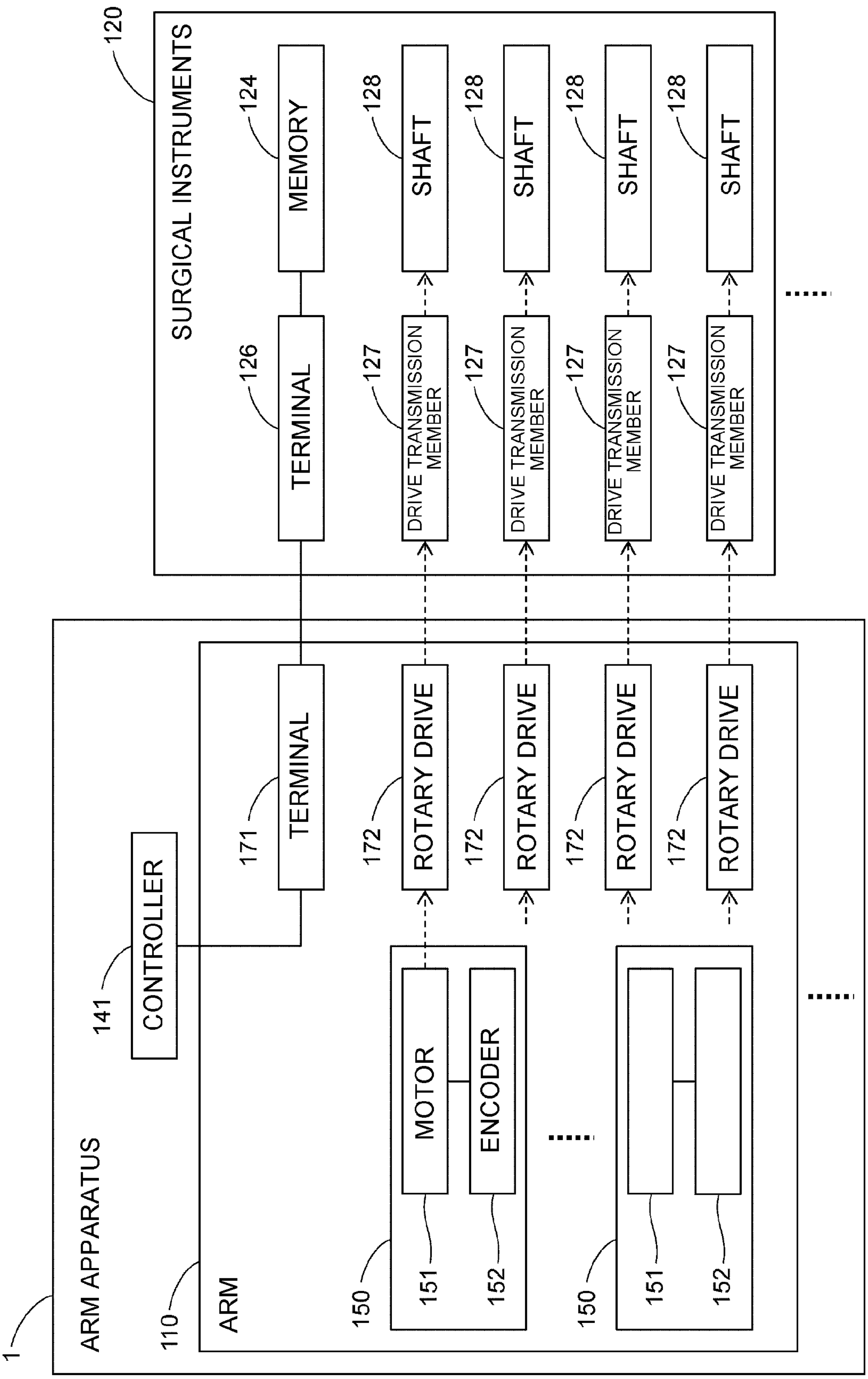

FIG.21

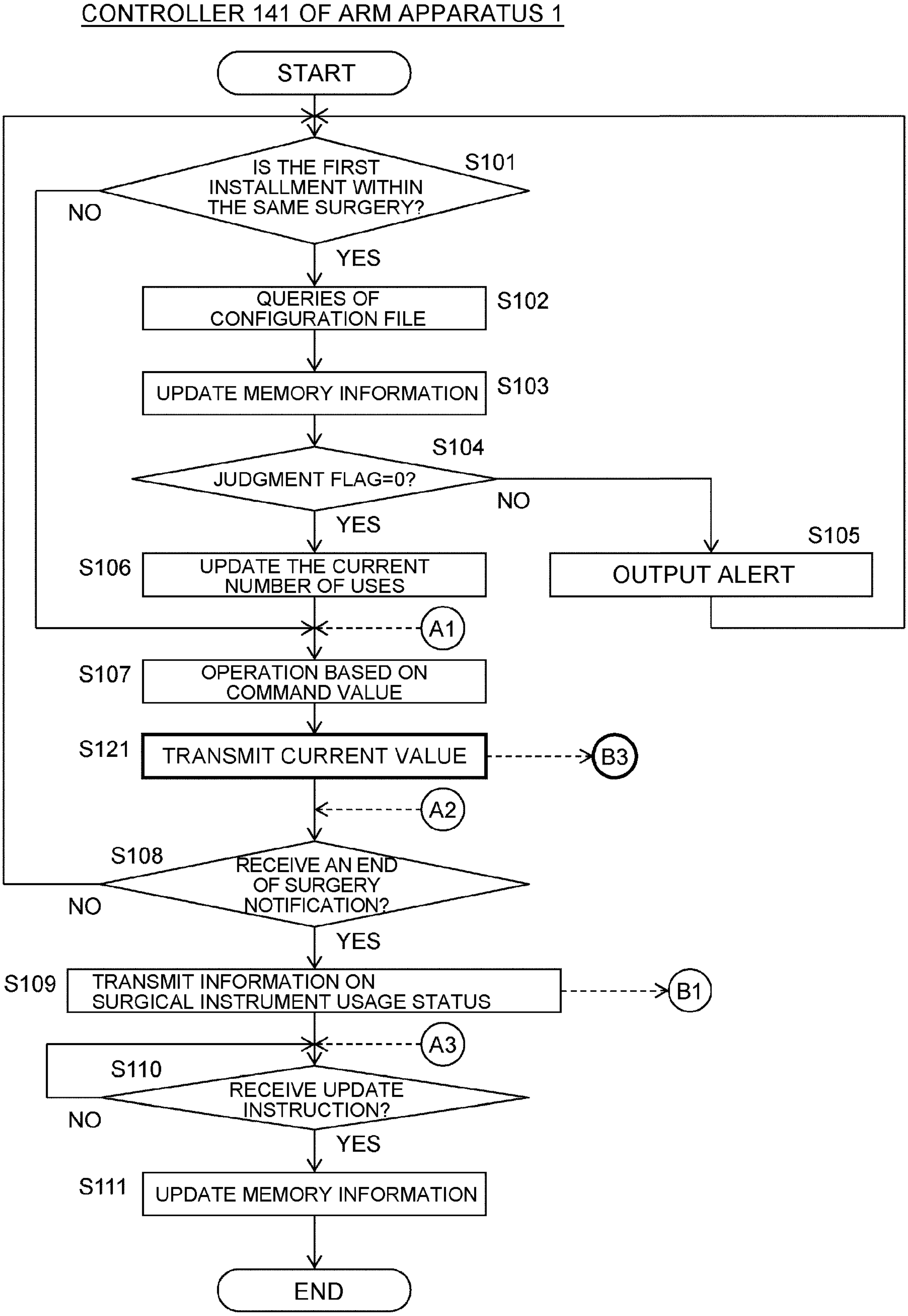
CONTROLLER 141 OF ARM APPARATUS 1
START
IS THE FIRST INSTALLMENT WITHIN THE SAME SURGERY?
S101
NO
YES
QUERIES OF CONFIGURATION FILE
S102
UPDATE MEMORY INFORMATION
S103
S104
JUDGMENT FLAG=0?
NO
YES
S105
S106
UPDATE THE CURRENT NUMBER OF USES
OUTPUT ALERT
A1
S107
OPERATION BASED ON COMMAND VALUE
S121
TRANSMIT CURRENT VALUE
B3
A2
S108
RECEIVE AN END OF SURGERY NOTIFICATION?
NO
YES
S109
TRANSMIT INFORMATION ON SURGICAL INSTRUMENT USAGE STATUS
B1
A3
S110
RECEIVE UPDATE INSTRUCTION?
NO
YES
S111
UPDATE MEMORY INFORMATION
END

FIG.22

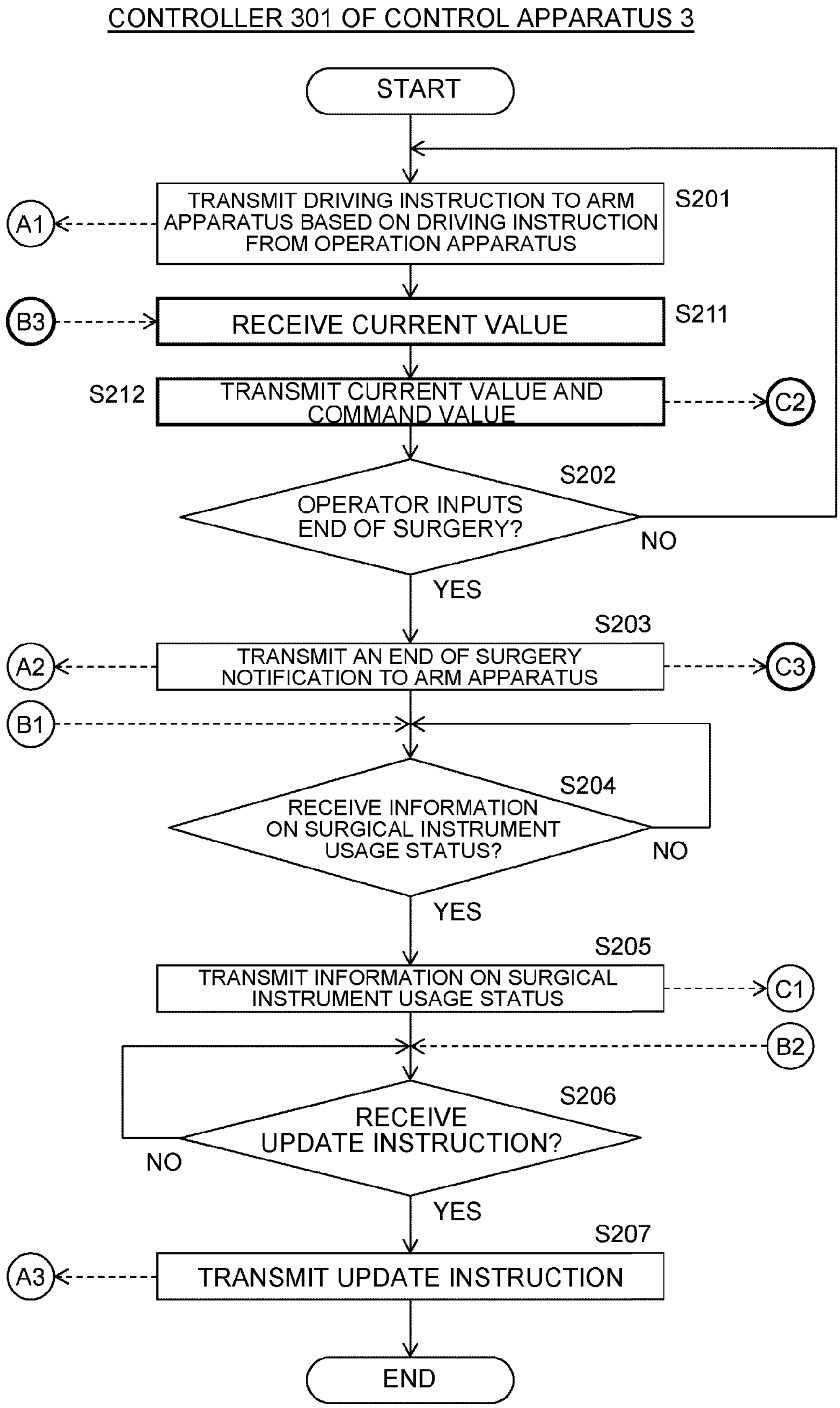
CONTROLLER 301 OF CONTROL APPARATUS 3
START
TRANSMIT DRIVING INSTRUCTION TO ARM APPARATUS BASED ON DRIVING INSTRUCTION FROM OPERATION APPARATUS
S201
A1
RECEIVE CURRENT VALUE
S211
B3
S212
TRANSMIT CURRENT VALUE AND COMMAND VALUE
C2
OPERATOR INPUTS END OF SURGERY?
S202
NO
YES
S203
TRANSMIT AN END OF SURGERY NOTIFICATION TO ARM APPARATUS
A2
C3
B1
RECEIVE INFORMATION ON SURGICAL INSTRUMENT USAGE STATUS?
S204
NO
YES
S205
TRANSMIT INFORMATION ON SURGICAL INSTRUMENT USAGE STATUS
C1
B2
RECEIVE UPDATE INSTRUCTION?
S206
NO
YES
S207
TRANSMIT UPDATE INSTRUCTION
A3
END

FIG.23

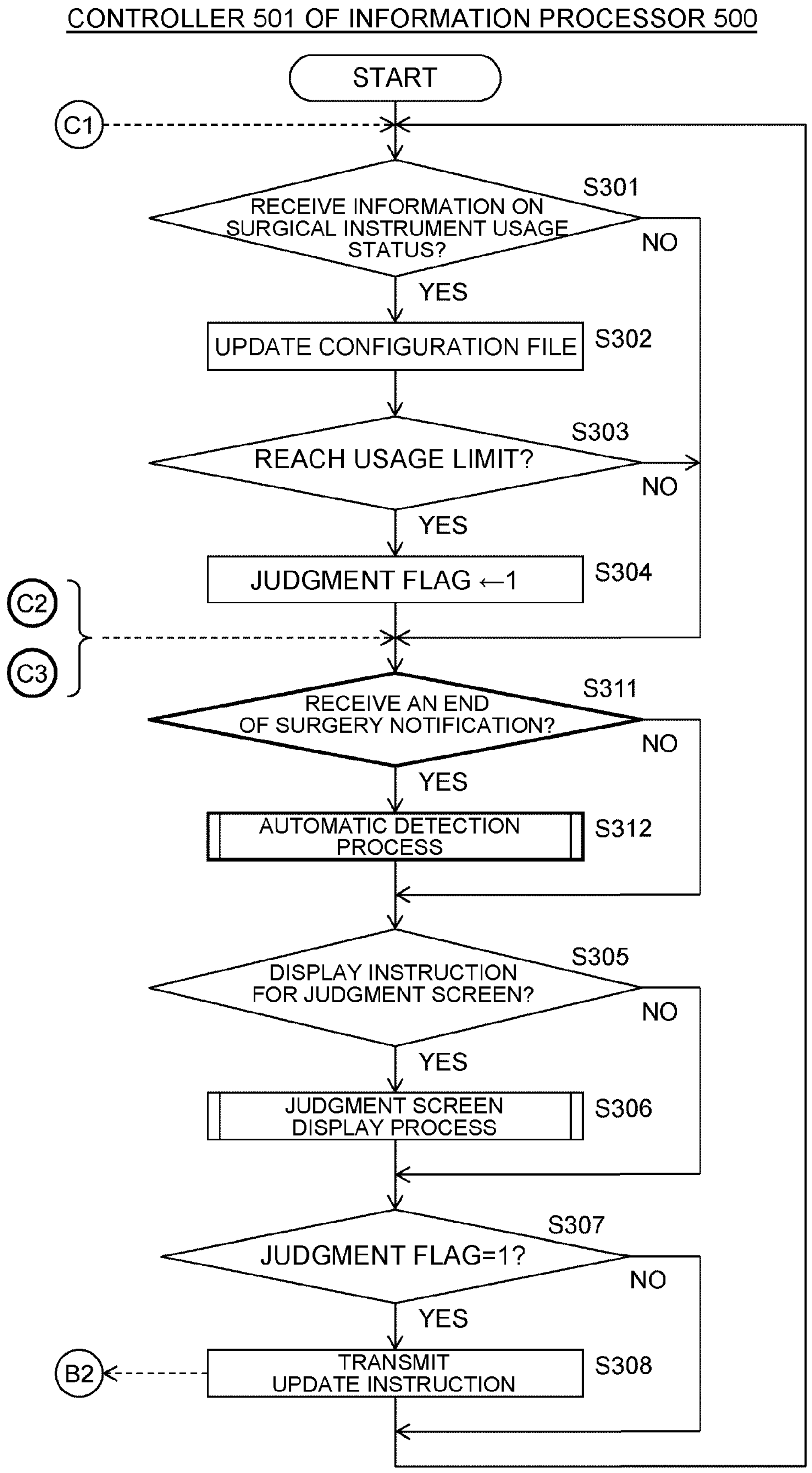
CONTROLLER 501 OF INFORMATION PROCESSOR 500
START
C1
RECEIVE INFORMATION ON SURGICAL INSTRUMENT USAGE STATUS?
S301
NO
YES
UPDATE CONFIGURATION FILE
S302
REACH USAGE LIMIT?
S303
NO
YES
JUDGMENT FLAG ←1
S304
C2
C3
RECEIVE AN END OF SURGERY NOTIFICATION?
S311
NO
YES
AUTOMATIC DETECTION PROCESS
S312
DISPLAY INSTRUCTION FOR JUDGMENT SCREEN?
S305
NO
YES
JUDGMENT SCREEN DISPLAY PROCESS
S306
JUDGMENT FLAG=1?
S307
NO
YES
TRANSMIT UPDATE INSTRUCTION
S308
B2

FIG.24

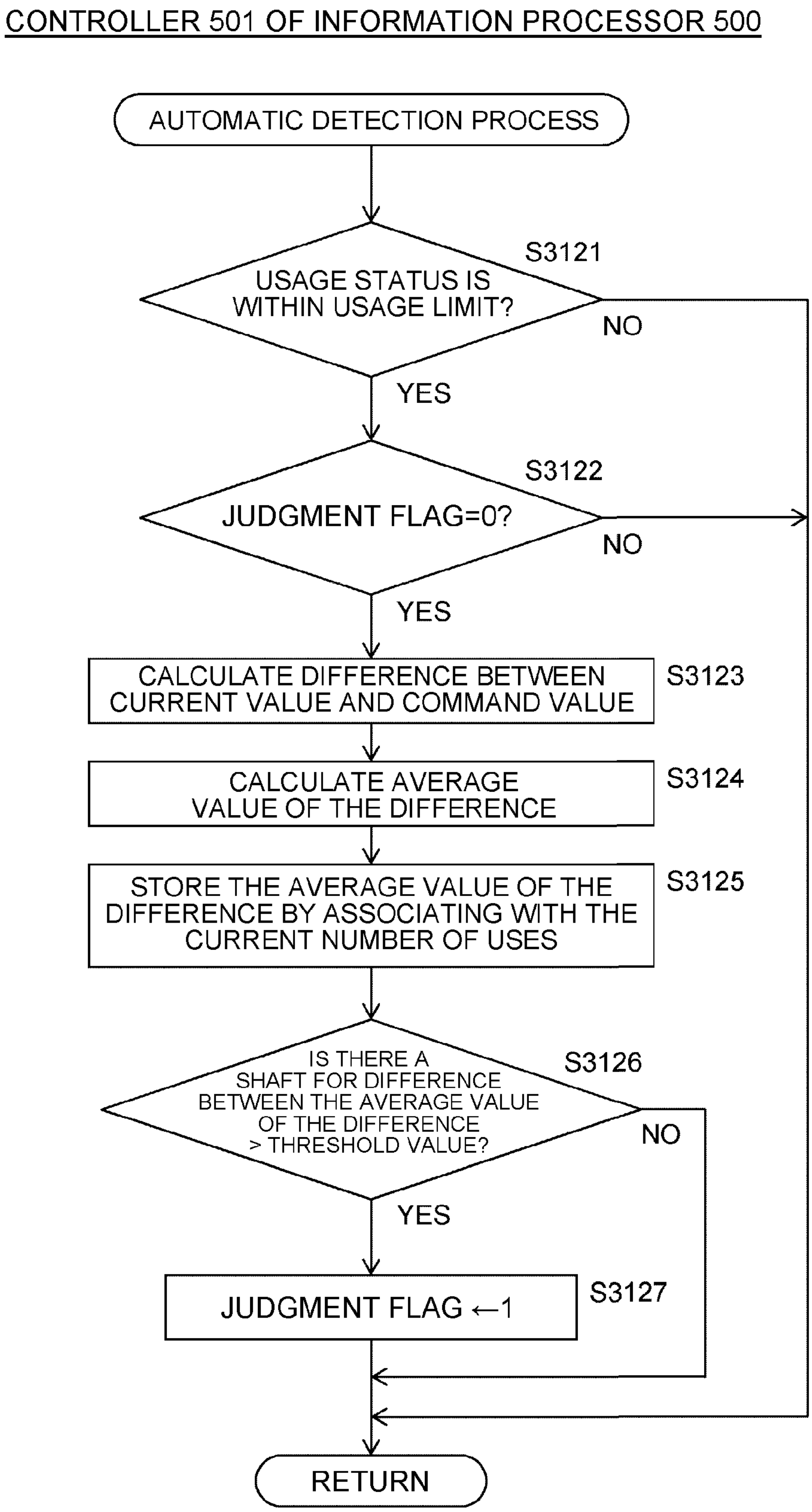
CONTROLLER 501 OF INFORMATION PROCESSOR 500
AUTOMATIC DETECTION PROCESS
S3121
USAGE STATUS IS WITHIN USAGE LIMIT?
NO
YES
S3122
JUDGMENT FLAG=0?
NO
YES
CALCULATE DIFFERENCE BETWEEN CURRENT VALUE AND COMMAND VALUE
S3123
CALCULATE AVERAGE VALUE OF THE DIFFERENCE
S3124
STORE THE AVERAGE VALUE OF THE DIFFERENCE BY ASSOCIATING WITH THE CURRENT NUMBER OF USES
S3125
IS THERE A SHAFT FOR DIFFERENCE BETWEEN THE AVERAGE VALUE OF THE DIFFERENCE > THRESHOLD VALUE?
S3126
NO
YES
JUDGMENT FLAG ←1
S3127
RETURN

FIG.25

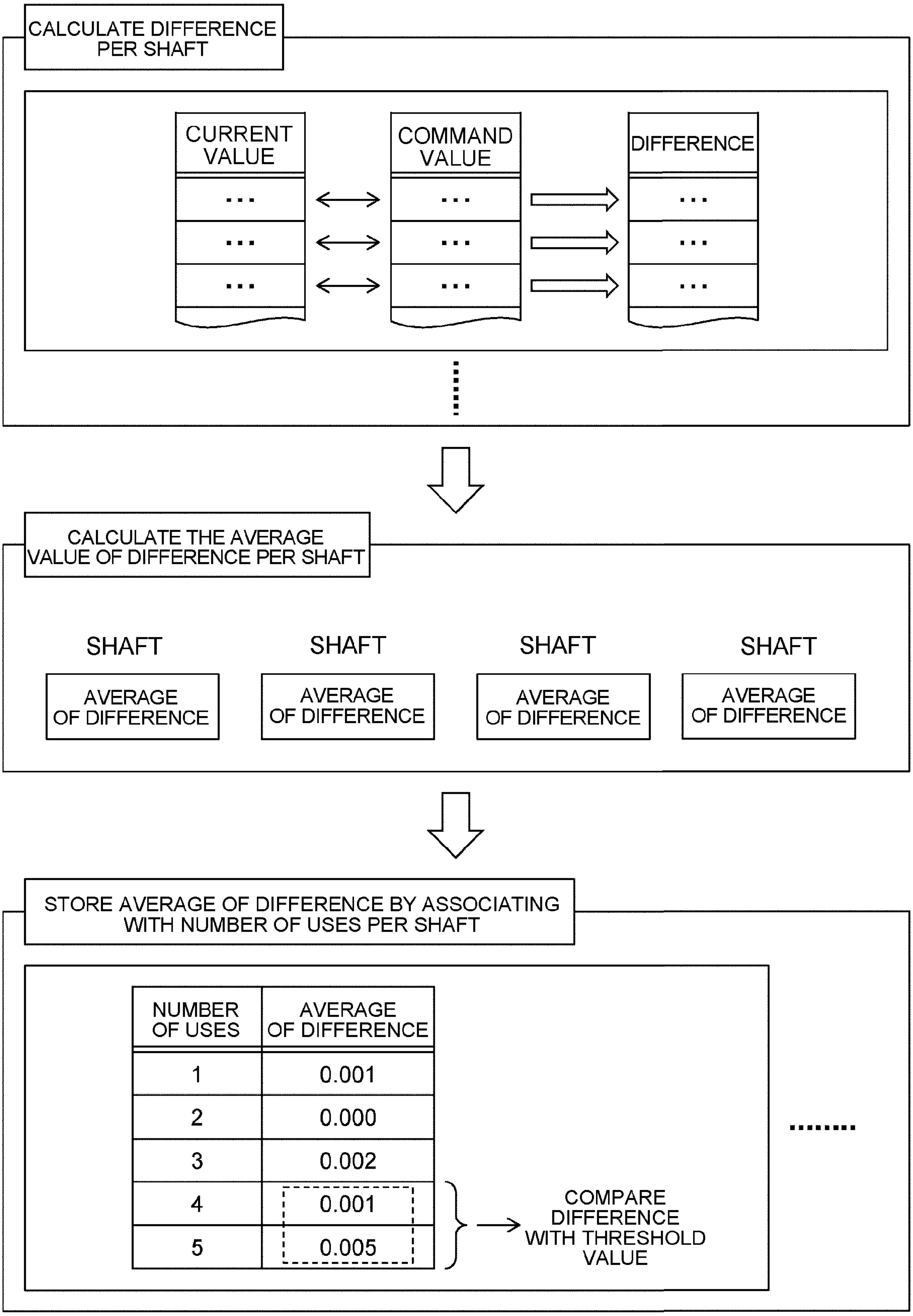
CALCULATE DIFFERENCE PER SHAFT
CURRENT VALUE
COMMAND VALUE
DIFFERENCE
...
CALCULATE THE AVERAGE VALUE OF DIFFERENCE PER SHAFT
SHAFT
AVERAGE OF DIFFERENCE
SHAFT
AVERAGE OF DIFFERENCE
SHAFT
AVERAGE OF DIFFERENCE
SHAFT
AVERAGE OF DIFFERENCE
STORE AVERAGE OF DIFFERENCE BY ASSOCIATING WITH NUMBER OF USES PER SHAFT
NUMBER OF USES
AVERAGE OF DIFFERENCE
1
0.001
2
0.000
3
0.002
4
0.001
5
0.005
COMPARE DIFFERENCE WITH THRESHOLD VALUE
........

FIG.26

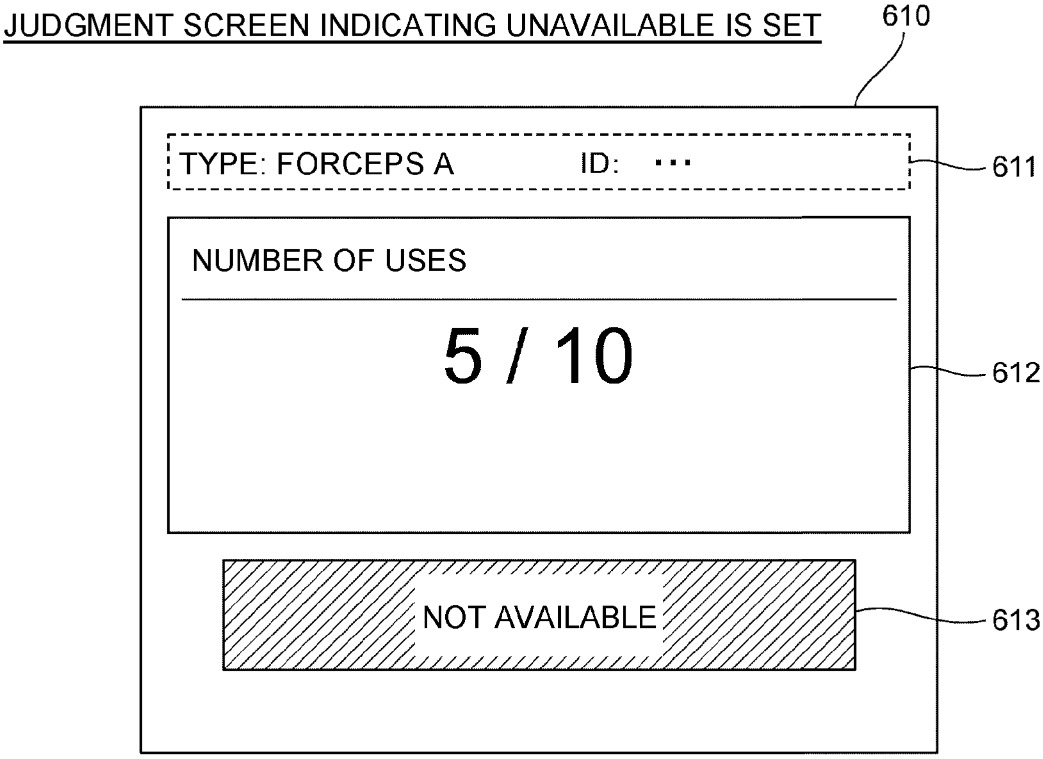
JUDGMENT SCREEN INDICATING UNAVAILABLE IS SET
610
TYPE: FORCEPS A
ID: ···
611
NUMBER OF USES
5 / 10
612
NOT AVAILABLE
613

FIG.27

EMBODIMENT 3

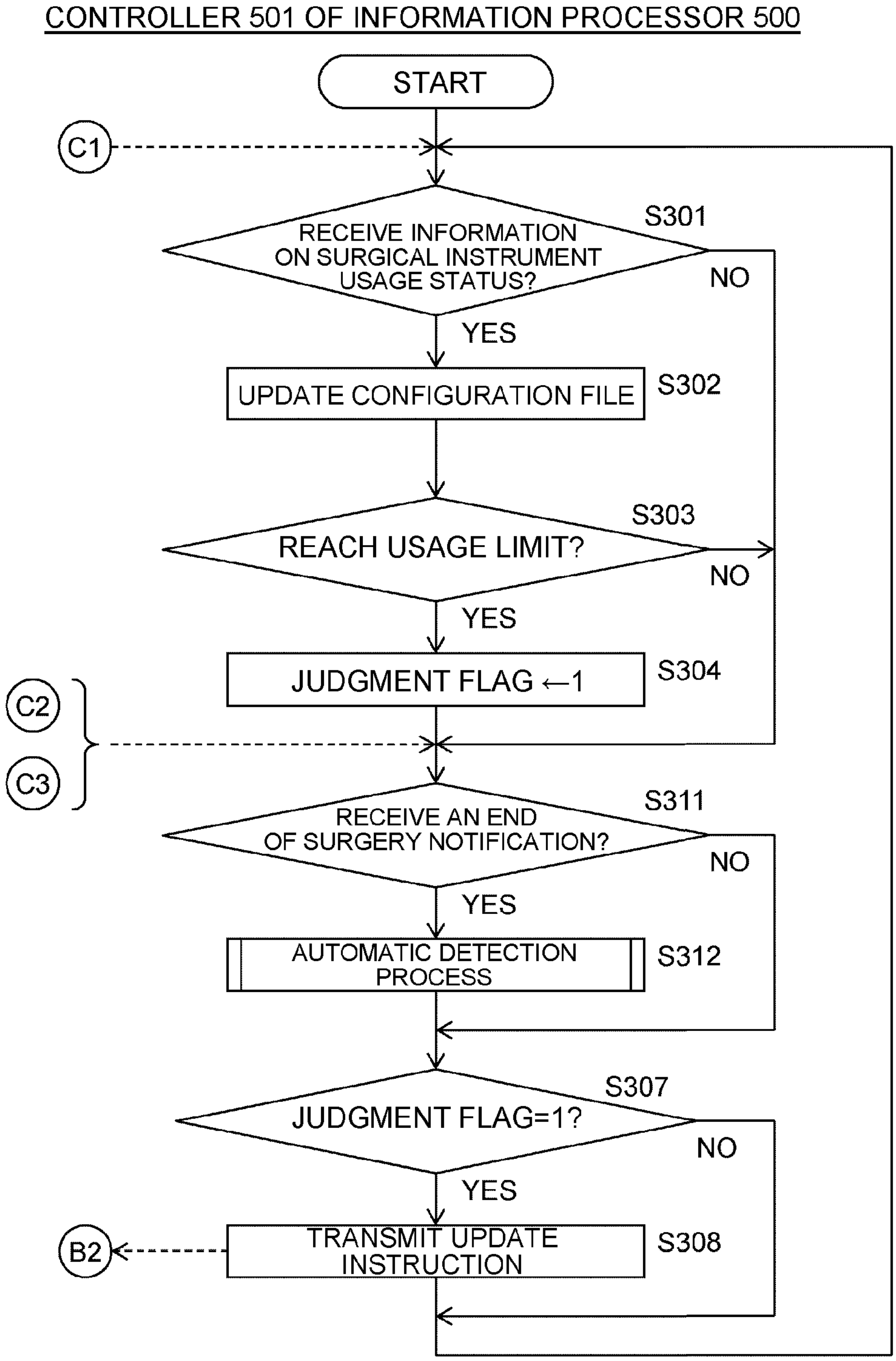

FIG.28

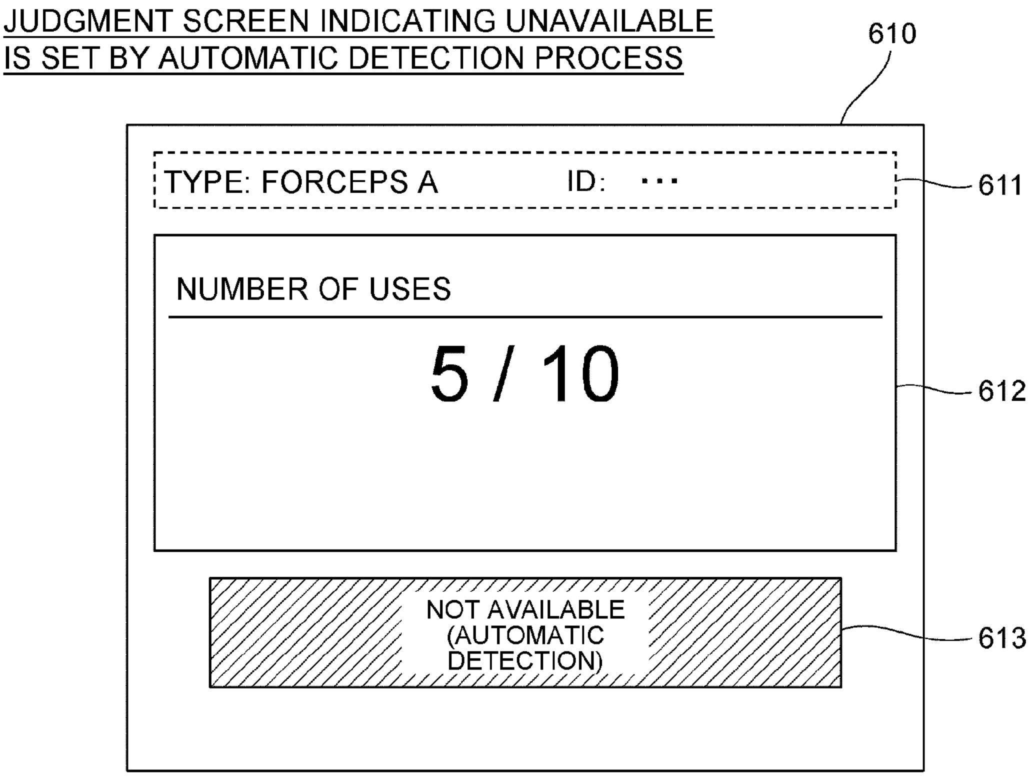
JUDGMENT SCREEN INDICATING UNAVAILABLE
IS SET BY AUTOMATIC DETECTION PROCESS
610
TYPE: FORCEPS A
ID: ···
611
NUMBER OF USES
5 / 10
612
NOT AVAILABLE
(AUTOMATIC
DETECTION)
613

FIG.29

EMBODIMENT 4

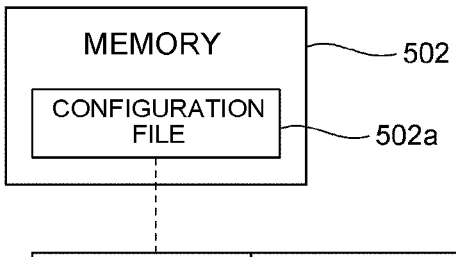


| SURGICAL INSTRUMENT ID | TYPE | MAXIMUM NUMBER OF USES | CURRENT NUMBER OF USES | JUDGMENT FLAG | UNAVAILABLE FACTOR |
|---|---|---|---|---|---|
| SI011 | FORCEPS A | 10 | 3 | 0 | 000 |
| SI012 | FORCEPS A | 10 | 10 | 1 | 001 |
| SI013 | FORCEPS A | 10 | 4 | 1 | 010 |
| SI014 | FORCEPS A | 10 | 6 | 1 | 011 |
| ... | ... | ... | ... | ... | ... |

FIG.30

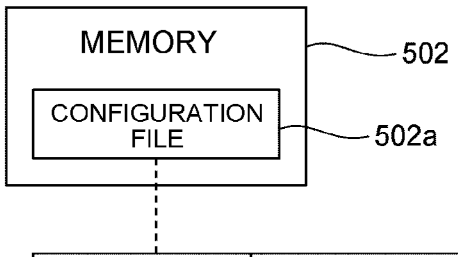


| SURGICAL INSTRUMENT ID | TYPE | MAXIMUM NUMBER OF USES | CURRENT NUMBER OF USES | FLAG A | FLAG B |
|---|---|---|---|---|---|
| SI011 | FORCEPS A | 10 | 3 | 0 | 0 |
| SI012 | FORCEPS A | 10 | 10 | 0 | 0 |
| SI013 | FORCEPS A | 10 | 4 | 1 | 0 |
| SI014 | FORCEPS A | 10 | 6 | 0 | 1 |
| ··· | ··· | ··· | ··· | ··· | ··· |

FIG.31

EMBODIMENT 5

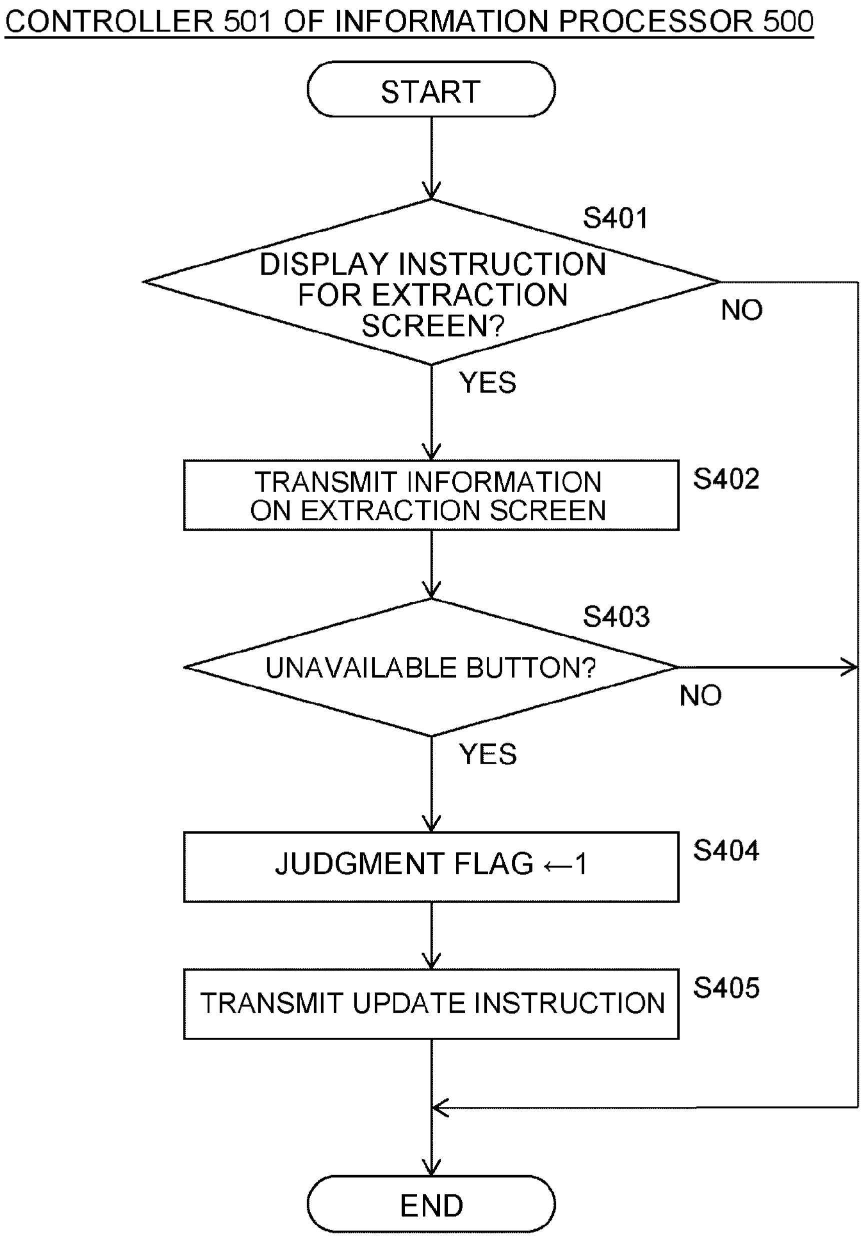

FIG.32

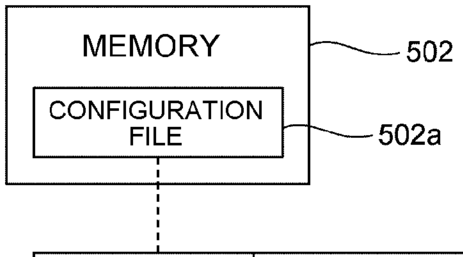


| SURGICAL INSTRUMENT ID | MANUFACTURER | TYPE | MAXIMUM NUMBER OF USES | CURRENT NUMBER OF USES | JUDGMENT FLAG |
|---|---|---|---|---|---|
| SI001 | MANUFACTURER 1 | FORCEPS A | 10 | 4 | 0 |
| SI002 | MANUFACTURER 1 | FORCEPS A | 10 | 6 | 1 |
| SI003 | MANUFACTURER 2 | FORCEPS A | 10 | 10 | 1 |
| ... | ... | ... | ... | ... | ... |

FIG.33

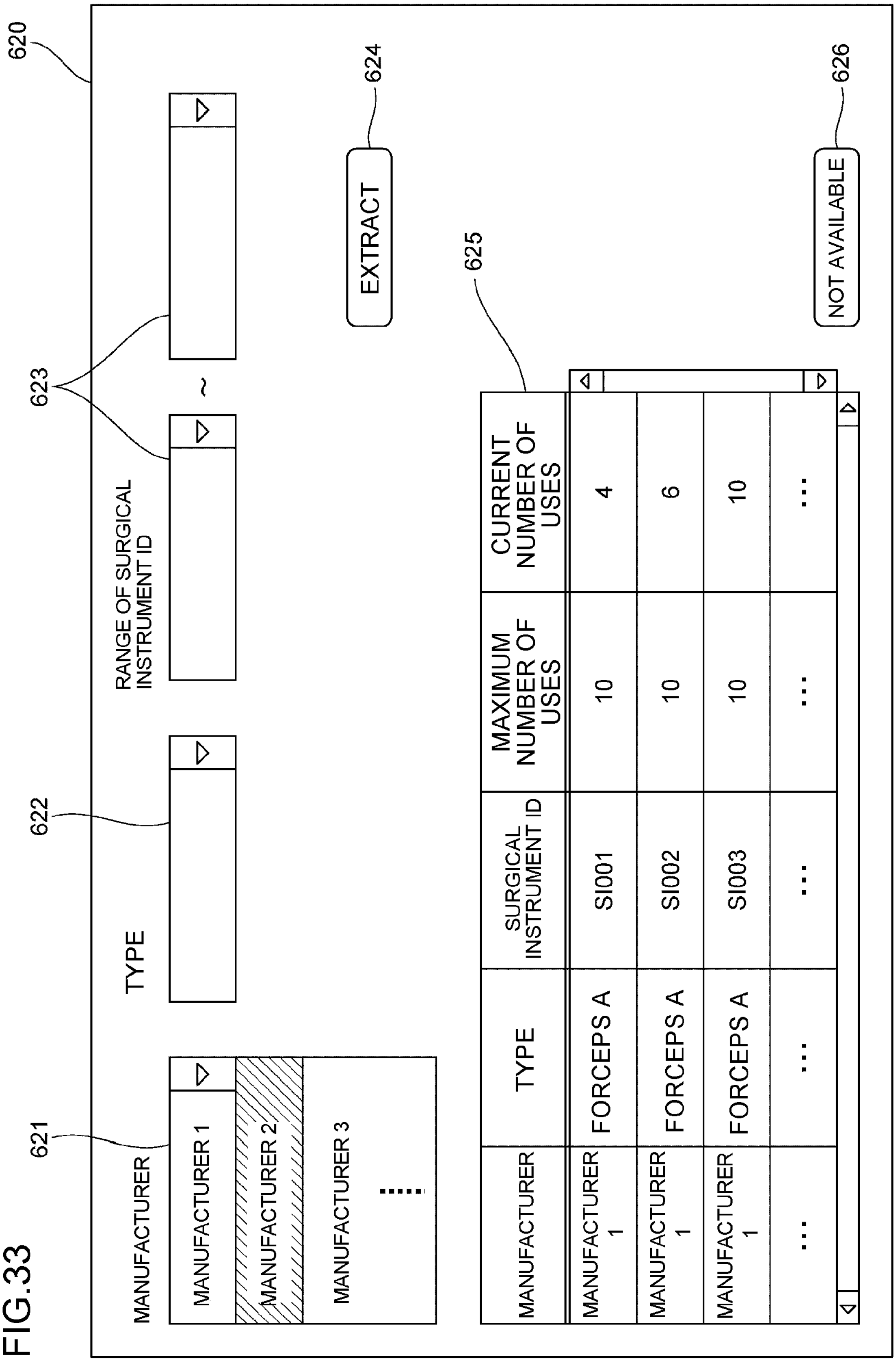
620
621
622
623
624
625
626
MANUFACTURER
MANUFACTURER 1
MANUFACTURER 2
MANUFACTURER 3
TYPE
RANGE OF SURGICAL INSTRUMENT ID
~
EXTRACT
NOT AVAILABLE
MANUFACTURER
TYPE
SURGICAL INSTRUMENT ID
MAXIMUM NUMBER OF USES
CURRENT NUMBER OF USES
MANUFACTURER 1
FORCEPS A
SI001
10
4
MANUFACTURER 1
FORCEPS A
SI002
10
6
MANUFACTURER 1
FORCEPS A
SI003
10
10
...

FIG.34

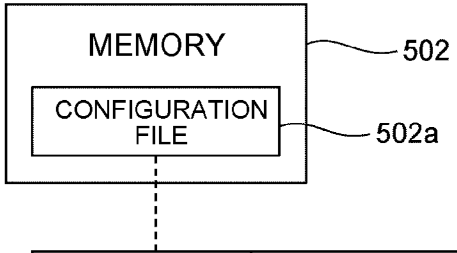


| SURGICAL INSTRUMENT ID | MANUFACTURER | LOT NUMBER | TYPE | MAXIMUM NUMBER OF USES | CURRENT NUMBER OF USES | JUDGMENT FLAG |
|---|---|---|---|---|---|---|
| SI001 | MANUFACTURER 1 | X1al38 | FORCEPS A | 10 | 4 | 0 |
| SI002 | MANUFACTURER 1 | Xksil73 | FORCEPS A | 10 | 6 | 1 |
| SI003 | MANUFACTURER 2 | KeUe11 | FORCEPS A | 10 | 10 | 1 |
| ... | ... | ... | ... | ... | ... | ... |

FIG.35

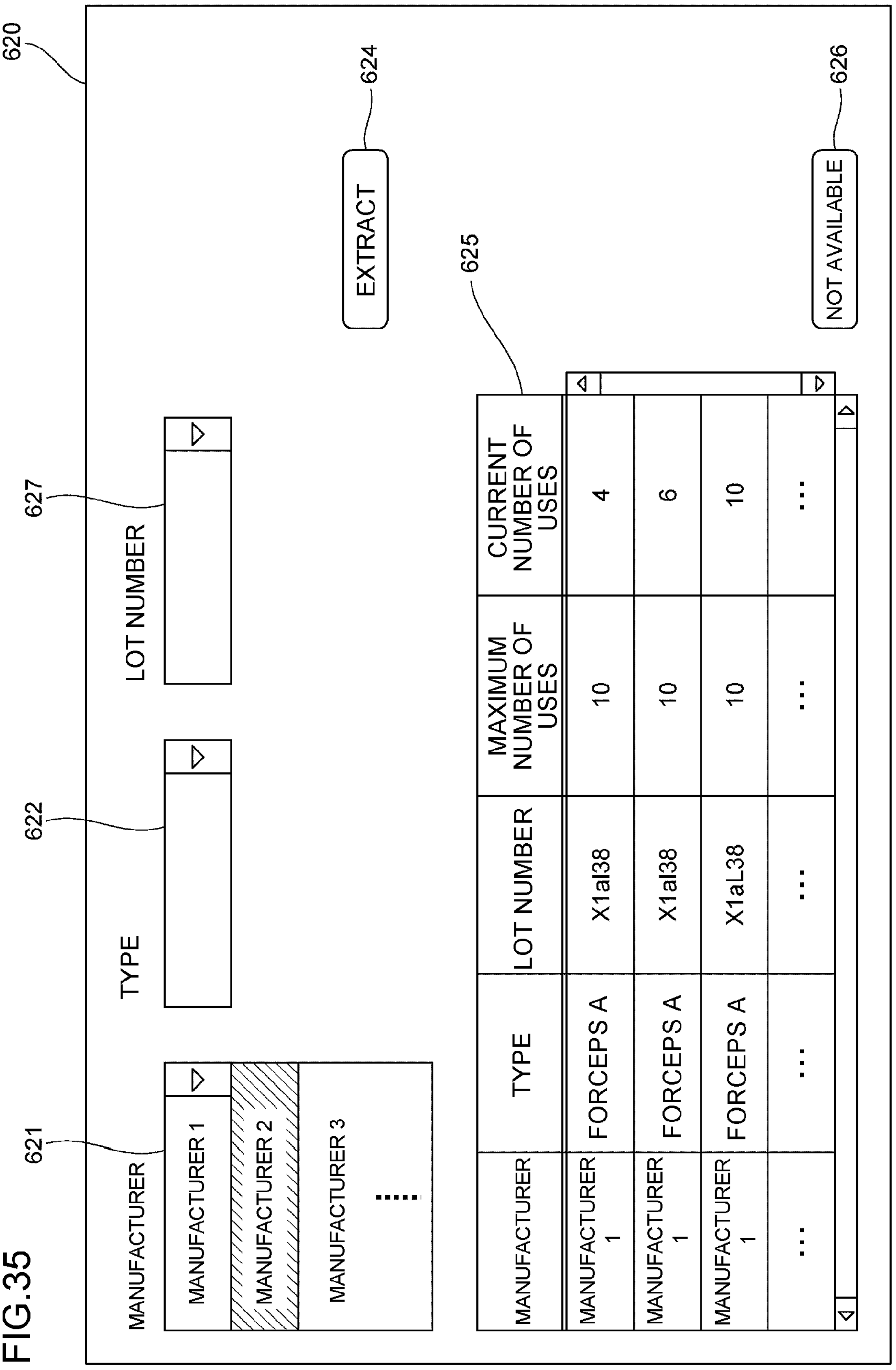
620
621
622
627
624
625
626
MANUFACTURER
MANUFACTURER 1
MANUFACTURER 2
MANUFACTURER 3
TYPE
LOT NUMBER
EXTRACT
NOT AVAILABLE
MANUFACTURER
TYPE
LOT NUMBER
MAXIMUM NUMBER OF USES
CURRENT NUMBER OF USES
MANUFACTURER 1
FORCEPS A
X1al38
10
4
MANUFACTURER 1
FORCEPS A
X1al38
10
6
MANUFACTURER 1
FORCEPS A
X1aL38
10
10

FIG.36

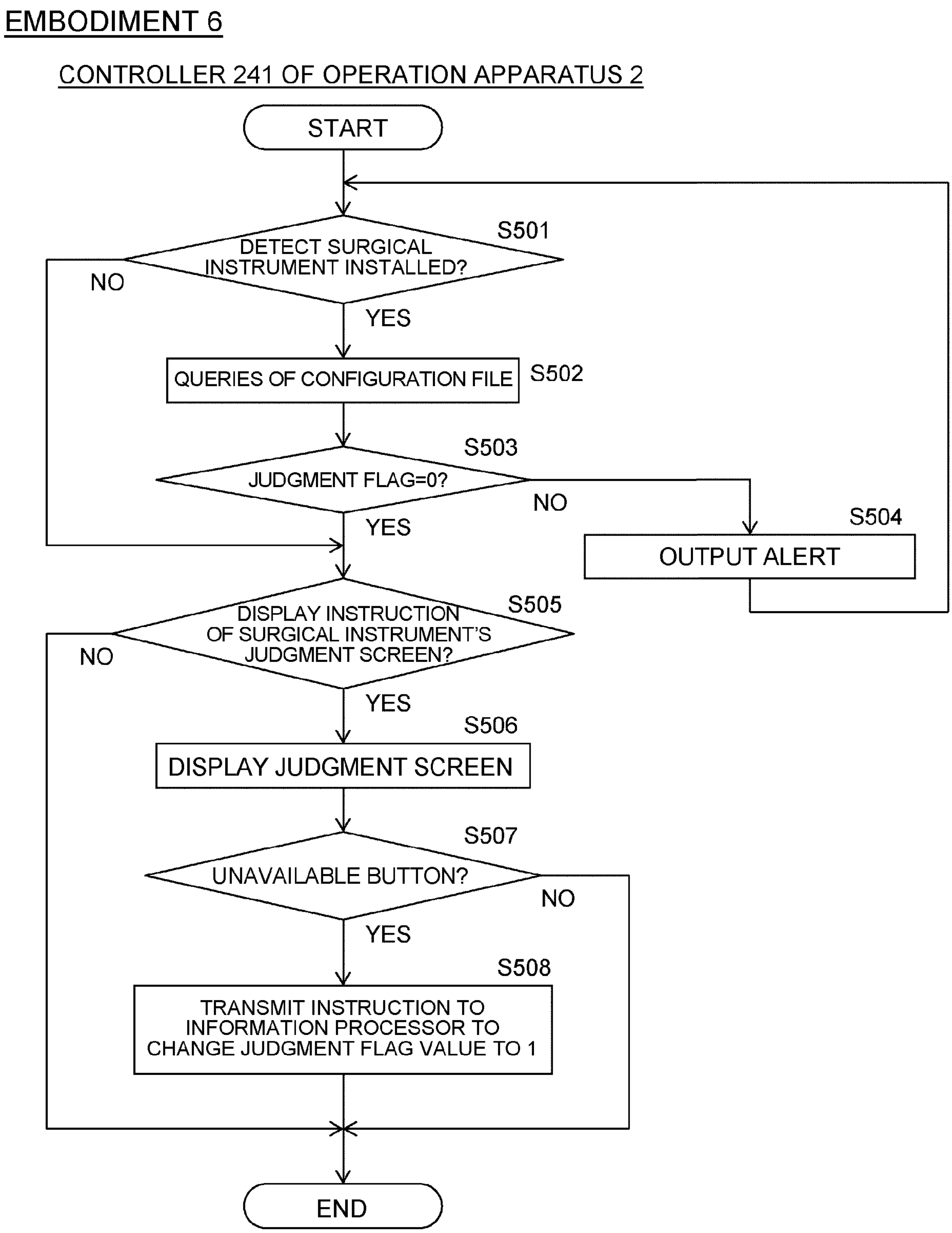
EMBODIMENT 6
CONTROLLER 241 OF OPERATION APPARATUS 2
START
S501
DETECT SURGICAL INSTRUMENT INSTALLED?
NO
YES
S502
QUERIES OF CONFIGURATION FILE
S503
JUDGMENT FLAG=0?
NO
YES
S504
OUTPUT ALERT
S505
DISPLAY INSTRUCTION OF SURGICAL INSTRUMENT'S JUDGMENT SCREEN?
NO
YES
S506
DISPLAY JUDGMENT SCREEN
S507
UNAVAILABLE BUTTON?
NO
YES
S508
TRANSMIT INSTRUCTION TO INFORMATION PROCESSOR TO CHANGE JUDGMENT FLAG VALUE TO 1
END

FIG.37

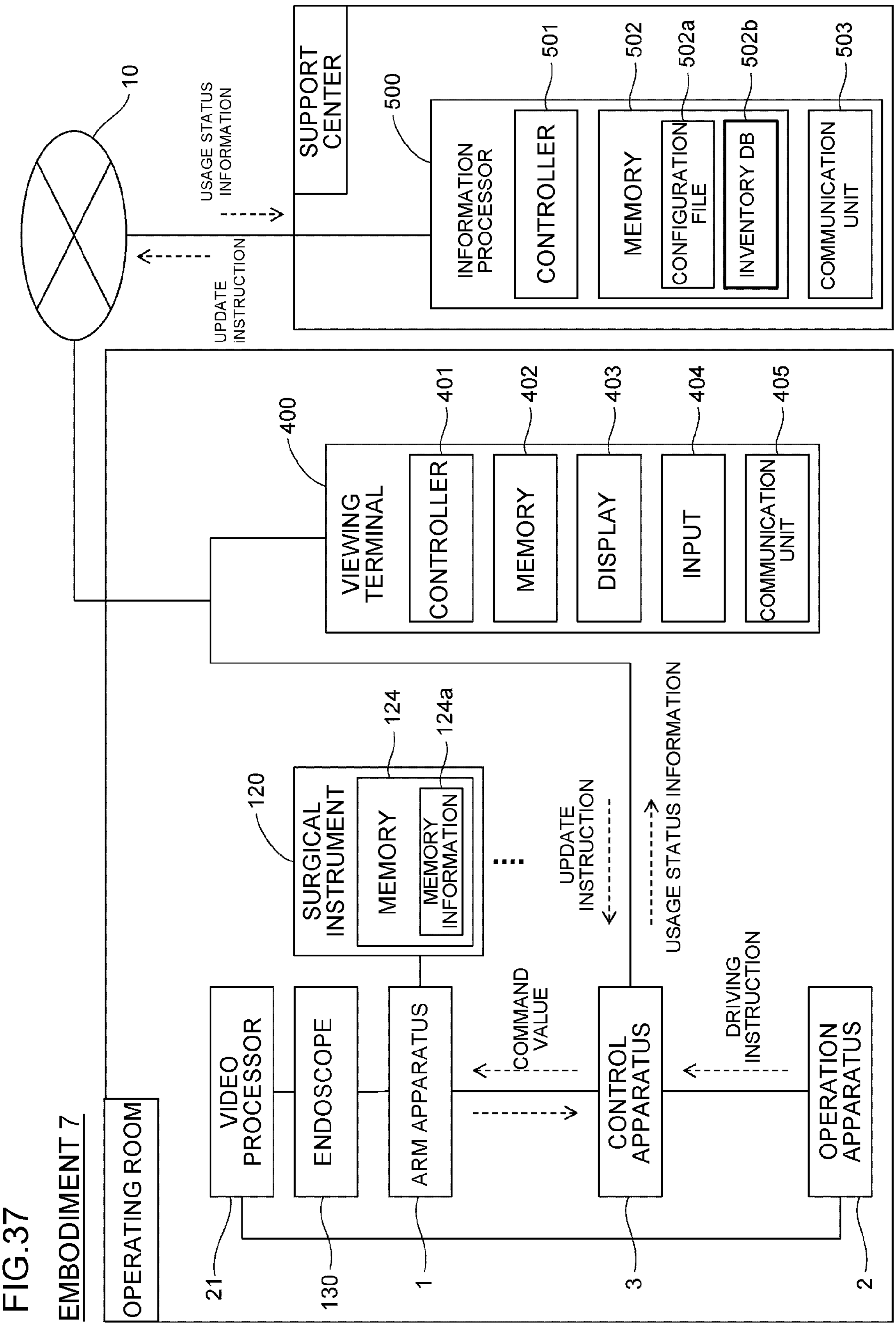
EMBODIMENT 7
OPERATING ROOM
VIDEO PROCESSOR
ENDOSCOPE
ARM APPARATUS
CONTROL APPARATUS
OPERATION APPARATUS
COMMAND VALUE
DRIVING INSTRUCTION
SURGICAL INSTRUMENT
MEMORY
MEMORY INFORMATION
UPDATE INSTRUCTION
USAGE STATUS INFORMATION
VIEWING TERMINAL
CONTROLLER
MEMORY
DISPLAY
INPUT
COMMUNICATION UNIT
SUPPORT CENTER
INFORMATION PROCESSOR
CONTROLLER
MEMORY
CONFIGURATION FILE
INVENTORY DB
COMMUNICATION UNIT
USAGE STATUS INFORMATION
UPDATE INSTRUCTION
21
130
1
3
2
120
124
124a
400
401
402
403
404
405
10
500
501
502
502a
502b
503

FIG.38

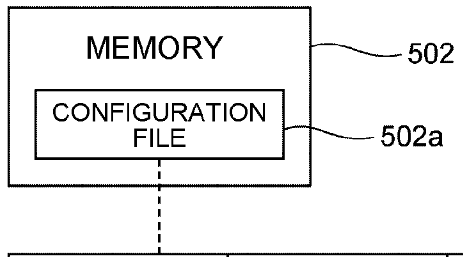


| SURGICAL INSTRUMENT ID | TYPE | MAXIMUM NUMBER OF USES | CURRENT NUMBER OF USES | START DATE AND TIME OF USES | JUDGMENT FLAG | COMMENTS |
|---|---|---|---|---|---|---|
| SI001 | FORCEPS A | 10 | 4 | 2022/07/29 10:00 | 0 | ... |
| SI002 | FORCEPS A | 10 | 6 | 2022/07/27 11:00 | 1 | ... |
| SI003 | FORCEPS A | 10 | 10 | 2022/07/25 10:00 | 1 | ... |
| SI021 | FORCEPS B | 10 | 3 | 2022/07/15 11:00 | 0 | ... |
| SI022 | FORCEPS C | 10 | 10 | 2022/07/10 13:00 | 1 | ... |
| ... | ... | ... | ... | ... | ... | ... |

FIG.39

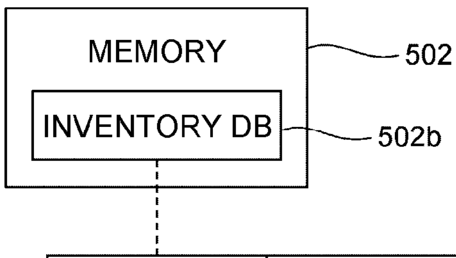


| TYPE | TOTAL NUMBER | NO. OF AVAILABILITY |
|---|---|---|
| FORCEPS A | 3 | 1 |
| FORCEPS B | 4 | 3 |
| FORCEPS C | 2 | 2 |
| ... | ... | ... |

FIG.40

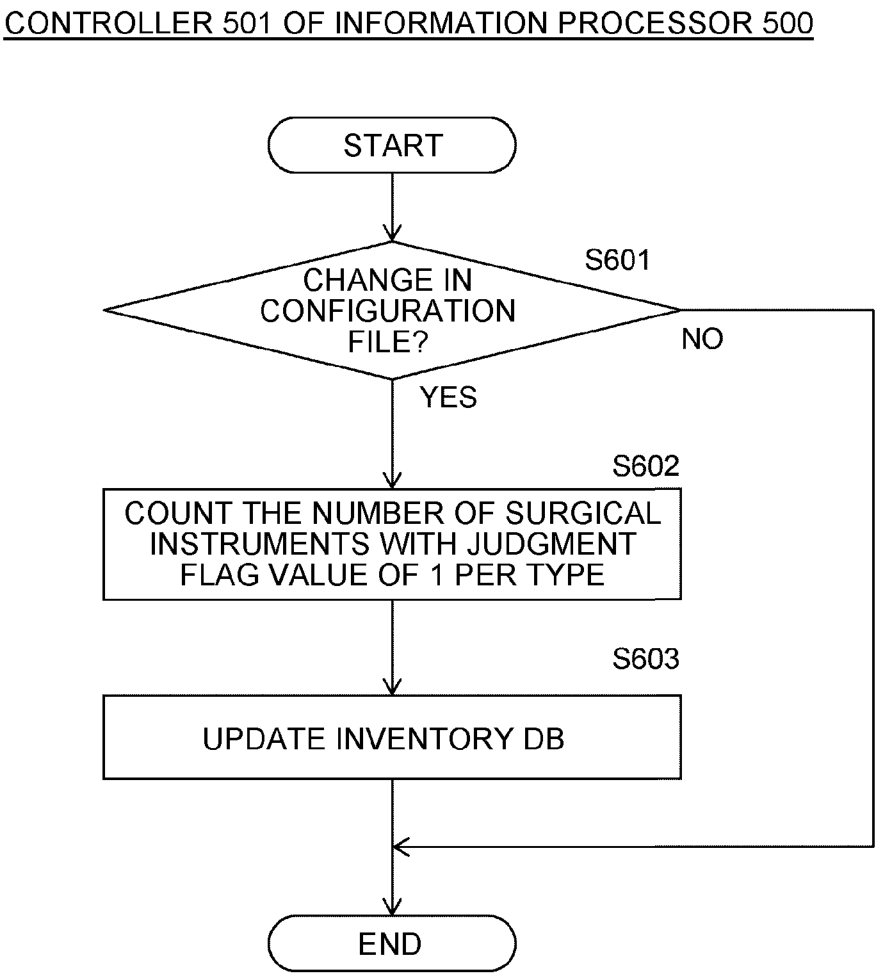
CONTROLLER 501 OF INFORMATION PROCESSOR 500
START
S601
CHANGE IN CONFIGURATION FILE?
NO
YES
S602
COUNT THE NUMBER OF SURGICAL INSTRUMENTS WITH JUDGMENT FLAG VALUE OF 1 PER TYPE
S603
UPDATE INVENTORY DB
END

FIG.41

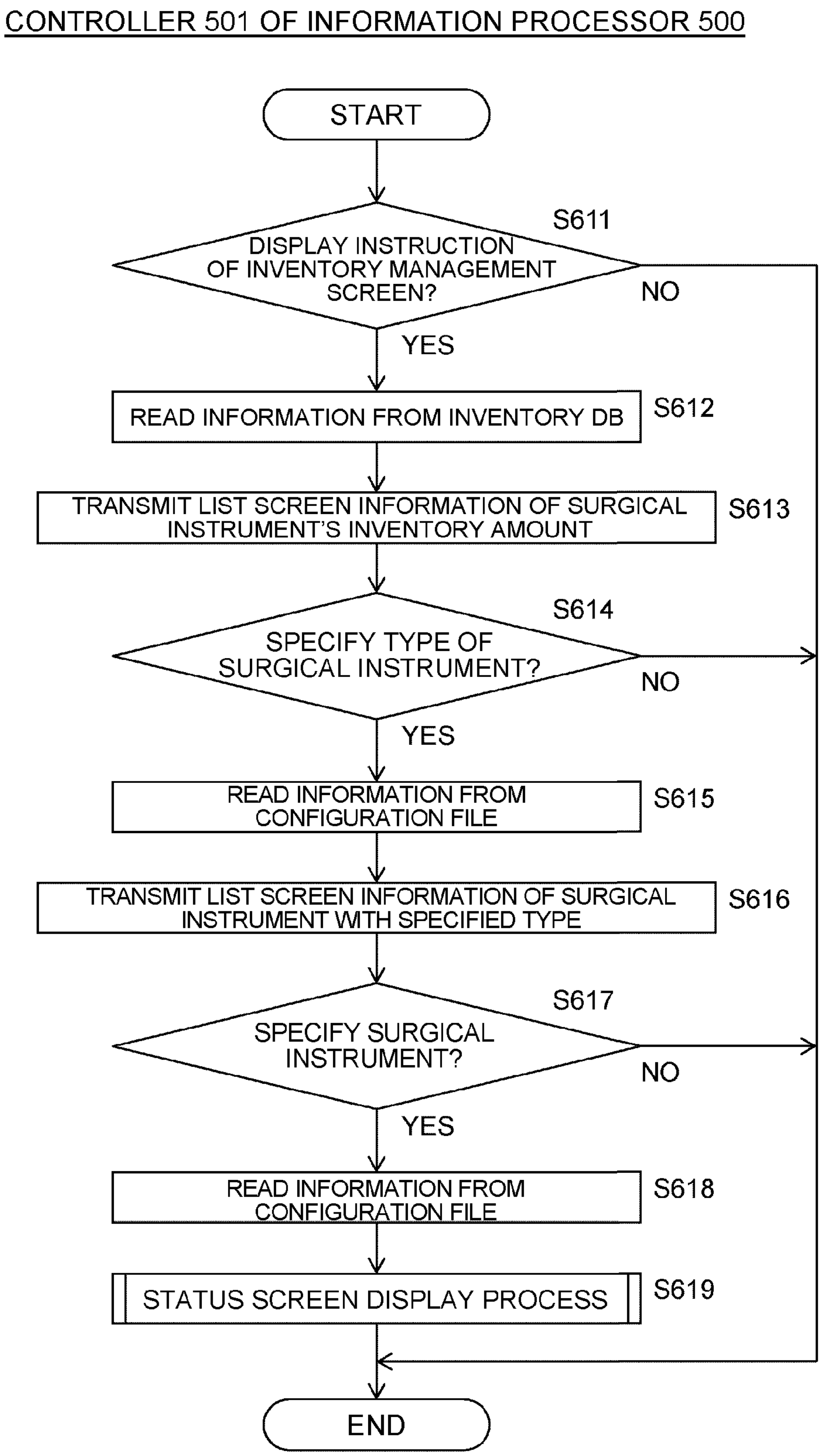
CONTROLLER 501 OF INFORMATION PROCESSOR 500
START
S611
DISPLAY INSTRUCTION OF INVENTORY MANAGEMENT SCREEN?
NO
YES
READ INFORMATION FROM INVENTORY DB
S612
TRANSMIT LIST SCREEN INFORMATION OF SURGICAL INSTRUMENT'S INVENTORY AMOUNT
S613
S614
SPECIFY TYPE OF SURGICAL INSTRUMENT?
NO
YES
READ INFORMATION FROM CONFIGURATION FILE
S615
TRANSMIT LIST SCREEN INFORMATION OF SURGICAL INSTRUMENT WITH SPECIFIED TYPE
S616
S617
SPECIFY SURGICAL INSTRUMENT?
NO
YES
READ INFORMATION FROM CONFIGURATION FILE
S618
STATUS SCREEN DISPLAY PROCESS
S619
END

FIG.42

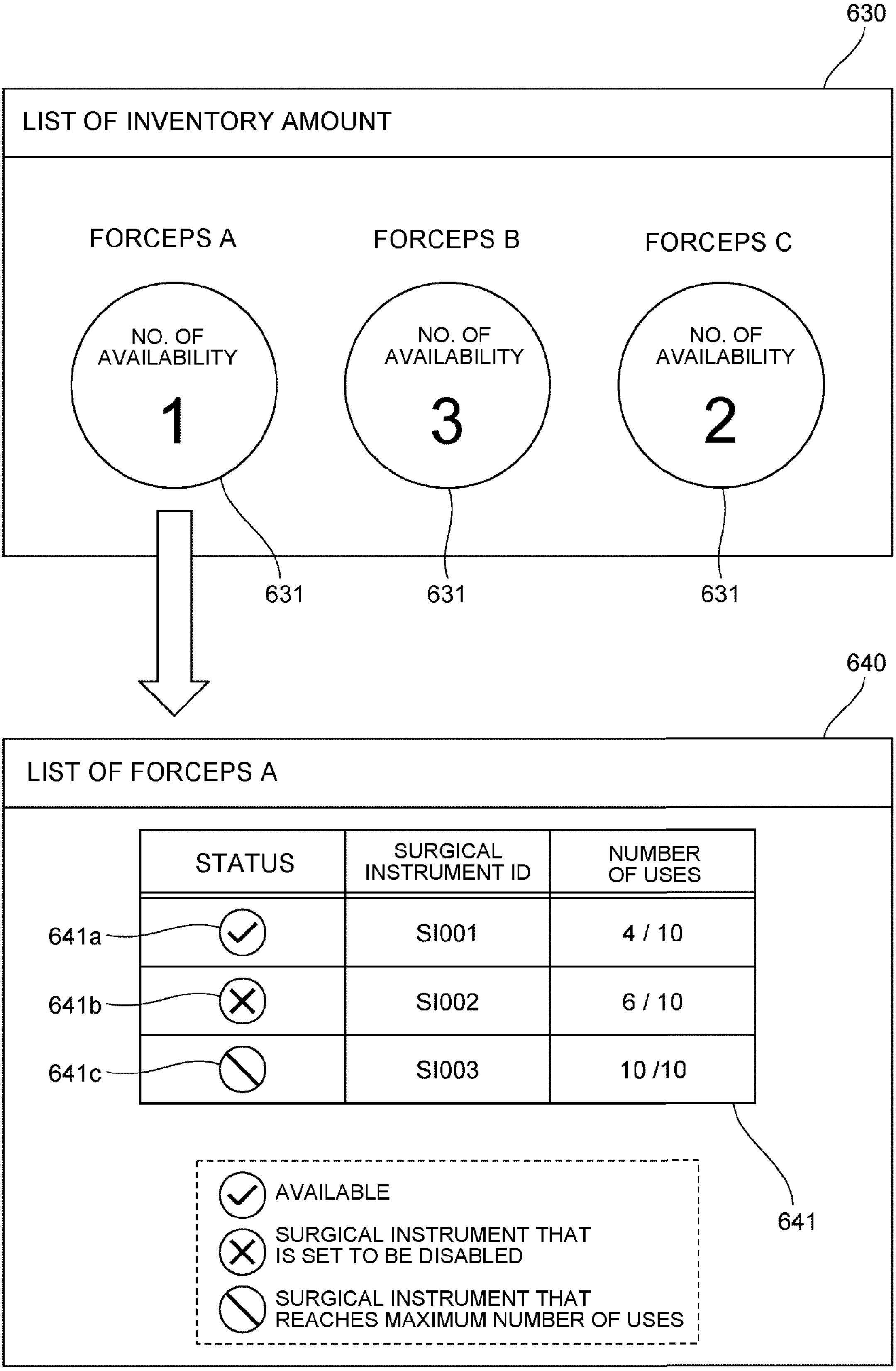
630
LIST OF INVENTORY AMOUNT
FORCEPS A
NO. OF AVAILABILITY
1
FORCEPS B
NO. OF AVAILABILITY
3
FORCEPS C
NO. OF AVAILABILITY
2
631
631
631
640
LIST OF FORCEPS A
STATUS
SURGICAL INSTRUMENT ID
NUMBER OF USES
641a
SI001
4 / 10
641b
SI002
6 / 10
641c
SI003
10 /10
641
AVAILABLE
SURGICAL INSTRUMENT THAT IS SET TO BE DISABLED
SURGICAL INSTRUMENT THAT REACHES MAXIMUM NUMBER OF USES

FIG.43

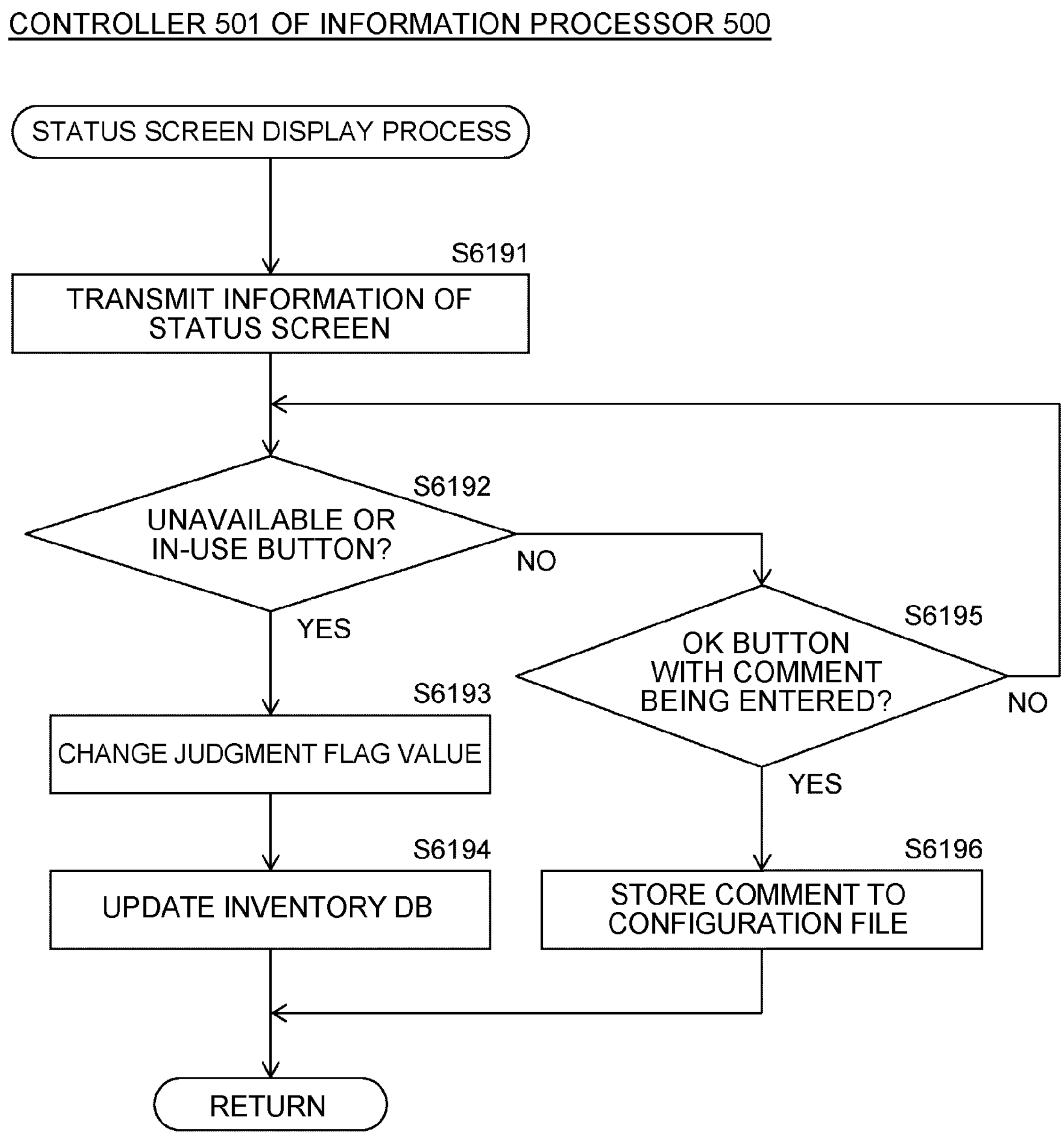
CONTROLLER 501 OF INFORMATION PROCESSOR 500
STATUS SCREEN DISPLAY PROCESS
S6191
TRANSMIT INFORMATION OF STATUS SCREEN
S6192
UNAVAILABLE OR IN-USE BUTTON?
NO
YES
S6193
CHANGE JUDGMENT FLAG VALUE
S6194
UPDATE INVENTORY DB
S6195
OK BUTTON WITH COMMENT BEING ENTERED?
NO
YES
S6196
STORE COMMENT TO CONFIGURATION FILE
RETURN

FIG.44

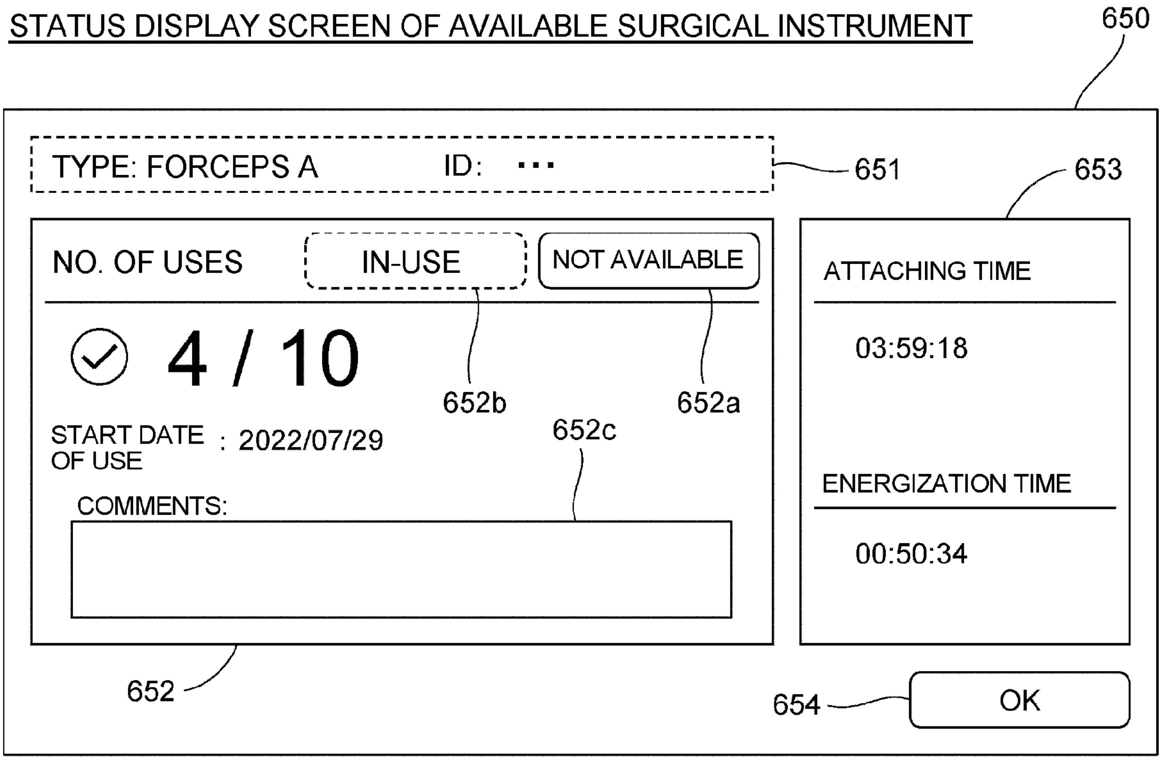
STATUS DISPLAY SCREEN OF AVAILABLE SURGICAL INSTRUMENT
650
TYPE: FORCEPS A ID: ···
651
653
NO. OF USES
IN-USE
NOT AVAILABLE
ATTACHING TIME
03:59:18
4 / 10
652b
652a
652c
START DATE OF USE : 2022/07/29
ENERGIZATION TIME
00:50:34
COMMENTS:
652
654
OK

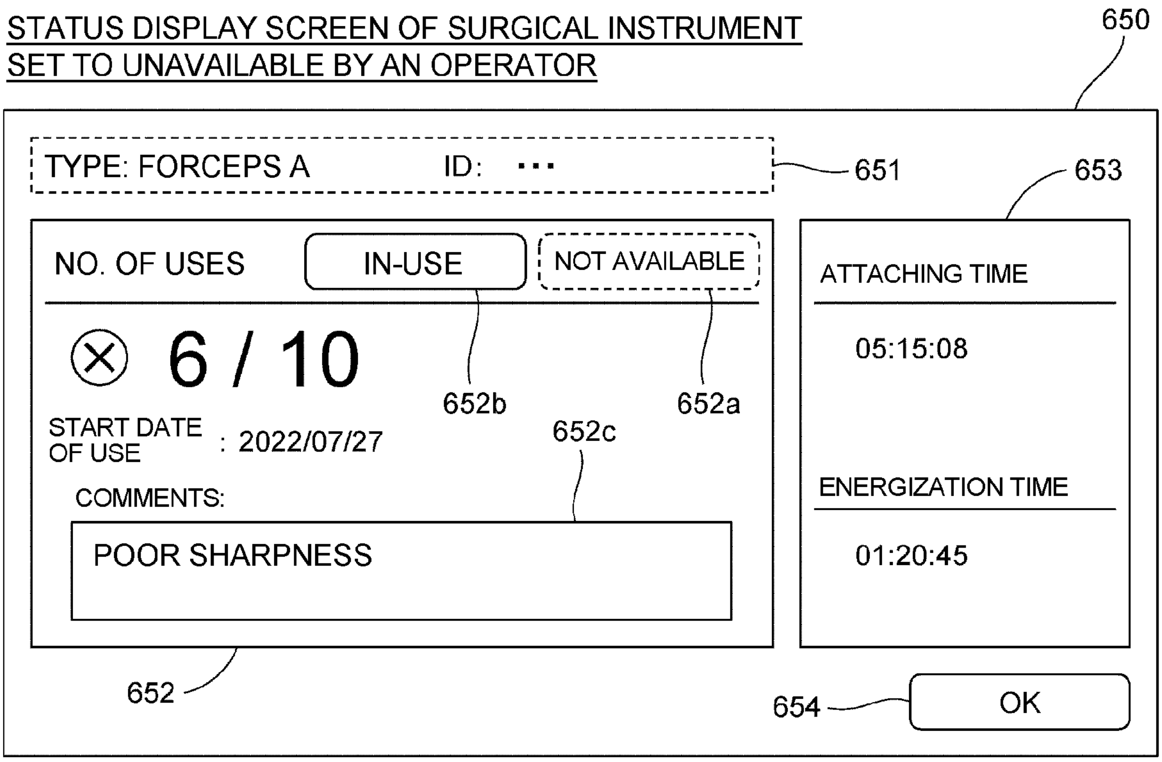
STATUS DISPLAY SCREEN OF SURGICAL INSTRUMENT SET TO UNAVAILABLE BY AN OPERATOR
650
TYPE: FORCEPS A ID: ···
651
653
NO. OF USES
IN-USE
NOT AVAILABLE
ATTACHING TIME
05:15:08
6 / 10
652b
652a
652c
START DATE OF USE : 2022/07/27
ENERGIZATION TIME
01:20:45
COMMENTS:
POOR SHARPNESS
652
654
OK

FIG.45

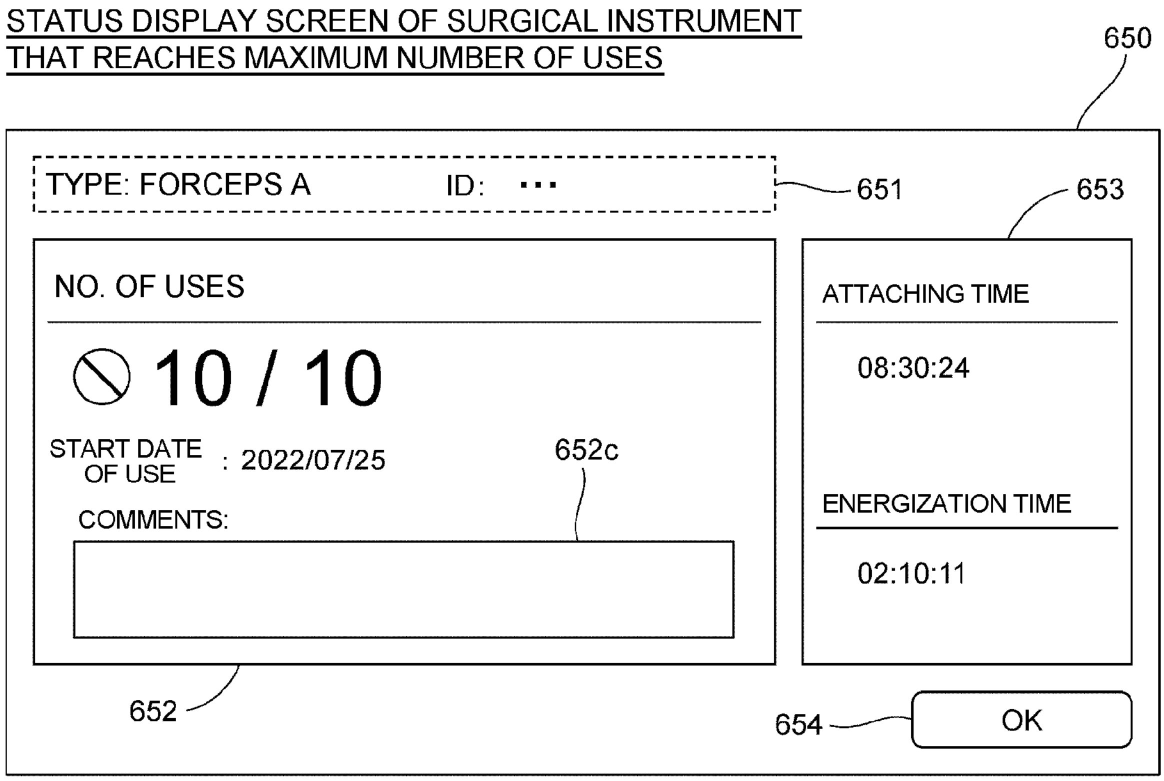
STATUS DISPLAY SCREEN OF SURGICAL INSTRUMENT
THAT REACHES MAXIMUM NUMBER OF USES
650
TYPE: FORCEPS A
ID: ···
651
653
NO. OF USES
10 / 10
START DATE OF USE : 2022/07/25
652c
COMMENTS:
ATTACHING TIME
08:30:24
ENERGIZATION TIME
02:10:11
652
654
OK

FIG.46

VARIATION OF EMBODIMENT 7

VARIATION OF COMMENT AREA

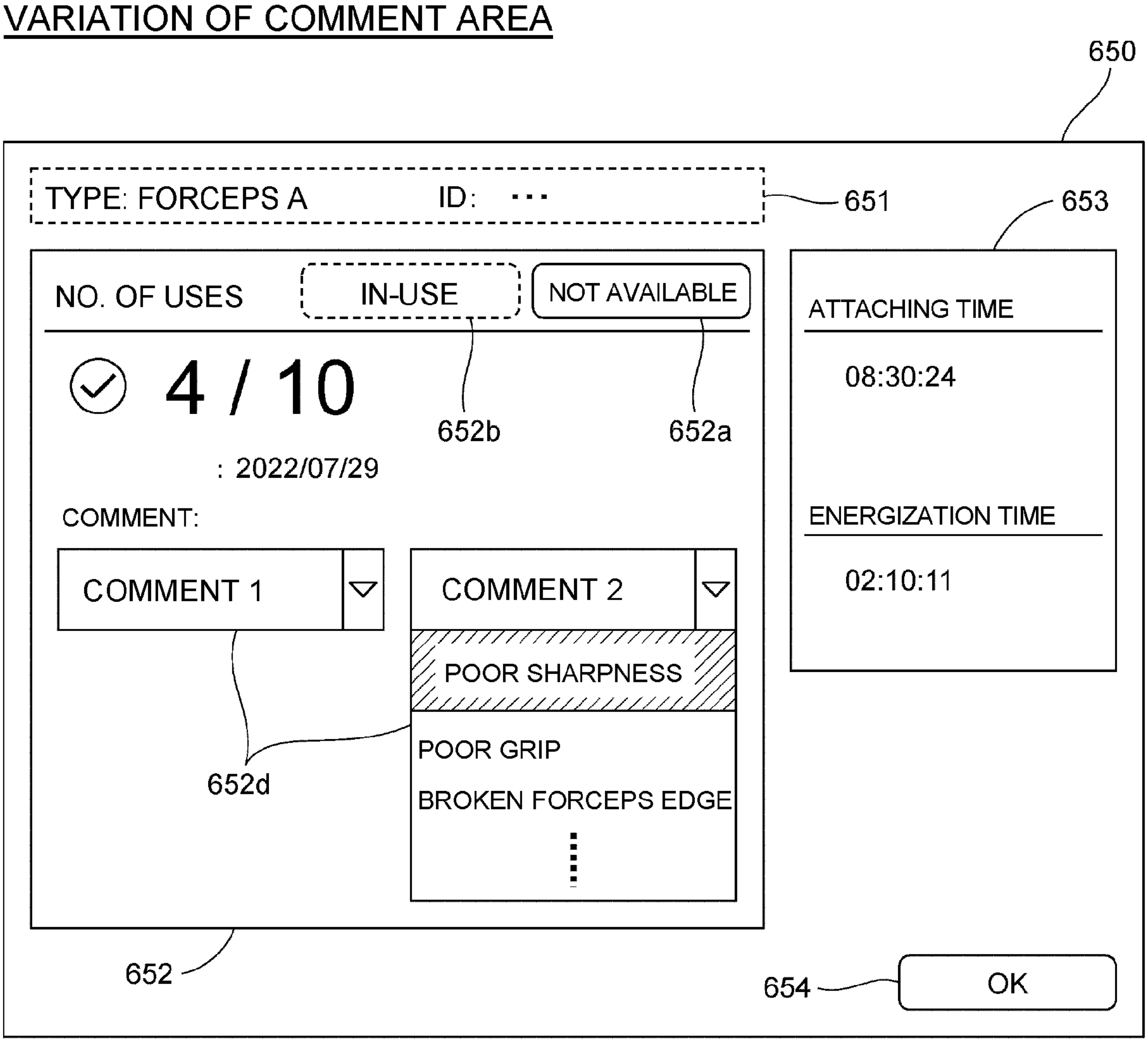

FIG.47

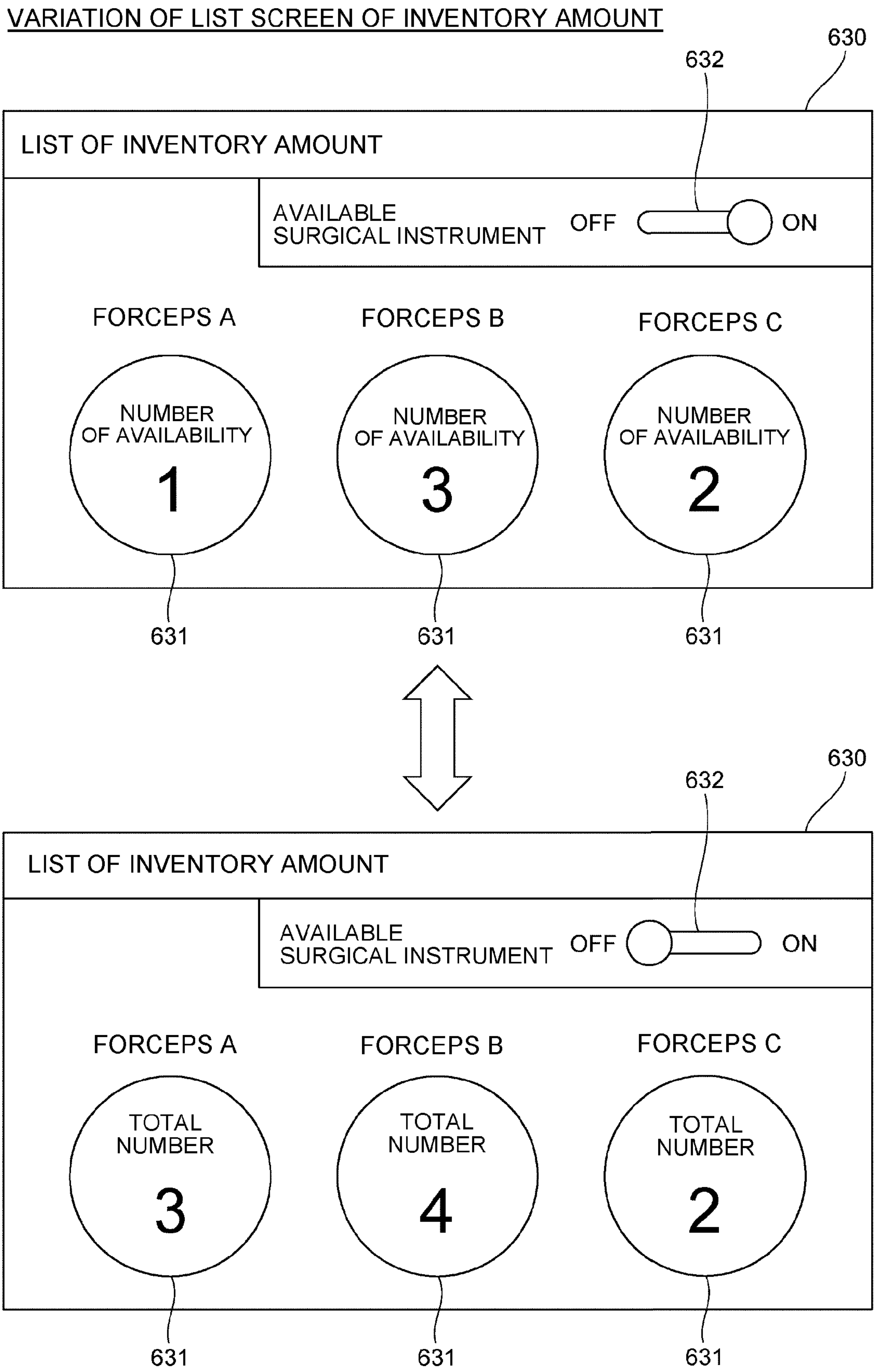
VARIATION OF LIST SCREEN OF INVENTORY AMOUNT
630
632
LIST OF INVENTORY AMOUNT
AVAILABLE SURGICAL INSTRUMENT
OFF
ON
FORCEPS A
FORCEPS B
FORCEPS C
NUMBER OF AVAILABILITY
1
NUMBER OF AVAILABILITY
3
NUMBER OF AVAILABILITY
2
631
631
631
630
632
LIST OF INVENTORY AMOUNT
AVAILABLE SURGICAL INSTRUMENT
OFF
ON
FORCEPS A
FORCEPS B
FORCEPS C
TOTAL NUMBER
3
TOTAL NUMBER
4
TOTAL NUMBER
2
631
631
631

FIG.48

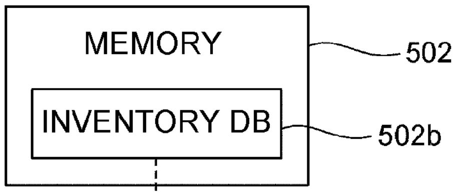


| TYPE | TOTAL NO. | NO. OF AVAILABILITY | NO. OF REMAINING COUNTS OF 1 | NO. OF REMAINING COUNTS OF 2 | NO. OF REMAINING COUNTS OF 3 OR MORE | NO. OF MAXIMUM USAGE LIMIT REACHED | NO. OF UNAVAILABLE BUTTON OPERATED |
|---|---|---|---|---|---|---|---|
| FORCEPS A | ... | ... | ... | ... | ... | ... | ... |
| FORCEPS B | ... | ... | ... | ... | ... | ... | ... |
| FORCEPS C | ... | ... | ... | ... | ... | ... | ... |
| ... | ... | ... | ... | ... | ... | ... | ... |

FIG.49

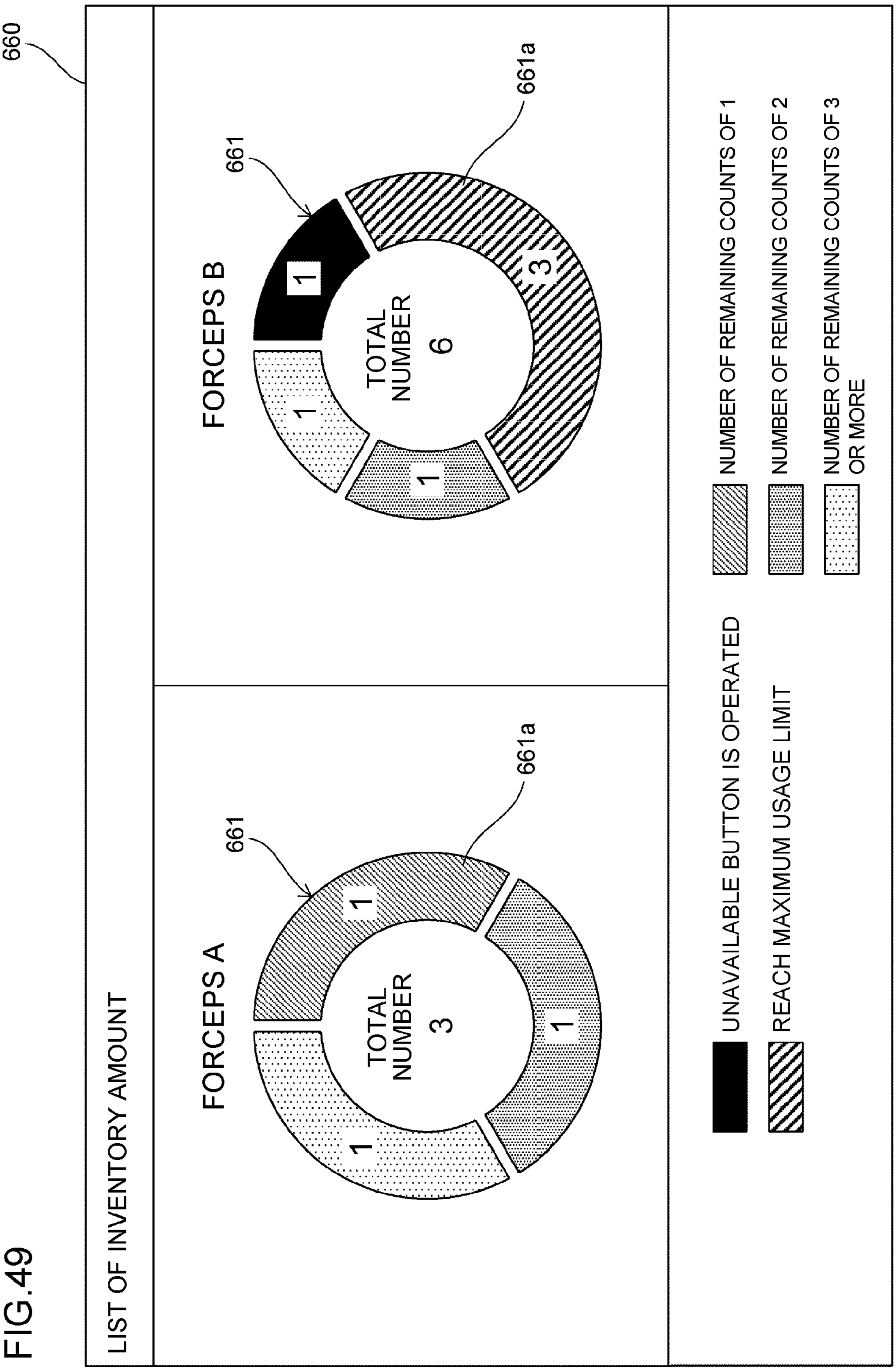
660
LIST OF INVENTORY AMOUNT
FORCEPS A
661
661a
TOTAL NUMBER 3
1
1
1
FORCEPS B
661
661a
TOTAL NUMBER 6
1
1
1
3
UNAVAILABLE BUTTON IS OPERATED
REACH MAXIMUM USAGE LIMIT
NUMBER OF REMAINING COUNTS OF 1
NUMBER OF REMAINING COUNTS OF 2
NUMBER OF REMAINING COUNTS OF 3 OR MORE

FIG.50

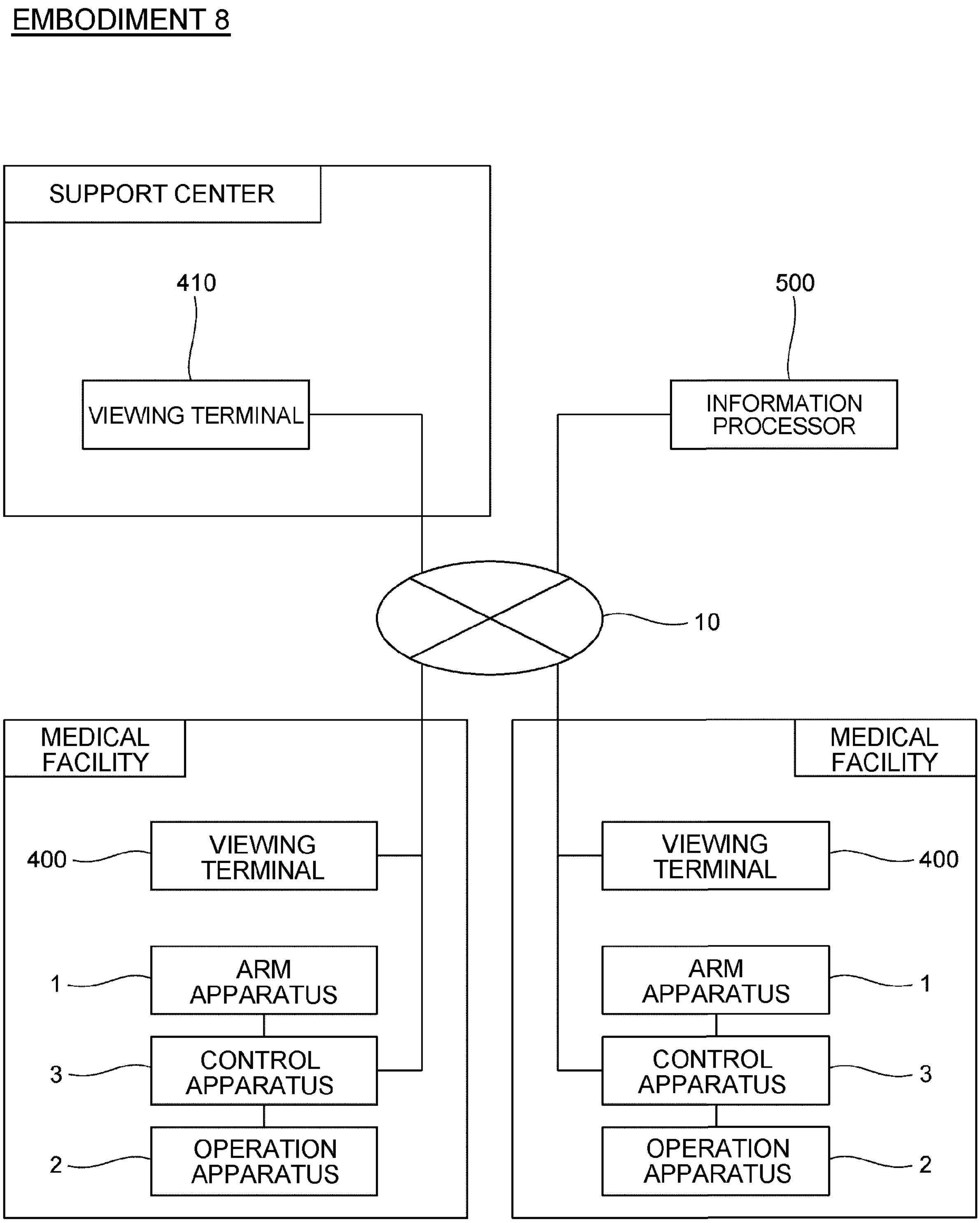
EMBODIMENT 8
SUPPORT CENTER
410
VIEWING TERMINAL
500
INFORMATION PROCESSOR
10
MEDICAL FACILITY
400
VIEWING TERMINAL
1
ARM APPARATUS
3
CONTROL APPARATUS
2
OPERATION APPARATUS
MEDICAL FACILITY
VIEWING TERMINAL
400
ARM APPARATUS
1
CONTROL APPARATUS
3
OPERATION APPARATUS
2

FIG.51

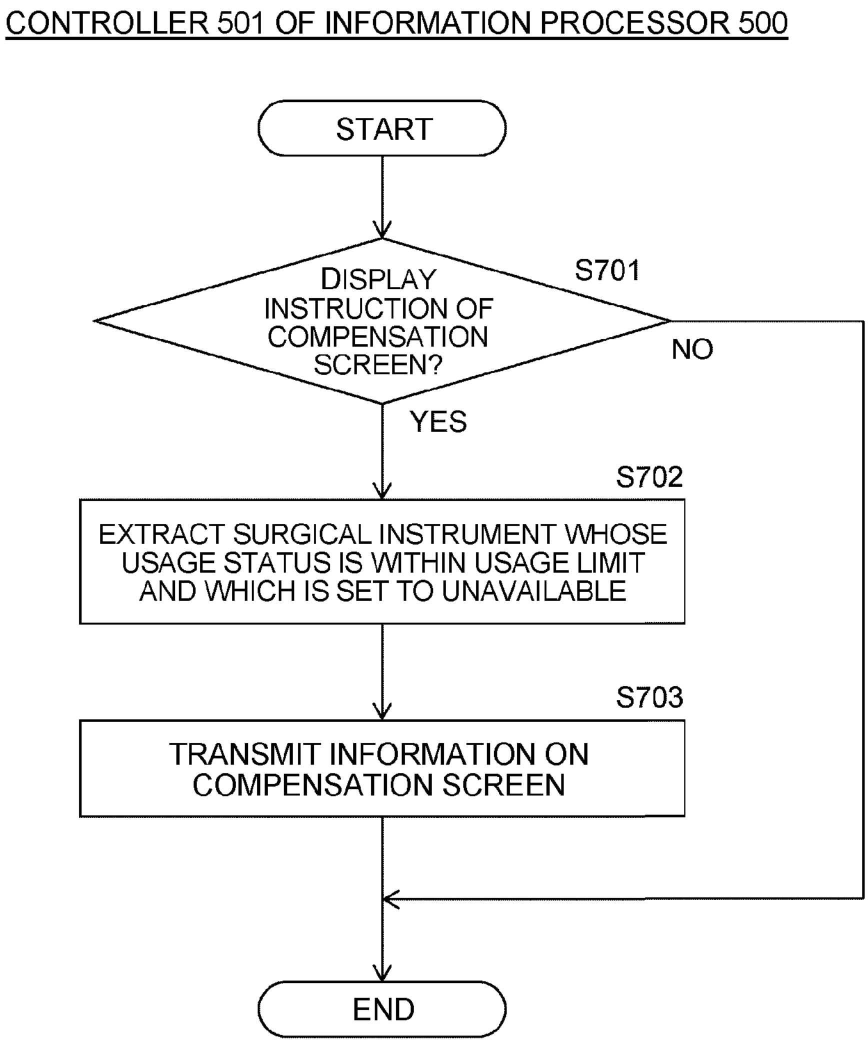
CONTROLLER 501 OF INFORMATION PROCESSOR 500
START
DISPLAY INSTRUCTION OF COMPENSATION SCREEN?
S701
NO
YES
S702
EXTRACT SURGICAL INSTRUMENT WHOSE USAGE STATUS IS WITHIN USAGE LIMIT AND WHICH IS SET TO UNAVAILABLE
S703
TRANSMIT INFORMATION ON COMPENSATION SCREEN
END

FIG.52

670

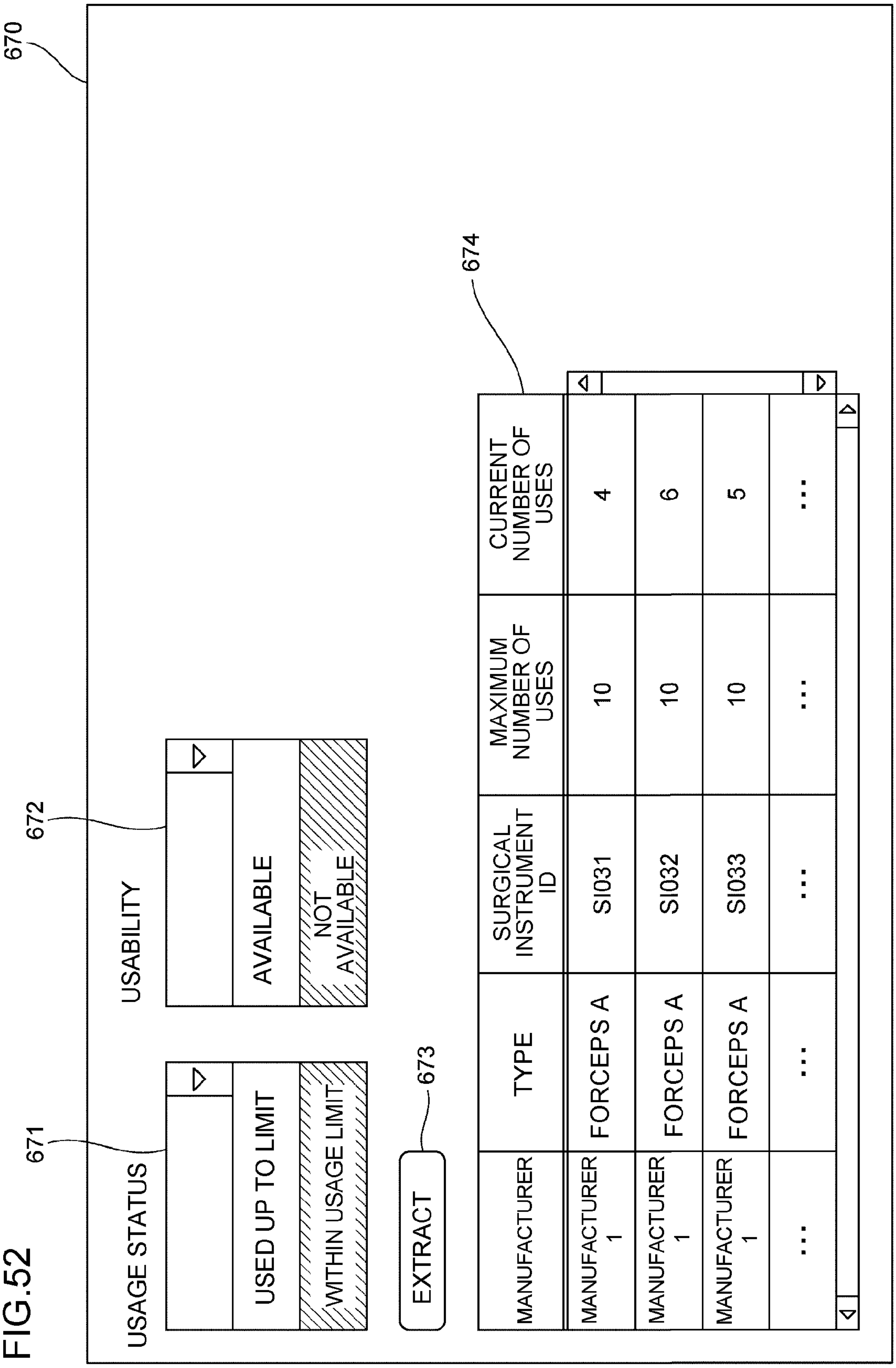
USAGE STATUS
USED UP TO LIMIT
WITHIN USAGE LIMIT
USABILITY
AVAILABLE
NOT AVAILABLE
EXTRACT
671
672
673
674
MANUFACTURER
TYPE
SURGICAL INSTRUMENT ID
MAXIMUM NUMBER OF USES
CURRENT NUMBER OF USES
MANUFACTURER 1
FORCEPS A
SI031
10
4
MANUFACTURER 1
FORCEPS A
SI032
10
6
MANUFACTURER 1
FORCEPS A
SI033
10
5
...

FIG.53

VARIATION 1 OF EMBODIMENT 8

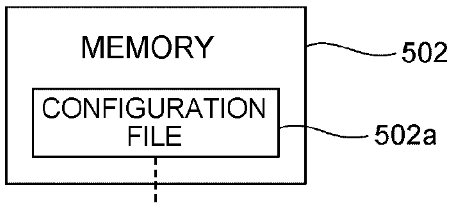


| SURGICAL INSTRUMENT ID | CUSTOMER ID | MANUFACTURER | TYPE | MAXIMUM NUMBER OF USES | CURRENT NUMBER OF USES | JUDGMENT FLAG | COMPENSATION FLAG |
|---|---|---|---|---|---|---|---|
| SI001 | C001 | MANUFACTURER 1 | FORCEPS A | 10 | 4 | 0 | 0 |
| SI002 | C002 | MANUFACTURER 1 | FORCEPS A | 10 | 6 | 1 | 1 |
| SI003 | C002 | MANUFACTURER 1 | FORCEPS A | 10 | 10 | 1 | 0 |
| ... | ... | ... | ... | ... | ... | ... | ... |

FIG.54

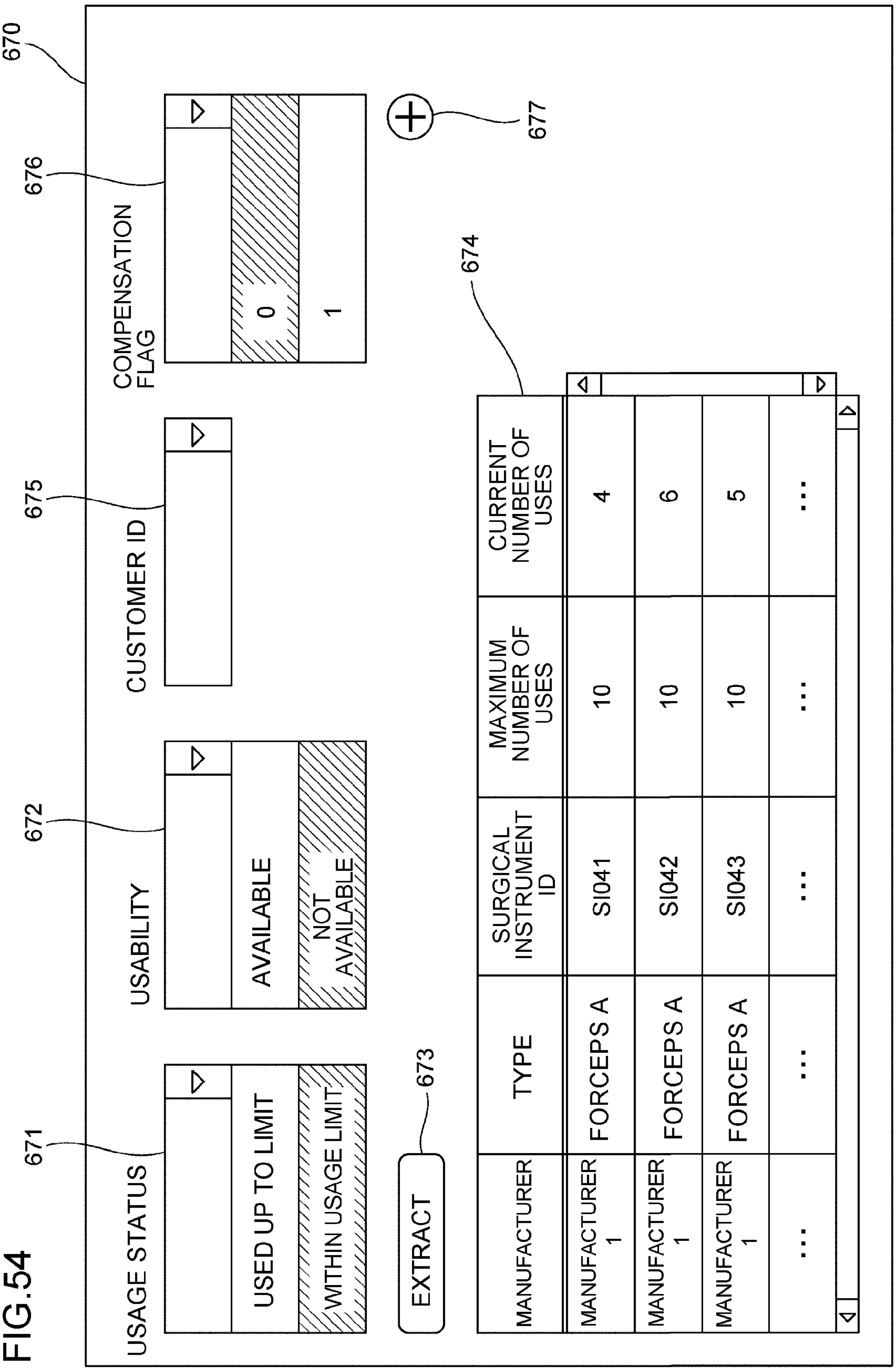
670
671
672
673
674
675
676
677
USAGE STATUS
USED UP TO LIMIT
WITHIN USAGE LIMIT
USABILITY
AVAILABLE
NOT AVAILABLE
CUSTOMER ID
COMPENSATION FLAG
0
1
EXTRACT
MANUFACTURER
TYPE
SURGICAL INSTRUMENT ID
MAXIMUM NUMBER OF USES
CURRENT NUMBER OF USES
MANUFACTURER 1
FORCEPS A
SI041
10
4
MANUFACTURER 1
FORCEPS A
SI042
10
6
MANUFACTURER 1
FORCEPS A
SI043
10
5
...

FIG.55

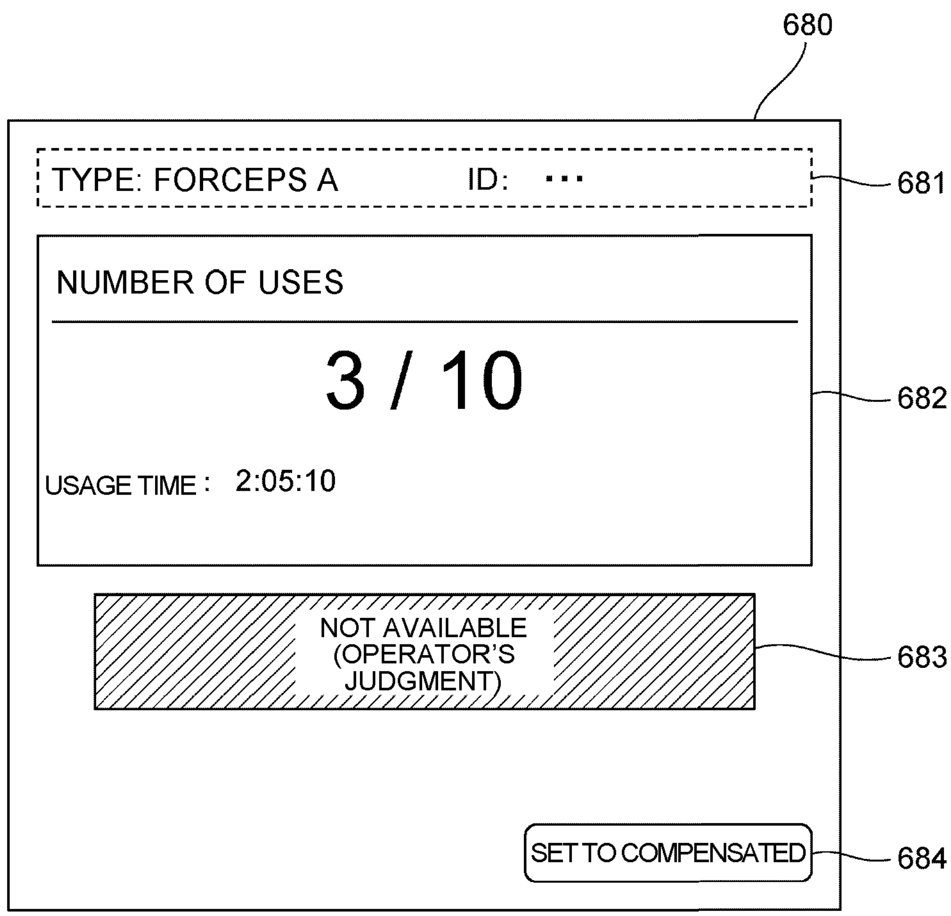
680
TYPE: FORCEPS A
ID: ···
681
NUMBER OF USES
3 / 10
USAGE TIME : 2:05:10
682
NOT AVAILABLE
(OPERATOR'S
JUDGMENT)
683
SET TO COMPENSATED
684

FIG.56

VARIATION 2 OF EMBODIMENT 8

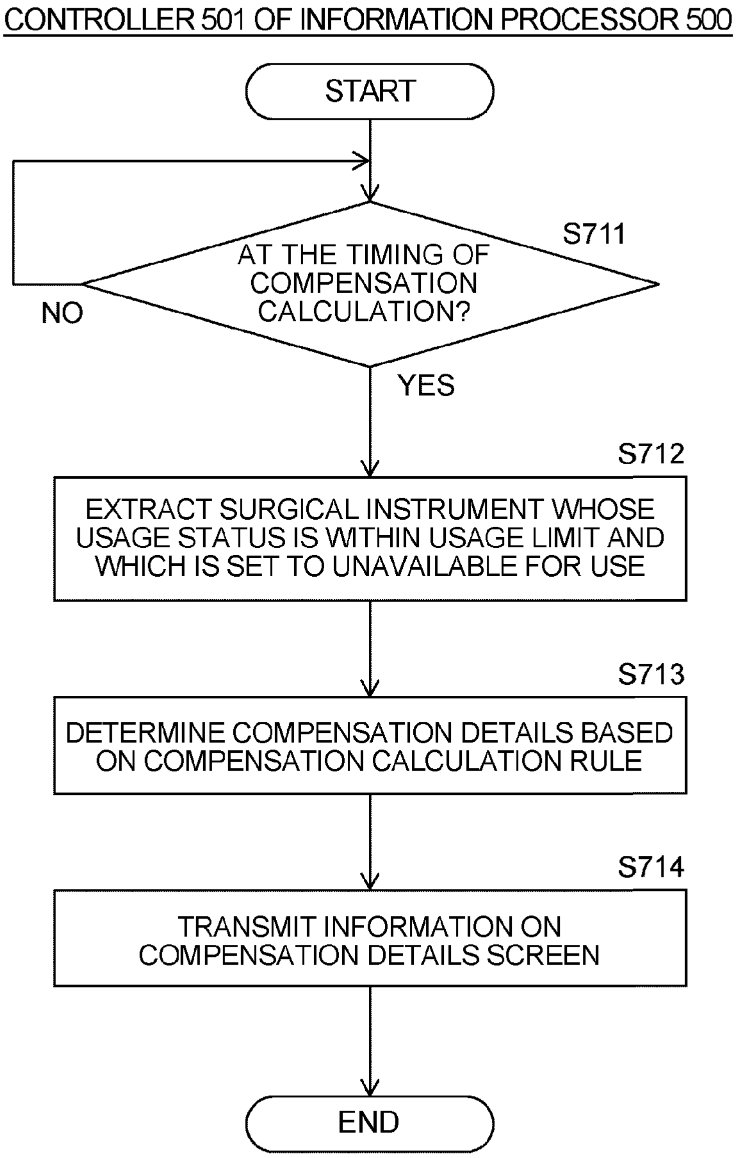

FIG.57

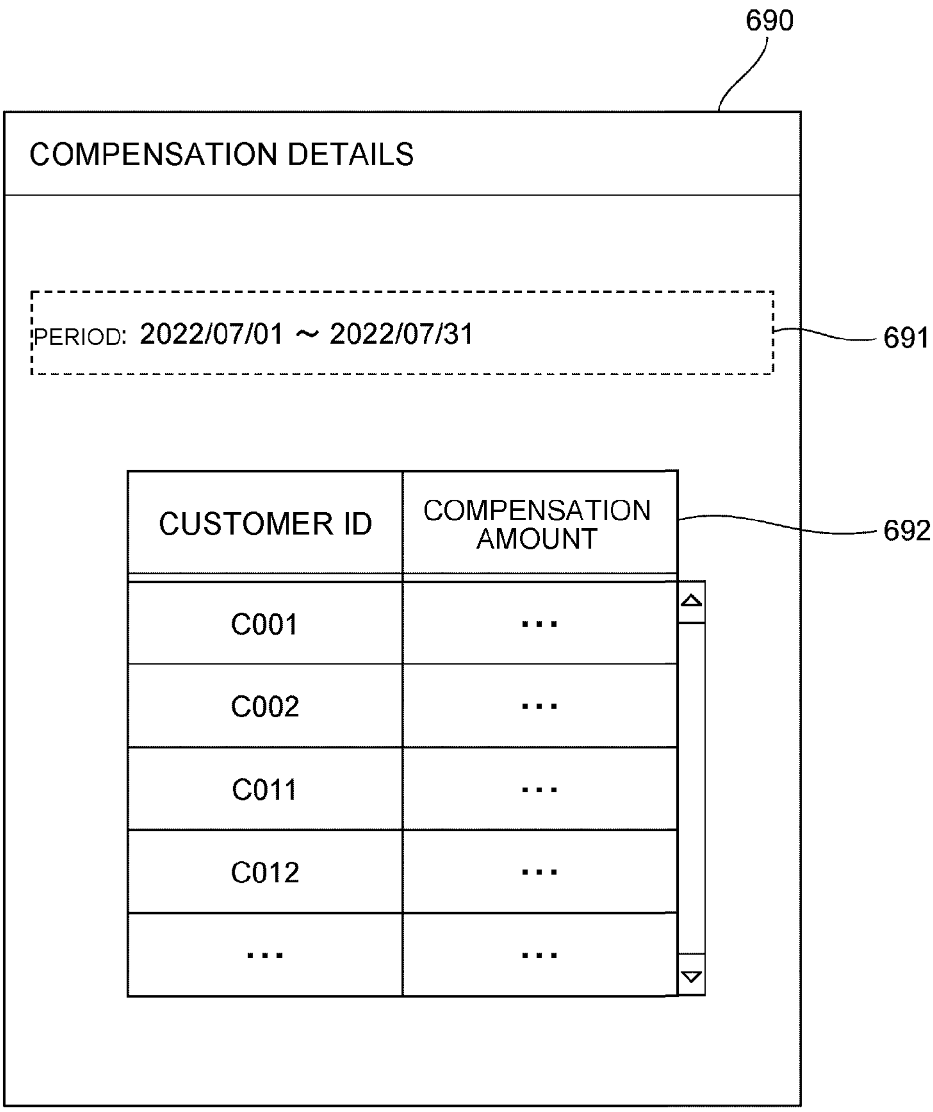
690
COMPENSATION DETAILS
PERIOD: 2022/07/01 ～ 2022/07/31
691
CUSTOMER ID
COMPENSATION AMOUNT
692
C001
C002
C011
C012
...

FIG.58

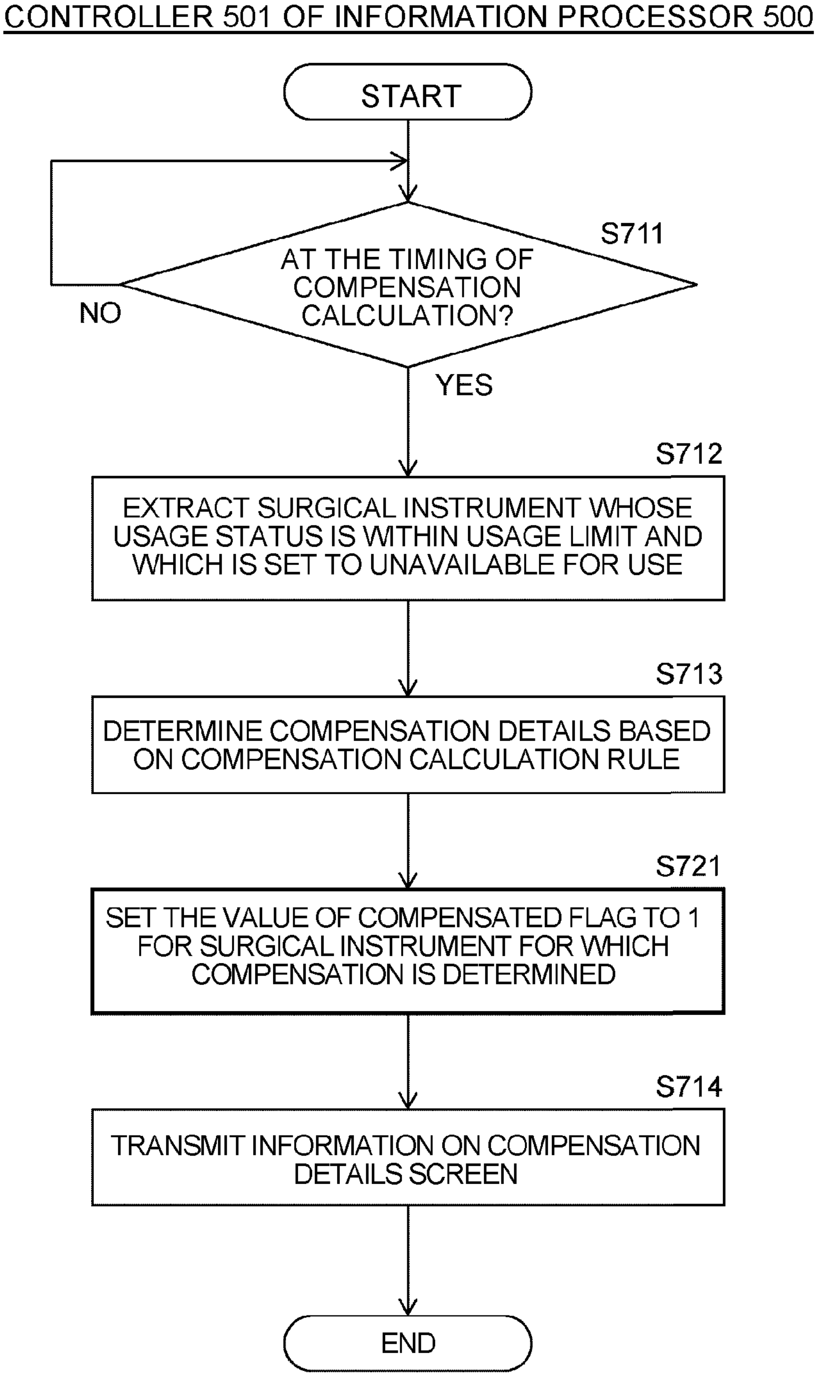
CONTROLLER 501 OF INFORMATION PROCESSOR 500
START
S711
AT THE TIMING OF COMPENSATION CALCULATION?
NO
YES
S712
EXTRACT SURGICAL INSTRUMENT WHOSE USAGE STATUS IS WITHIN USAGE LIMIT AND WHICH IS SET TO UNAVAILABLE FOR USE
S713
DETERMINE COMPENSATION DETAILS BASED ON COMPENSATION CALCULATION RULE
S721
SET THE VALUE OF COMPENSATED FLAG TO 1 FOR SURGICAL INSTRUMENT FOR WHICH COMPENSATION IS DETERMINED
S714
TRANSMIT INFORMATION ON COMPENSATION DETAILS SCREEN
END

FIG.59

EMBODIMENT 9

USER INFORMATION

| USER ID | CUSTOMER ID |
|---|---|
| 1111 | C001 |
| 1112 | C001 |
| 1121 | C002 |
| admin1 | service1 |
| ... | ... |
| | |

FIG.60

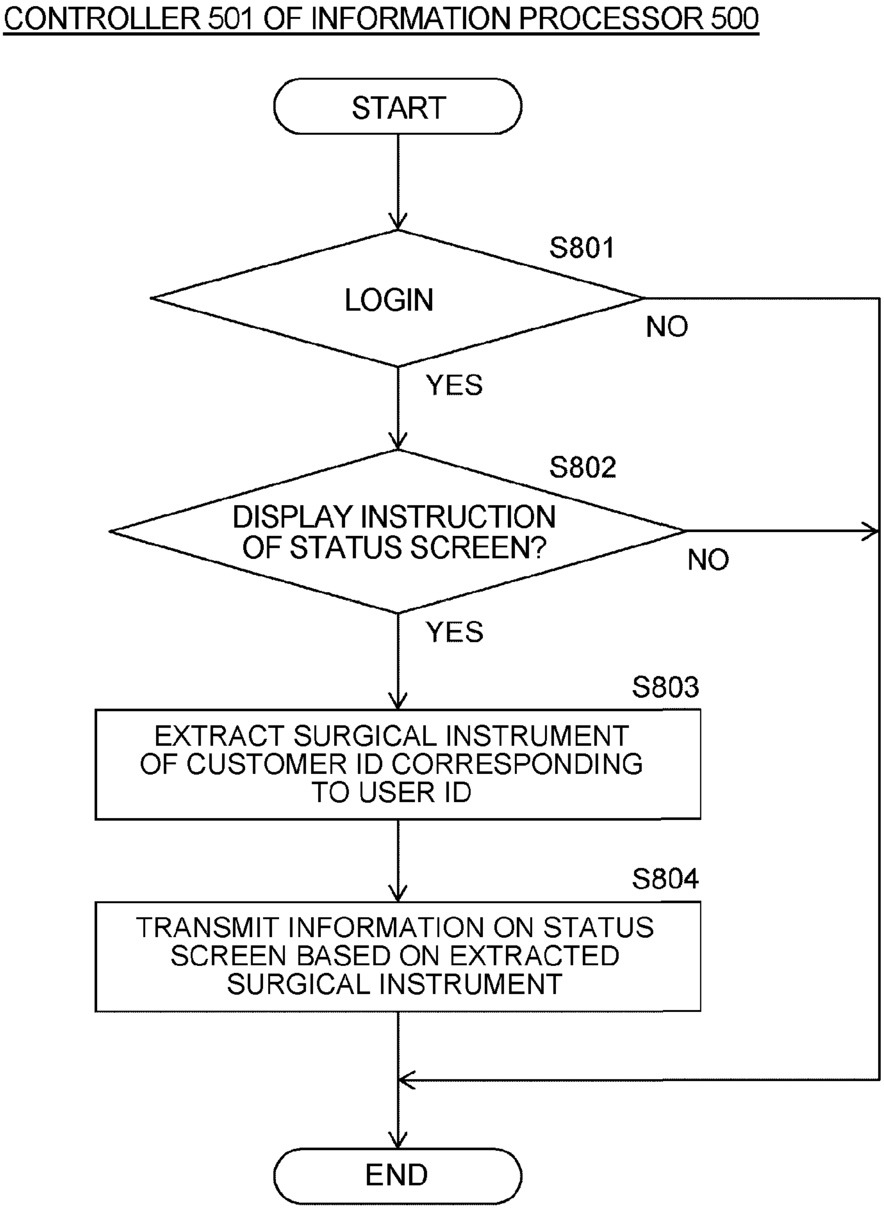
CONTROLLER 501 OF INFORMATION PROCESSOR 500
START
S801
LOGIN
NO
YES
S802
DISPLAY INSTRUCTION OF STATUS SCREEN?
NO
YES
S803
EXTRACT SURGICAL INSTRUMENT OF CUSTOMER ID CORRESPONDING TO USER ID
S804
TRANSMIT INFORMATION ON STATUS SCREEN BASED ON EXTRACTED SURGICAL INSTRUMENT
END

FIG.61

STATUS DISPLAY SCREEN DISPLAYED WHEN USER ID IS "ADMIN1"

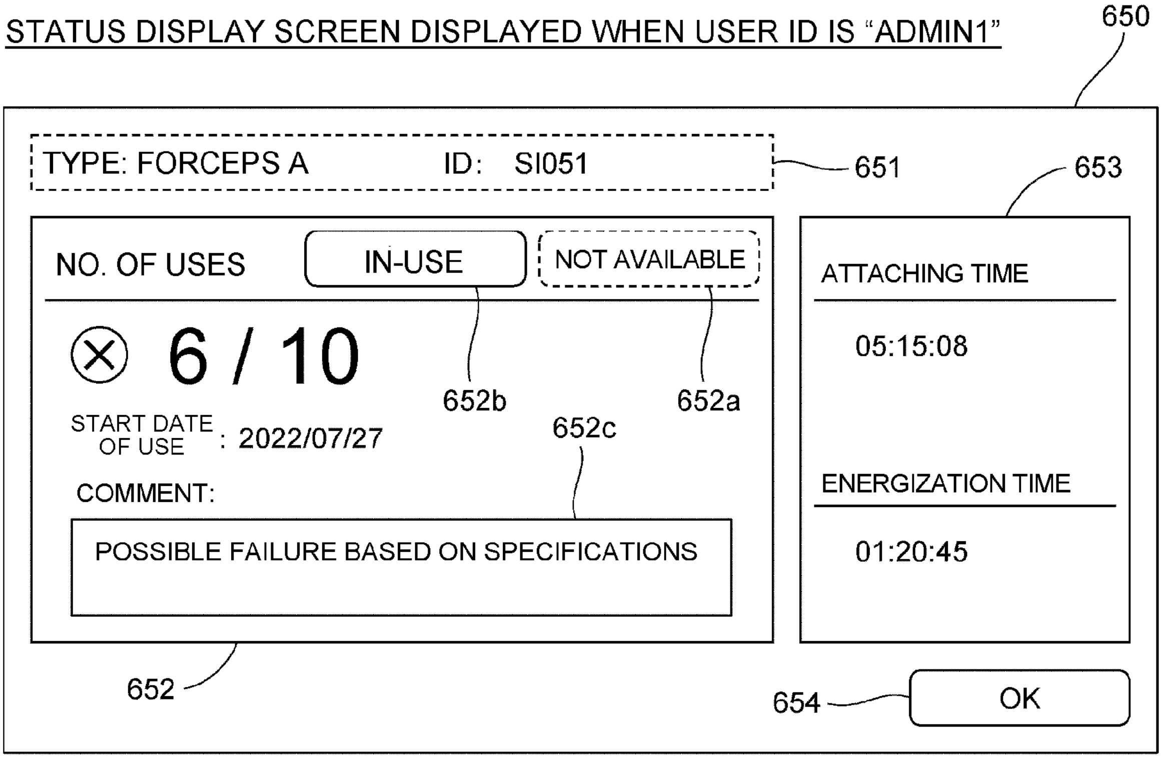


STATUS DISPLAY SCREEN DISPLAYED WHEN USER ID IS "1111"

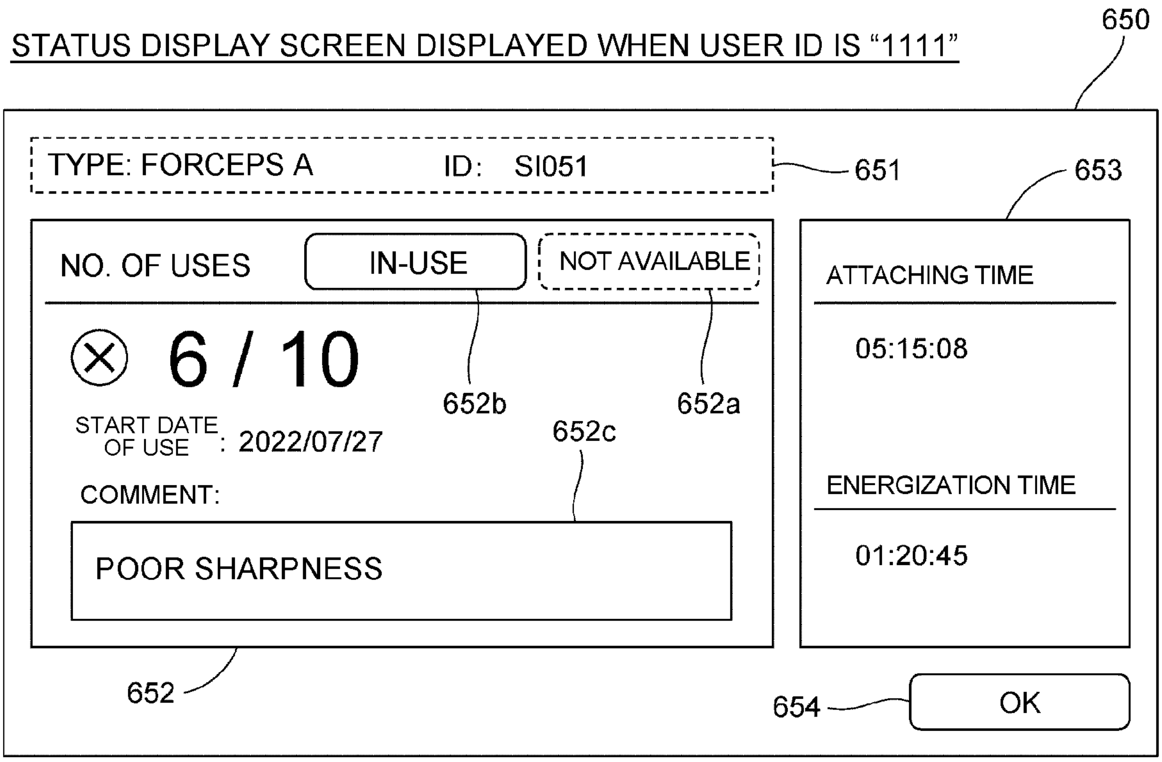

FIG.62

EMBODIMENT 10

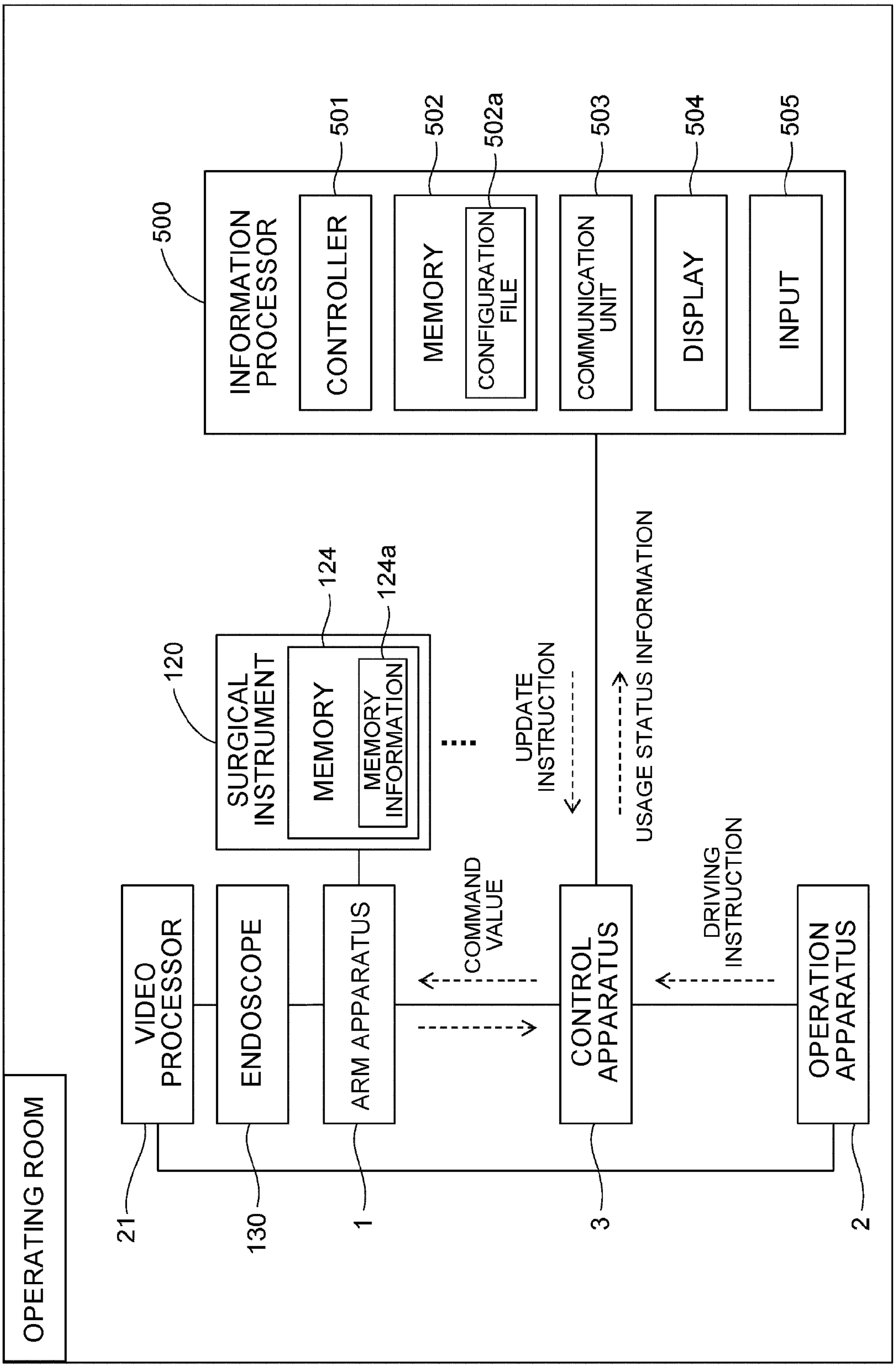

FIG.63 EMBODIMENT 11

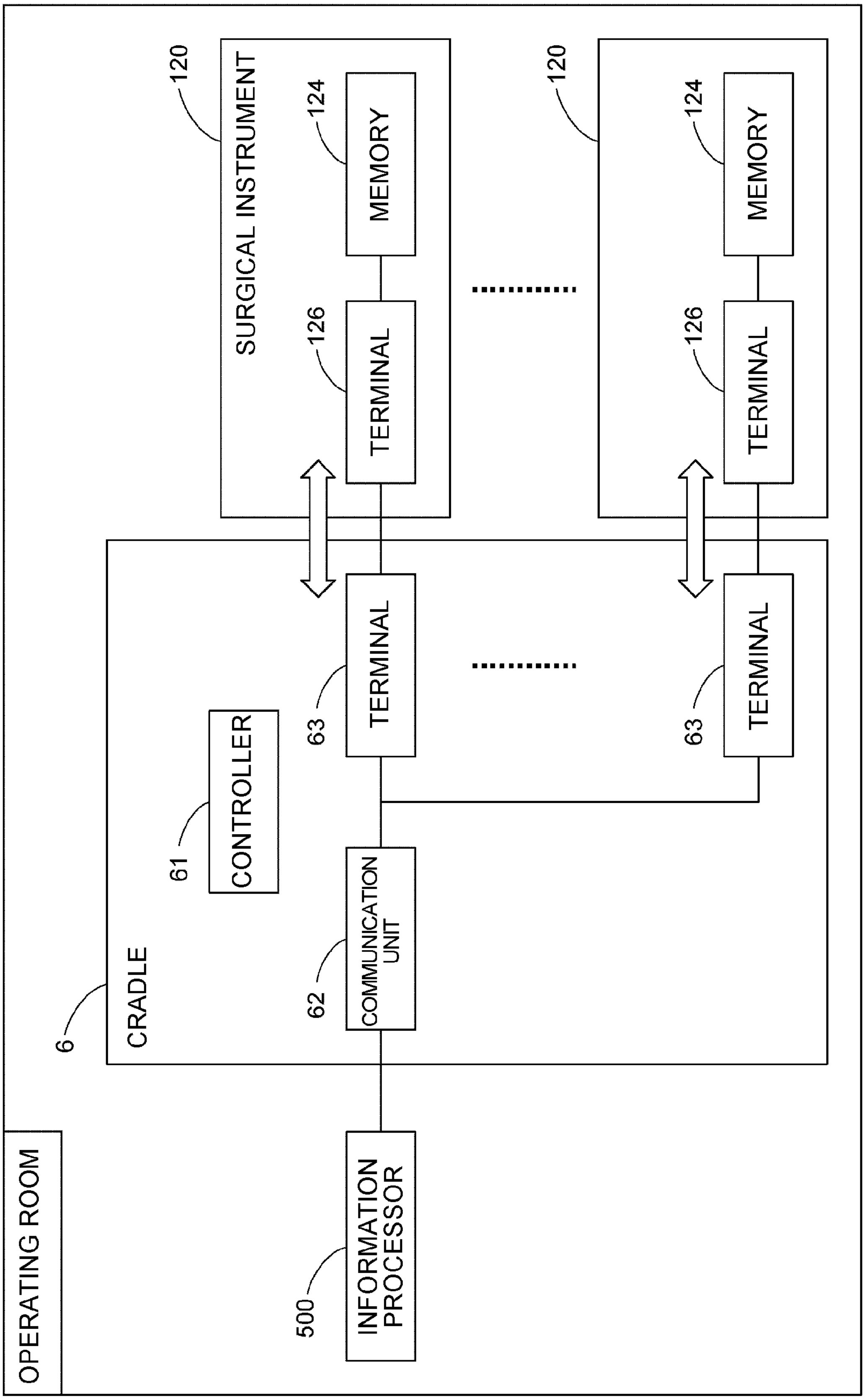

Europäisches Patentamt
European Patent Office
Office européen des brevets

# EUROPEAN SEARCH REPORT

Application Number
EP 23 21 8602

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/249198 A1 (JHAVERI MUFADDAL [US] ET AL) 11 August 2022 (2022-08-11) | 1-10, 14-16, 22,23, 25,26 | INV. A61B34/00 A61B34/37 |
| Y | * paragraphs [0062] - [0063], [0078]; figure 1 * * paragraphs [0069], [0071], [0072], [0078], [0086] - [0090]; figure 8 * ----- | 11-13, 18-21,24 | |
| Y | WO 2022/219496 A1 (CILAG GMBH INT [CH]) 20 October 2022 (2022-10-20) * paragraphs [0146], [0147] * ----- | 11-13 | |
| Y | US 2019/201117 A1 (YATES DAVID C [US] ET AL) 4 July 2019 (2019-07-04) | 18-21,24 | |
| A | * paragraphs [0292] - [0322] * ----- | 12 | |
| X | EP 2 845 546 A1 (ETHICON ENDO SURGERY INC [US]) 11 March 2015 (2015-03-11) * paragraphs [0016], [0164], [0190], [0195]; figures 75,177,181 * ----- | 1-5, 7-10,15, 16 | TECHNICAL FIELDS SEARCHED (IPC) A61B |
| A | WO 2021/118733 A1 (COVIDIEN LP [US]) 17 June 2021 (2021-06-17) * paragraph [0015] * ----- | 2 | |

The present search report has been drawn up for all claims

2

| Place of search | Date of completion of the search | Examiner |
|---|---|---|
| The Hague | 2 May 2024 | Schmidt, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 21 8602

This annex lists the patent family members relating to the patent documents cited in the above-mentioned European search report. The members are as contained in the European Patent Office EDP file on
The European Patent Office is in no way liable for these particulars which are merely given for the purpose of information.

02-05-2024

| Patent document cited in search report | Publication date | Patent family member(s) | Publication date |
|---|---|---|---|
| US 2022249198 A1 | 11-08-2022 | CN 115135272 A | 30-09-2022 |
| | | EP 4106662 A1 | 28-12-2022 |
| | | KR 20220143708 A | 25-10-2022 |
| | | US 2021251720 A1 | 19-08-2021 |
| | | US 2022249198 A1 | 11-08-2022 |
| | | WO 2021167626 A1 | 26-08-2021 |
| WO 2022219496 A1 | 20-10-2022 | BR 112023021228 A2 | 06-02-2024 |
| | | EP 4136649 A1 | 22-02-2023 |
| | | JP 2024517603 A | 23-04-2024 |
| | | WO 2022219496 A1 | 20-10-2022 |
| US 2019201117 A1 | 04-07-2019 | BR 112020013102 A2 | 01-12-2020 |
| | | CN 111527553 A | 11-08-2020 |
| | | EP 3635736 A1 | 15-04-2020 |
| | | JP 7350748 B2 | 26-09-2023 |
| | | JP 2021509507 A | 25-03-2021 |
| | | US 2019201117 A1 | 04-07-2019 |
| | | WO 2019130094 A1 | 04-07-2019 |
| EP 2845546 A1 | 11-03-2015 | BR 112016003537 A2 | 29-03-2022 |
| | | CN 105658154 A | 08-06-2016 |
| | | EP 2845546 A1 | 11-03-2015 |
| | | JP 6602764 B2 | 06-11-2019 |
| | | JP 2016530949 A | 06-10-2016 |
| | | RU 2016110420 A | 28-09-2017 |
| WO 2021118733 A1 | 17-06-2021 | CN 114746222 A | 12-07-2022 |
| | | EP 4072793 A1 | 19-10-2022 |
| | | US 2023024362 A1 | 26-01-2023 |
| | | WO 2021118733 A1 | 17-06-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8285517 B